# EUROPEAN PATENT SPECIFICATION

(11) **EP 4 025 153 B1**
(45) Date of publication and mention of the grant of the patent: **17.07.2024**
(21) Application number: 20775120.7
(22) Date of filing: 03.09.2020
(51) Int. Cl.: A61B 90/00, A61B 17/34, A61B 17/04

(54) **MEDICAL INSTRUMENTS WITH ECHOGENICITY-ENHANCEMENT CHARACTERISTICS**
MEDIZINISCHE INSTRUMENTE MIT ECHOGENITÄTSVERBESSERUNGSEIGENSCHAFTEN
INSTRUMENTS MÉDICAUX AYANT DES CARACTÉRISTIQUES D'AMÉLIORATION D'ÉCHOGÉNICITÉ

(30) Priority: 04.09.2019 US 201962895650 P; 21.07.2020 US 202063054751 P
(43) Date of publication of application: 13.07.2022
(73) Proprietor: Edwards Lifesciences Corporation, Irvine, CA 92614-5688 (US)
(72) Inventor: CORTEZ, Felino V. Jr., Irvine, CA 92614 (US); ETHERIDGE, Julie Marie, Irvine, CA 92614 (US); ZANETTI, Luke Anthony, Irvine, CA 92614 (US); EPSTEIN, Stephen, Irvine, CA 92614 (US); COURNANE, Stephen, Irvine, CA 92614 (US); KEFFER, Ashley Nicolette, Irvine, CA 92614 (US); NICHOLSON, Wesley Paul, Sunderland, Maryland 20689 (US)
(74) Representative: Eisenführ Speiser
(86) International application number: PCT/US2020/049115
(87) International publication number: WO 2021/046163

(56) References cited:
- EP-A1- 2 207 494
- US-A1- 2004 073 158
- US-A1- 2011 152 611
- US-A1- 2012 283 553
- US-A1- 2016 151 086
- US-A1- 2017 095 314

## Description

### RELATED APPLICATION

This application claims the benefit of U.S. Patent Application No. 62/895,650, filed on September 4, 2019, and U.S. Patent Application No. 63/054, 751, filed on July 21, 2020.

### BACKGROUND

### Technical Field

The present disclosure generally relates to the field of medical devices and processes.

### Description of Related Art

Certain medical procedures can be aided by the use of echo imaging technology. Echogenicity of devices used in such medical procedures can affect visibility of such devices under echo imaging.
US 2017/0095314 A1 discloses a kit for performing a reduced radiation percutaneous procedure. The kit includes a needle access device having a needle connected to a hub portion having an opaque cap portion, a non-opaque body portion positioned between the opaque cap portion and the needle, and a channel extending through the opaque cap portion. A sticker has an adhesive side adapted to adhere to the skin of a patient, and a display surface opposite the adhesive side is configured to enhance visualization of the sticker in low light. Moreover, a guidewire has a floppy portion with a distal end, an intermediate region connected to the floppy portion, such that the intermediate region is less floppy than the floppy portion. An ultrasonic-profile-enhancing feature is disposed within 3 centimeters of the distal end of the floppy portion.
US 2011/0152611 A1 discloses a fiducial deployment system with a handle configured for actuation of the same. A fiducial may include one or more protuberances configured to engage one or more slots in a needle of the system. The needle may be configured to deliver a plurality of fiducials to a target location in serial fashion, one at a time. In certain embodiments, echogenic placement of fiducials may present certain advantages. The handle may include structures configured for incrementally or otherwise controlledly deploying one or more fiducials by advancing a sty let through and/or retracting the body of a needle in which fiducials are disposed.
US 2016/0151086 A1 discloses a needle assembly including a hollow needle shaft. The assembly includes a plastics sleeve extruded onto and extending along the outside of the needle shaft, and that the extruded sleeve contains a plurality of gas bubbles within the thickness of the sleeve such that the sleeve is visible under ultrasound observation and such that the ultrasound visibility of the assembly with the sleeve is greater than that of the needle alone.
US 2004/0073158 A1 discloses a guide catheter comprising an elongated sheath including a fully radiopaque and echogenic distal tip, the distal tip having a length greater than or equal to approximately 0.2032 cm (0.08 inch).
US 2012/0283553 A1 discloses an echogenically enhanced interventional device comprising (a) an interventional device to be imaged ultrasonically, said tool or device having a surface with one or more apertures, and (b) a polymeric film in close contact with the surface of said device which covers at least a portion of the one or more apertures.

### SUMMARY

The present invention relates to a medical device shaft according to each of claims 1 and 7. Furthermore, the present invention relates to a method of manufacturing a medical instrument according to claim 10. Preferred embodiments are defined in the dependent claims.
Described herein are methods and devices that facilitate visibility of medical device shafts and other devices under echo imaging. In some implementations, the present disclosure relates to a medical device shaft comprising a rigid, elongate shaft, an end effector form coupled to a distal end of the elongate shaft, and echogenic additives associated with at least one of the elongate shaft and the end effector.

The echogenic additives can comprise microspheres. For example, the microspheres can be gas-filled glass spheres. In some examples, the echogenic additives are embedded within the end effector form. The echogenic additives are mixed with a coating material disposed on at least a portion of the elongate shaft. For example, the coating material can have an extruded sleeve form. The end effector form is a unitary form with the extruded sleeve. For example, the end effector form can be die-cut from the extruded sleeve. In some examples, the echogenic additives comprise particulates mixed into a polymeric material and the particulates are more, or less, dense than the polymeric material. For example, the particulates can comprise one or more of zinc oxide, iron oxide, titanium dioxide, platinum oxide, and silver oxide.

In some implementations, the present disclosure relates to a method of manufacturing a medical instrument. The method comprises providing a rigid, elongate shaft and coupling an end effector form to the elongate shaft. The end effector form includes echogenicity-enhancing features.

The method further comprises adding echogenic additives to a polymeric material. The method may further comprise applying the polymeric material with the echogenic additives to at least a portion of the shaft. The method may further comprise applying the polymeric material with the echogenic additives to at least a portion of the end effector form. Coupling the end effector form to the elongate shaft involves molding the end effector form from the polymeric material to the elongate shaft. For example, the method may further comprise, prior to said molding the end effector form to the elongate shaft, applying a liquid to one or more of the elongate shaft and a mold used to mold the end effector form to produce echogenic surface features in the end effector.

The method may further comprise using an abrasive surface to produce a rough surface on one or more of the elongate shaft and the end effector form, wherein the rough surface is configured to produce echo scattering. In some examples, the end effector form comprises porous material having greater echogenicity characteristics than the elongate shaft. For example, the porous material may be aerogel.

In some implementations, the present disclosure relates to a medical device shaft comprising a rigid, elongate shaft, an end effector form coupled to a distal end of the elongate shaft, and a metal echogenic-enhancement feature coupled to the end effector form.

In some examples, the metal echogenic-enhancement feature comprises one or more loops of wire secured to one or more of the elongate shaft and the end effector form. The metal echogenic-enhancement feature comprises a washer form coupled to a distal end of the end effector form. For example, the washer form may be at least partially embedded in the end effector form.

In some implementations, the present disclosure relates to a medical device shaft comprising an elongate shaft having one or more channels formed in an exterior surface of the shaft and an end effector form coupled to a distal end of the elongate shaft.

The medical device shaft can further comprise an outer covering disposed around at least a portion of the shaft and covering at least a portion of the one or more channels. The outer covering can comprise a polymer tube. In some examples, the one or more channels are helical over a length of the shaft.

For purposes of summarizing the disclosure, certain aspects, advantages and novel features are described herein. It is to be understood that not necessarily all such advantages may be achieved in accordance with any particular embodiment. Thus, the disclosed examples may be carried out in a manner that achieves or optimizes one advantage or group of advantages as taught herein without necessarily achieving other advantages as may be taught or suggested herein.

### BRIEF DESCRIPTION OF THE DRAWINGS

Various embodiments are depicted in the accompanying drawings for illustrative purposes and should in no way be interpreted as limiting the scope of the disclosure. In addition, various features of different disclosed embodiments can be combined to form additional embodiments, which are part of this disclosure. Throughout the drawings, reference numbers may be reused to indicate correspondence between reference elements. However, it should be understood that the use of similar reference numbers in connection with multiple drawings does not necessarily imply similarity between respective embodiments associated therewith. Furthermore, it should be understood that the features of the respective drawings are not necessarily drawn to scale, and the illustrated sizes thereof are presented for the purpose of illustration of disclosed aspects thereof. Generally, certain of the illustrated features may be relatively smaller than as illustrated in some embodiments or configurations.
Figure 1 is a cutaway view of the human heart.
Figure 2 is a perspective view of a tissue anchor delivery device in accordance with one or more examples.
Figure 3 is a cutaway view of a tissue anchor delivery device disposed at least partially within a chamber of a heart in accordance with one or more examples.
Figure 4 shows a perspective view of a dilator and introducer system in accordance with one or more examples.
Figure 5 shows an introducer device and tissue anchor delivery shaft in accordance with one or more examples.
Figure 6 is a close-up view of a distal portion of a tissue anchor delivery device shaft assembly positioned against a target heart valve leaflet in accordance with one or more examples.
Figure 7 is a close-up view of a distal portion of a tissue anchor delivery device shaft having a needle and tissue anchor sutureform projected therefrom through a target heart valve leaflet in accordance with one or more examples.
Figure 8 is a close-up view of a distal portion of a tissue anchor delivery device shaft assembly positioned against a target heart valve leaflet and an associated tissue anchor deployed on a distal side of the leaflet in accordance with one or more examples.
Figure 9 shows a cutaway view of a deployed leaflet anchor in a heart in accordance with one or more examples.
Figures I0A and I0B show side views of certain imaging probes disposed in positions to generate echo images of a patient in accordance with one or more examples.
Figure 11 shows a perspective view of a distal portion of a multi-lumen medical device shaft in accordance with one or more examples.
Figure 12 shows a side view of the multi-lumen medical device shaft of Figure 11 in accordance with one or more examples.
Figure 13 shows a front axial view of the multi-lumen medical device shaft of Figure 11 in accordance with one or more examples.
Figure 14 is a flow diagram illustrating a process for targeting a heart valve leaflet using a multi-lumen shaft in accordance with one or more examples.
Figure 15 is a perspective view of a distal portion of a multi-lumen medical device shaft in accordance with one or more examples.
Figure 16 is a perspective view of a distal portion of a multi-lumen medical device shaft in accordance with one or more examples.
Figure 17 is a perspective view of a distal portion of a multi-lumen medical device shaft in accordance with one or more examples.
Figure 18 is a perspective view of a distal portion of a multi-lumen medical device shaft in accordance with one or more examples.
Figure 19 is a flow diagram illustrating a process for manufacturing a multi-lumen medical device shaft using an extrusion process in accordance with one or more examples.
Figure 20 is a perspective view of a distal portion of a multi-lumen medical device shaft in accordance with one or more examples.
Figure 21 is a perspective view of a distal portion of a multi-lumen medical device shaft having a needle and tissue anchor sutureform projected from a working lumen thereof in accordance with one or more examples.
Figure 22 shows a perspective view of a portion of an image-enhancement sleeve for association with a medical device shaft in accordance with one or more examples.
Figure 23 shows a medical device shaft having one or more coils wrapped around the shaft in accordance with one or more examples.
Figure 24A is a close-up view of a distal portion of the medical device shaft of Figure 23 in accordance with one or more examples.
Figure 24B is a cross-sectional view of a portion of the medical device shaft portion shown in Figure 24A in accordance with one or more examples.
Figure 25 shows a medical device shaft having one or more coils with non-uniform spacing wrapped around the shaft in accordance with one or more examples.
Figure 26 shows an imaging window showing a representation of a medical device shaft in accordance with one or more examples.
Figure 27 is a flow diagram illustrating a process for manufacturing a medical device shaft having image-enhancement features in accordance with one or more examples.
Figure 28 is a perspective view of a portion of a medical device shaft having image-enhancement channels formed therein using a die tool in accordance with one or more examples.
Figure 29 is an axial view of a portion of the medical device shaft shown in Figure 28 in accordance with one or more examples.
Figure 30 shows a side view of a medical device shaft having one or more image-enhancement channels implemented therein in accordance with one or more examples.
Figure 31A is a close-up view of a distal portion of the medical device shaft of Figure 30 in accordance with one or more examples.
Figure 31B is a cross-sectional view of a portion of the medical device shaft portion shown in Figure 31A in accordance with one or more examples.
Figure 32 is a perspective view of a portion of a multi-lumen medical device shaft in accordance with one or more examples.
Figure 33 is a cross-sectional side view of the portion of the multi-lumen medical device shaft shown in Figure 32 in accordance with one or more examples.
Figure 34 is a cross-sectional side view of a medical device shaft having one or more image-enhancement balloon features in accordance with one or more examples.
Figure 35 shows a perspective view of a medical device shaft having one or more image-enhancement balloon features in accordance with one or more examples.
Figure 36 is a close-up view of a distal portion of the medical device shaft of Figure 35 in accordance with one or more examples.
Figure 37 is a flow diagram of a process for targeting a valve leaflet using a multi-lumen/chamber medical device shaft containing echogenic contrast media in accordance with one or more examples.
Figure 38 shows a valve repair system and introducer including a guidewire port in accordance with one or more examples.
Figure 39 shows a cutaway view of the introducer shown in Figure 38 in accordance with one or more examples.
Figure 40 is a flow diagram illustrating a process for advancing a medical device shaft using a guidewire in accordance with one or more examples.
Figure 41 shows a perspective view of a distal portion of a medical device shaft including a guidewire-engagement feature in accordance with one or more examples.
Figure 42A provides a side view of a medical instrument including a shaft and an end effector associated with a distal end portion of the shaft in accordance with one or more examples.
Figure 42B provides an end view of the end effector of the medical instrument shown in Figure 42A in accordance with one or more examples.
Figure 43 provides a cross-sectional view of certain cardiac anatomy in accordance with one or more examples.
Figures 44A and 44B show bicommissural cross-sectional and echo imaging views, respectively, of the cardiac anatomy shown in Figure 43 in accordance with one or more examples.
Figures 45A and 45B show long-axis cross-sectional and echo imaging views, respectively, of the cardiac anatomy shown in Figure 43 in accordance with one or more examples.
Figure 46 shows side, perspective, and end views of a shaft and end effector of a medical instrument having relatively echogenic coating applied thereto in accordance with one or more examples.
Figure 47 shows side, perspective, and end views of a shaft of a medical instrument having a relatively echogenic sleeve disposed thereon in accordance with one or more examples.
Figure 48 shows side and perspective views of a shaft of a medical instrument having relatively echogenic bands associated therewith in accordance with one or more examples.
Figure 49 shows side, perspective, and end views of a shaft and end effector of a medical instrument having relatively echogenic microspheres associated with one or more portions thereof in accordance with one or more examples.
Figure 50 shows side, perspective, and end views of a shaft and end effector of a medical instrument having relatively echogenic rough surfaces associated with one or more portions thereof in accordance with one or more examples.
Figure 51 shows side, perspective, and end views of a shaft and end effector of a medical instrument having relatively echogenic particulates associated with one or more portions thereof in accordance with one or more examples.
Figure 52 shows side, perspective, and end views of a shaft and end effector of a medical instrument having relatively echogenic aerogel material associated with one or more portions thereof in accordance with one or more examples.
Figure 53 shows side, perspective, and end views of a shaft of a medical instrument having a relatively echogenic washer-type end effector in accordance with one or more examples.
Figure 54 shows side, perspective, and end views of a shaft of a medical instrument having an end effector with a relatively echogenic, washer-type component embedded at least partially therein in accordance with one or more examples.
Figure 55 shows side, perspective, and end views of a shaft and end effector of a medical instrument having certain relatively echogenic air pockets and/or surface imperfections associated with one or more portions thereof in accordance with one or more examples.
Figure 56 shows side, perspective, and end views of a shaft of a medical instrument including an end effector with one or more relatively echogenic wires and/or loops associated therewith in accordance with one or more examples.
Figures 57A and 57B show echo images of relatively low- and high-echogenicity shafts and/or end effectors associated with a medical instrument in accordance with one or more examples.
Figure 58 is a flow diagram for a process for manufacturing an echogenically-enhanced medical instrument shaft in accordance with one or more examples.
Figure 59 shows a side vies of a shaft having one or more grooves formed therein in accordance with one or more examples.
Figure 60 shows a side view of a shaft having an echogenic form disposed on a portion thereof in accordance with one or more examples.

To further clarify various aspects of embodiments of the present disclosure, a more particular description of certain embodiments will be made by reference to various aspects of the appended drawings. It is appreciated that these drawings depict only typical embodiments of the present disclosure and are therefore not to be considered limiting of the scope of the disclosure. Moreover, while the figures can be drawn to scale for some embodiments, the figures are not necessarily drawn to scale for all embodiments. Embodiments of the present disclosure will be described and explained with additional specificity and detail through the use of the accompanying drawings.

### DETAILED DESCRIPTION

The following description refers to the accompanying drawings, which illustrate specific embodiments. Other embodiments having different structures and operation do not depart from the scope of the disclosure. Embodiments of the present disclosure relate to devices and methods for improving visibility of component(s) of medical devices or systems under echo imaging. The terms "echo," "echo imaging," "echo image," "cardiac echo," "echocardiography," "echocardiogram," "medical ultrasound," "ultrasound," and like terms are used herein according to their broad and ordinary meanings and may refer to any type of ultrasound (e.g., medical ultrasound), diagnostic sonography, ultrasonography, or any other type of sonic/sound-based imaging technology or modality and/or image(s) generated in connection therewith, whether related to images of cardiac anatomy or other anatomy or subject matter. Embodiments of the present disclosure relate to echogenic features, components, and/or devices associated with a delivery device or system for implementing tissue anchor delivery and/or valve repair, such as mitral valve repair, on a beating heart. Such delivery devices/systems may be referred to herein as tissue anchor delivery devices/systems and/or valve repair devices/systems. The terms "echogenic" and "echogenicity" are used herein according to their broad and ordinary meanings and may refer to the ability of a material, device, component, or surface to reflect, return, or bounce a sound wave (e.g., an echo). As used herein, echogenicity is considered relatively higher when the surface reflecting the sound wave/echo reflects relatively higher-amplitude sound waves. Generally, a material, device, component, or surface described herein as echogenic, or as having image-enhancement characteristics, may be considered to have a relatively higher echogenicity than an at least partially convex stainless-steel surface. The term "associated with" is used herein according to its broad and ordinary meaning. For example, where a first feature, element, component, device, or member is described as being "associated with" a second feature, element, component, device, or member, such description should be understood as indicating that the first feature, element, component, device, or member is physically coupled, attached, or connected to, integrated with, or otherwise physically related to the second feature, element, component, device, or member.

Real-time echo (e.g., ultrasound) guidance for certain interventions/procedures (e.g., mitral valve repair procedures) can be implemented in reliance at least in part on visualization of certain surgical components, such as tissue anchor delivery device shafts, and the like. For example, as a medical device shaft is advanced towards and positioned onto the target anchor deployment site (e.g., mitral valve leaflet), visibility of the shaft under echo can be critical. Instances of the present disclosure provide enhanced echogenicity/visibility for medical device shafts and other devices under echo imaging to enable the echo cardiographer and surgeon to relatively safely and/or quickly steer and navigate the shaft in the target anatomical cavity (e.g., cardiac ventricle) and to allow for precise targeting of the shaft to improve success rates associated with surgical procedures.

The following includes a general description of human cardiac anatomy that is relevant to certain features and examples disclosed herein and is included to provide context for certain aspects of the present disclosure. In humans and other vertebrate animals, the heart generally comprises a muscular organ having four pumping chambers, wherein the flow thereof is at least partially controlled by various heart valves, namely, the aortic, mitral (or bicuspid), tricuspid, and pulmonary valves. The valves may be configured to open and close in response to a pressure gradient present during various stages of the cardiac cycle (e.g., relaxation and contraction) to at least partially control the flow of blood to a respective region of the heart and/or to blood vessels (e.g., pulmonary, aorta, etc.).

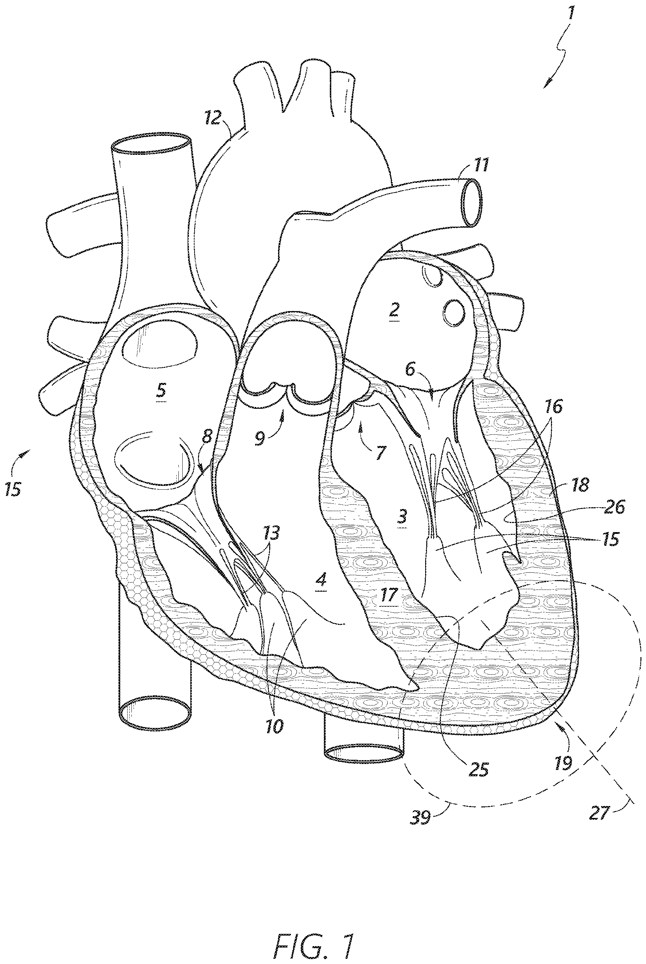

Figure 1 illustrates an example representation of a heart 1 having various features relevant to certain aspects of the present disclosure. The heart 1 includes four chambers, namely the left ventricle 3, the left atrium 2, the right ventricle 4, and the right atrium 5. A wall of muscle 17, referred to as the septum, separates the left 2 and right 5 atria and the left 3 and right 4 ventricles. The inferior tip 19 of the heart 1 is referred to as the apex and is generally located on the midclavicular line, in the fifth intercostal space. The apex 19 can be considered part of the greater apical region 39.

The left ventricle 3 is the primary pumping chamber of the heart 1. A healthy left ventricle is generally conical or apical in shape in that it is longer (along a longitudinal axis extending in a direction from the aortic valve 7 to the apex 19) than it is wide (along a transverse axis extending between opposing walls 25, 26 at the widest point of the left ventricle) and descends from a base 15 with a decreasing cross-sectional circumference to the point or apex 19. Generally, the apical region 39 of the heart is a bottom region of the heart that is within the left or right ventricular region but is distal to the mitral 6 and tricuspid 8 valves and toward the tip of the heart. More specifically, the apical region 39 may be considered to be within about 20 cm to the right or to the left of the median axis 27 of the heart 1.

The pumping of blood from the left ventricle is accomplished by a squeezing motion and a twisting or torsional motion. The squeezing motion occurs between the lateral wall 18 of the left ventricle and the septum 17. The twisting motion is a result of heart muscle fibers that extend in a circular or spiral direction around the heart. When these fibers contract, they produce a gradient of angular displacements of the myocardium from the apex 19 to the base 15 about the longitudinal axis of the heart. The resultant force vectors extend at angles from about 30-60 degrees to the flow of blood through the aortic valve 7. The contraction of the heart is manifested as a counterclockwise rotation of the apex 19 relative to the base 15, when viewed from the apex 19. A healthy heart can pump blood from the left ventricle in a very efficient manner due to the spiral contractility of the heart.

The heart 1 further includes four valves for aiding the circulation of blood therein, including the tricuspid valve 8, which separates the right atrium 5 from the right ventricle 4. The tricuspid valve 8 may generally have three cusps or leaflets and may generally close during ventricular contraction (e.g., systole) and open during ventricular expansion (e.g., diastole). The valves of the heart 1 further include the pulmonary valve 9, which separates the right ventricle 4 from the pulmonary artery 11 and may be configured to open during systole so that blood may be pumped toward the lungs, and close during diastole to prevent blood from leaking back into the heart from the pulmonary artery. The pulmonary valve 9 generally has three cusps/leaflets, wherein each one may have a crescent-type shape. The heart 1 further includes the mitral valve 6, which generally has two cusps/leaflets and separates the left atrium 2 from the left ventricle 3. The mitral valve 6 may generally be configured to open during diastole so that blood in the left atrium 2 can flow into the left ventricle 3, and advantageously close during diastole to prevent blood from leaking back into the left atrium 2. The aortic valve 7 separates the left ventricle 3 from the aorta 12. The aortic valve 7 is configured to open during systole to allow blood leaving the left ventricle 3 to enter the aorta 12, and close during diastole to prevent blood from leaking back into the left ventricle 3.

The atrioventricular (e.g., mitral and tricuspid) heart valves may comprise a collection of chordae tendineae (13, 16) and papillary muscles (10, 15) for securing the leaflets of the respective valves to promote and/or facilitate proper coaptation of the valve leaflets and prevent prolapse thereof. The papillary muscles, for example, may generally comprise finger-like projections from the ventricle wall. With respect to the tricuspid valve 8, the normal tricuspid valve may comprise three leaflets and three corresponding papillary muscles 10 (two shown in Figure 1). The leaflets of the tricuspid valve may be referred to as the anterior, posterior and septal leaflets, respectively. The valve leaflets are connected to the papillary muscles 10 by the chordae tendineae 13, which are disposed in the right ventricle 4 along with the papillary muscles 10.

Surrounding the ventricles (3, 4) are a number of arteries (not shown) that supply oxygenated blood to the heart muscle and a number of veins that return the blood from the heart muscle. The coronary sinus (not shown) is a relatively large vein that extends generally around the upper portion of the left ventricle 3 and provides a return conduit for blood returning to the right atrium 5. The coronary sinus terminates at the coronary ostium (not shown) through which the blood enters the right atrium.

With respect to the mitral valve 6, a normal mitral valve may comprise two leaflets (anterior and posterior) and two corresponding papillary muscles 15. The papillary muscles 15 originate in the left ventricle wall and project into the left ventricle 3. Generally, the anterior leaflet may cover approximately two-thirds of the valve annulus. Although the anterior leaflet covers a greater portion of the annulus, the posterior leaflet may comprise a larger surface area in certain anatomies.

Various disease processes can impair the proper functioning of one or more of the valves of the heart. These disease processes include degenerative processes (e.g., Barlow's Disease, fibroelastic deficiency), inflammatory processes (e.g., rheumatic heart disease) and infectious processes (e.g., endocarditis). Additionally, damage to the ventricle from prior heart attacks (e.g., myocardial infarction secondary to coronary artery disease) or other heart diseases (e.g., cardiomyopathy) can distort the valve's geometry causing it to dysfunction. However, the vast majority of patients undergoing valve surgery, such as mitral valve surgery, suffer from a degenerative disease that causes a malfunction in one or more leaflets of the valve which results in prolapse and regurgitation.

The mitral valve 6 and tricuspid valve 8 can be divided into three parts: an annulus, leaflets, and a sub-valvular apparatus. The sub-valvular apparatus can be considered to include the papillary muscles 10, 15 and the chordae tendineae 13, 16, which can elongate and/or rupture. If a valve is functioning properly, when closed, the free margins or edges of the leaflets come together and form a tight junction, the arc of which, in the mitral valve, is known as the line, plane or area of coaptation. Normal mitral and tricuspid valves open when the ventricles relax allowing blood from the atrium to fill the decompressed ventricle. When the ventricle contracts, the chordae tendineae advantageously properly tether or position the valve leaflets such that the increase in pressure within the ventricle causes the valve to close, thereby preventing blood from leaking into the atrium and assuring that substantially all of the blood leaving the ventricle is ejected through the aortic valve 7 or pulmonic valve 9 and into the arteries of the body. Accordingly, proper function of the valves depends on a complex interplay between the annulus, leaflets, and sub-valvular apparatus. Lesions in any of these components can cause the valve to dysfunction and thereby lead to valve regurgitation.

Generally, there are three mechanisms by which a heart valve becomes regurgitant or incompetent; they include Carpentier's type I, type II and type III malfunctions. A Carpentier type I malfunction involves the dilation of the annulus such that normally functioning leaflets are distracted from each other and fail to form a tight seal (e.g., do not coapt properly). Included in a type I mechanism malfunction are perforations of the valve leaflets, as in endocarditis. A Carpentier's type II malfunction involves prolapse of one or both leaflets above the plane of coaptation. This is the most common cause of mitral regurgitation and is often caused by the stretching or rupturing of chordae tendineae normally connected to the leaflet. A Carpentier's type III malfunction involves restriction of the motion of one or more leaflets such that the leaflets are abnormally constrained below the level of the plane of the annulus. Leaflet restriction can be caused by rheumatic disease (Illa) or dilation of the ventricle (IIIb).

One or more chambers in the heart 1 may be accessed in accordance with certain heart valve-repair procedures and/or other interventions. Access into a chamber in the heart may be made at any suitable site of entry. In some implementations, access is made to a chamber of the heart, such as a target ventricle (e.g., left ventricle) associated with a diseased heart valve, through the apical region 39. For example, access into the left ventricle 3 (e.g., to perform a mitral valve repair) may be gained by making a relatively small incision at the apical region 39, close to (or slightly skewed toward the left of) the median axis 27 of the heart. Access into the right ventricle 4 (e.g., to perform a tricuspid valve repair) may be gained by making a small incision into the apical region 39, close to or slightly skewed toward the right of the median axis 27 of the heart. Accordingly, the ventricle can be accessed directly via the apex, or via an off-apex location that is in the apical region 39 but slightly removed from the tip/apex, such as via lateral ventricular wall, a region between the apex and the base of a papillary muscle, or even directly at the base of a papillary muscle. In some implementations, the incision made to access the appropriate ventricle of the heart is no longer than about 1 mm to about 5 cm, from 2.5 mm to about 2.5 cm, or from about 5 mm to about 1 cm in length. When a percutaneous approach is sought, no incision into the apex region of the heart may be made, but rather access into the apical region 39 may be gained by direct needle puncture, for instance by an 18-gauge needle, through which an appropriate repair instrument can be advanced.

Certain features disclosed herein relate to certain heart valve repair systems and devices, and/or systems, process, and devices for repairing any other type of target organ tissue. In some implementations, a tissue anchor delivery device may be employed in repairing a mitral valve in patients suffering from degenerative mitral regurgitation or other condition. In some implementations, a transapical off-pump echo-guided repair procedure is implemented in which at least part (e.g., a shaft portion/assembly) of a valve repair system is inserted in the left ventricle and steered to the surface of the diseased portion of a target mitral valve leaflet and used to deploy/implant a tissue anchor in the target leaflet. The tissue anchor (e.g., sutureform formed into a bulky knot) may advantageously be integrated or coupled with one or more artificial/synthetic cords serving a function similar to that of chordae tendinea. Such artificial cord(s) may comprise suture(s) and/or suture tail portions associated with a knot-type tissue anchor and may comprise any suitable or desirable material, such as expanded polytetrafluoroethylene (ePTFE) or the like. The term "suture" is used herein according to its broad and ordinary meaning and may refer to any elongate cord, strip, strand, line, tie, string, ribbon, strap, or portion thereof, or other type of material used in medical procedures. One having ordinary skill in the art will understand that a wire or other similar material may be used in place of a suture. Furthermore, in some contexts herein, the terms "cord," "chordae," and "suture" may be used substantially interchangeably. In addition, use of the singular form of any of the suture-related terms listed above, including the terms "suture" and "cord," may be used to refer to a single suture/cord, or to a portion thereof. For example, where a suture knot or anchor is deployed on a distal side of a tissue portion, and where two suture portions extend from the knot/anchor on a proximal side of the tissue, either of the suture portions may be referred to as a "suture" or a "cord," regardless of whether both portions are part of a unitary suture or cord.

Processes for repairing a target organ tissue, such as repair of mitral valve leaflets to address mitral valve regurgitation, can include inserting a tissue anchor delivery device, such as a delivery device as described in PCT Application No. PCT/US2012/043761, (published as WO 2013/003228, and referred to herein as "the '761 PCT Application") and/or in PCT Application No. PCT/US2016/055170 (published as WO 2017/059426 and referred to herein as "the '170 PCT Application") into a body and extending a distal end of the delivery device to a proximal side of the target tissue (e.g., leaflet).

The '761 PCT Application and the '170 PCT Application describe in detail methods and devices for performing non-invasive procedures to repair a cardiac valve, such as a mitral valve. Such procedures include procedures to repair regurgitation that occurs when the leaflets of the mitral valve do not coapt properly at peak contraction pressures, resulting in an undesired backflow of blood from the ventricle into the atrium. As described in the '761 PCT Application and the '170 PCT Application, after the malfunctioning cardiac valve has been assessed and the source of the malfunction verified, a corrective procedure can be performed. Various procedures can be performed in accordance with the methods described therein to effectuate a cardiac valve repair, which may depend on the specific abnormality and the tissues involved.

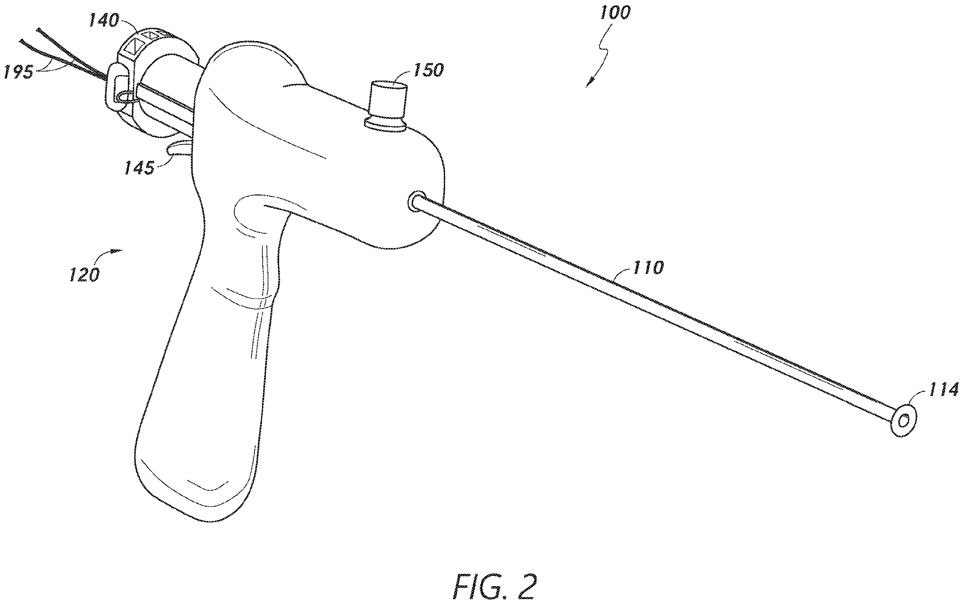

Figure 2 is a perspective view of a tissue anchor delivery system 100 in accordance with one or more examples. The tissue anchor delivery system 100 may be used to repair a heart valve, such as a mitral valve, and improve functionality thereof. For example, the tissue anchor delivery system 100 may be used to reduce the degree of mitral regurgitation in patients suffering from mitral regurgitation caused by, for example, midsegment prolapse of valve leaflets as a result of degenerative mitral valve disease. In order to repair such a valve, the tissue anchor delivery system 100 may be utilized to deliver and anchor tissue anchors, such as suture-knot-type tissue anchors, in a prolapsed valve leaflet. As described in detail below, such procedure may be implemented on a beating heart.

The delivery system 100 includes a rigid elongate tube 110 forming at least one internal working lumen. Although described in certain examples and/or contexts as comprising a rigid elongate tube, it should be understood that tubes, shafts, lumens, conduits, and the like disclosed herein may be either rigid, at least partially rigid, at least flexible, and/or at least partially flexible. Therefore, any such component described herein, whether or not referred to as rigid herein should be interpreted as possibly being at least partially flexible. In accordance with the present disclosure, the rigid elongate tube 110 may be referred to as a shaft for simplicity. Implementation of a valve-repair procedure utilizing the delivery system 100 can be performed in conjunction with certain imaging technology designed to provide visibility of the shaft 110 of the delivery system 100 according to a certain imaging modality, such as echo imaging. Generally, when performing a valve-repair procedure utilizing the tissue anchor delivery system 100, the operating physician may advantageously work in concert with an imaging technician, who may coordinate with the physician to facilitate successful execution of the valve-repair procedure.

In addition to the delivery shaft 110, the delivery system 100 may include a plunger feature 140, which may be used or actuated to manually deploy a pre-formed knot, such as a bulky knot as described in detail below. The tissue anchor delivery system 100 may further include a plunger lock mechanism 145, which may serve as a safety lock that locks the valve delivery system until ready for use or deployment of a leaflet anchor as described herein. The plunger 140 may have associated therewith a suture-release mechanism, which may be configured to lock in relative position a pair of suture tails 195 associated with a pre-formed knot anchor (not shown) to be deployed. For example, the suture portions 195 may be ePTFE sutures. The system 100 may further comprise a flush port 150, which may be used to de-air the lumen of the shaft 110. For example, heparinized saline flush, or the like, may be connected to the flush port 150 using a female Luer fitting to de-air the valve repair system 100. The term "lumen" is used herein according to its broad and ordinary meaning, and may refer to a physical structure forming a cavity, void, pathway, or other channel, such as an at least partially rigid elongate tubular structure, or may refer to a cavity, void, pathway, or other channel, itself, that occupies a space within an elongate structure (e.g., a tubular structure). Therefore, with respect to an elongate tubular structure, such as a shaft, tube, or the like, the term "lumen" may refer to the elongate tubular structure and/or to the channel or space within the elongate tubular structure.

The lumen of the shaft 110 may house a needle (not shown) that is wrapped at least in part with a pre-formed knot sutureform anchor, as described in detail herein. In some examples, the shaft 110 presents a relatively low profile. For example, the shaft 110 may have a diameter of approximately 3 mm or less (e.g., 9 Fr). The shaft 110 is associated with an atraumatic tip 114 feature. The atraumatic tip 114 can be an echogenic leafletpositioner component, which may be used for deployment and/or positioning of the suture-type tissue anchor. The atraumatic tip 114, disposed at the distal end of the shaft 110, may be configured to have deployed therefrom a wrapped pre-formed suture knot (e.g., sutureform), as described herein.

The atraumatic tip 114 may be referred to as an "end effector." In addition to a preformed knot sutureform and associated needle, the shaft 110 may house an elongated knot pusher tube (not shown; also referred to herein as a "pusher"), which may be actuated using the plunger 140 in some examples. As described in further detail below, the tip 114 provides a surface against which the target valve leaflet may be held in connection with deployment of a leaflet anchor.

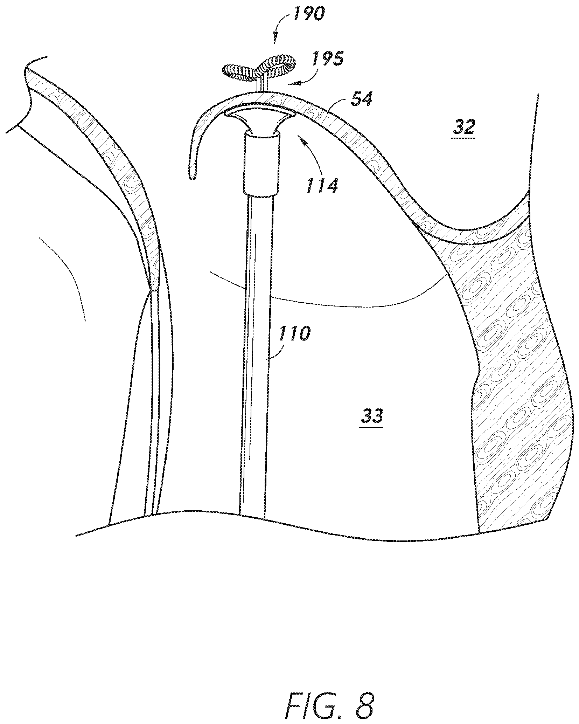

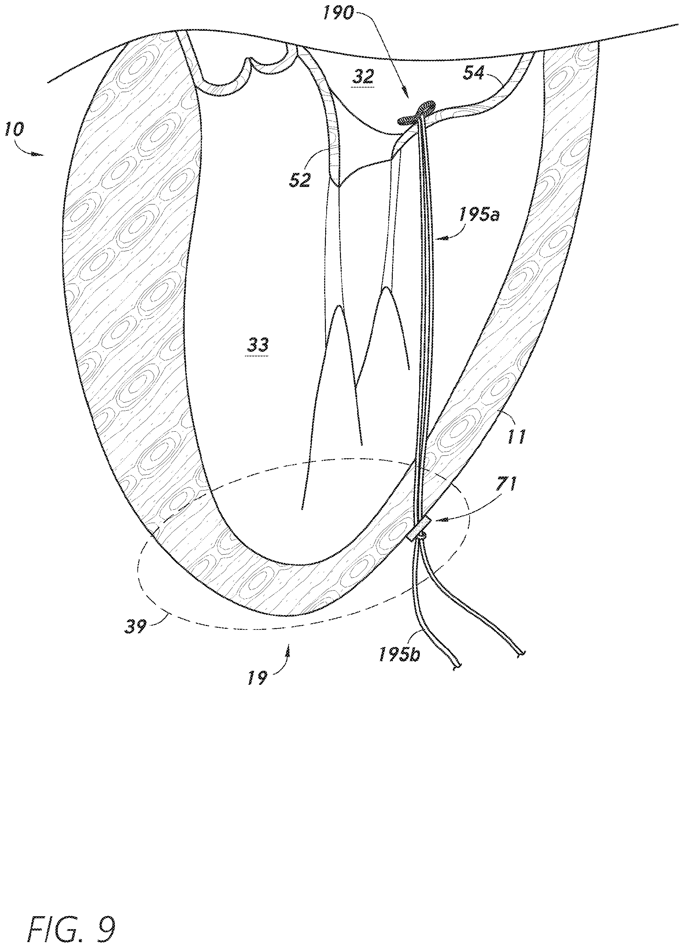

The delivery device 100 may be used to deliver a "bulky knot" type tissue anchor, as described in greater detail below. For example, the delivery device 100 may be utilized to deliver a tissue anchor (e.g., bulky knot) on a distal side of a mitral valve leaflet. The tip 114 (e.g., end effector), can be placed in contact with the ventricular side of a leaflet of a mitral valve. The tip 114 can be coupled to the distal end portion of the shaft 110, wherein the proximal end portion of the shaft 110 may be coupled to a handle portion 120 of the delivery device 100, as shown. Generally, the elongate pusher (not shown) may be movably disposed within a lumen of the shaft 110 and coupled to a pusher hub (not shown) that is movably disposed within the handle 120 and releasably coupled to the plunger 140. A needle (not shown) carrying a pre-formed tissue anchor sutureform can be movably disposed within a lumen of the pusher and coupled to a needle hub (not shown) that is also coupled to the plunger 140. The plunger 140 can be used to actuate or move the needle and the pusher during deployment of a distal anchor (see, e.g., Figures 8 and 9) and is movably disposed at least partially within the handle 120. For example, the handle 120 may define a lumen in which the plunger 140 can be moved. During operation, the pusher may also move within the lumen of the handle 120. The plunger lock 145 can be used to prevent the plunger 140 from moving within the handle 120 during storage and prior to performing a procedure to deploy a tissue anchor.

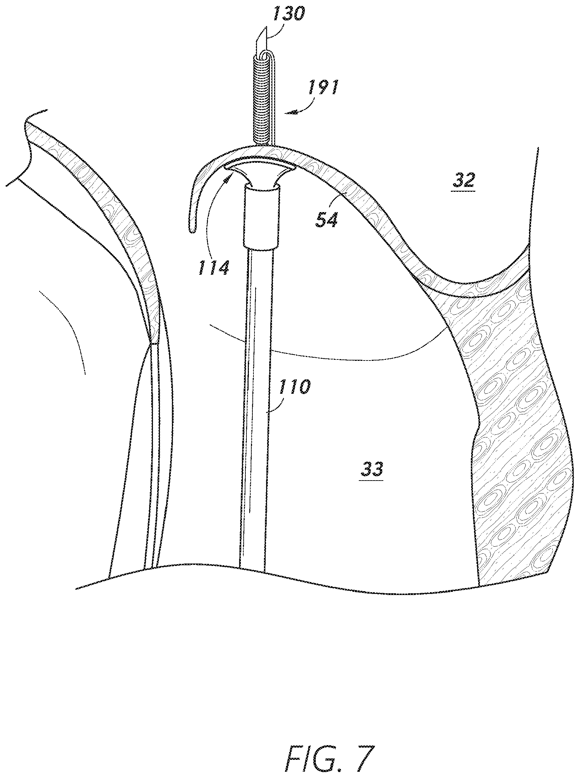

The needle may have the pre-formed knot disposed about a distal portion thereof while maintained in the shaft 110. For example, the pre-formed knot may be formed of one or more sutures configured in a coiled sutureform (see Figure 7) having a plurality of winds/turns around the needle over a portion of the needle that is associated with a longitudinal slot in the needle that runs from the distal end thereof. Although the term "sutureform" is used herein, it should be understood that such components/forms may comprise suture, wire, or any other elongate material wrapped or formed in a desired configuration. The coiled sutureform can be provided or shipped disposed around the needle. In some instances, two suture tails extend from the coiled sutureform. The suture tails 195 may extend through the lumen of the needle and/or through a passageway of the plunger 140 and may exit the plunger 140 at a proximal end portion thereof. The coiled sutureform may advantageously be configured to be formed into a suture-type tissue anchor (referred to herein as a "bulky knot") in connection with an anchor-deployment procedure, as described in more detail below. The coiled sutureform can be configurable to a knot/deployed configuration by approximating opposite ends of the coiled portion thereof towards each other to form one or more loops.

The delivery device can further include a suture/tether catch mechanism (not shown) coupled to the plunger 140 at a proximal end of the delivery device 100, which may be configured to releasably hold or secure a suture 195 extending through the delivery device 100 during delivery of a tissue anchor as described herein. The suture catch can be used to hold the suture 195 with a friction fit or with a clamping force and can have a lock that can be released after the tissue anchor has been deployed/formed into a bulky knot, as described herein.

As described herein, the anchor delivery device 100 can be used in beating heart mitral valve repair procedures. In some instances, the shaft 110 of the delivery device 100 can be configured to extend and contract with the beating of the heart. During systolic contraction, the median axis of the heart generally shortens. For example, the distance from the apex 19 of the heart to the valve leaflets 52, 54 can vary by about 1 centimeter (cm) to about 2 centimeters (cm) with each heartbeat in some patients. In some instances, the length of the shaft 110 that protrudes from the handle 120 can change with the length of the median axis of the heart. That is, distal end of the shaft 110 can be configured to be floating such that the shaft can extend and retract with the beat of the heart so as to maintain contact with the target mitral valve leaflet.

Advancement of the delivery device 100 may be performed in conjunction with echo imaging, direct visualization (e.g., direct transblood visualization), and/or any other suitable remote visualization technique/modality. With respect to cardiac procedures, for example, the delivery device 100 may be advanced in conjunction with transesophageal (TEE) guidance and/or intracardiac echocardiography (ICE) guidance to facilitate and to direct the movement and proper positioning of the device for contacting the appropriate target cardiac region and/or target cardiac tissue (e.g., a valve leaflet, a valve annulus, or any other suitable cardiac tissue). Typical procedures that can be implemented using echo guidance are set forth in Suematsu, Y., J. Thorac. Cardiovasc. Surg. 2005; 130:1348-56 ("Suematsu").

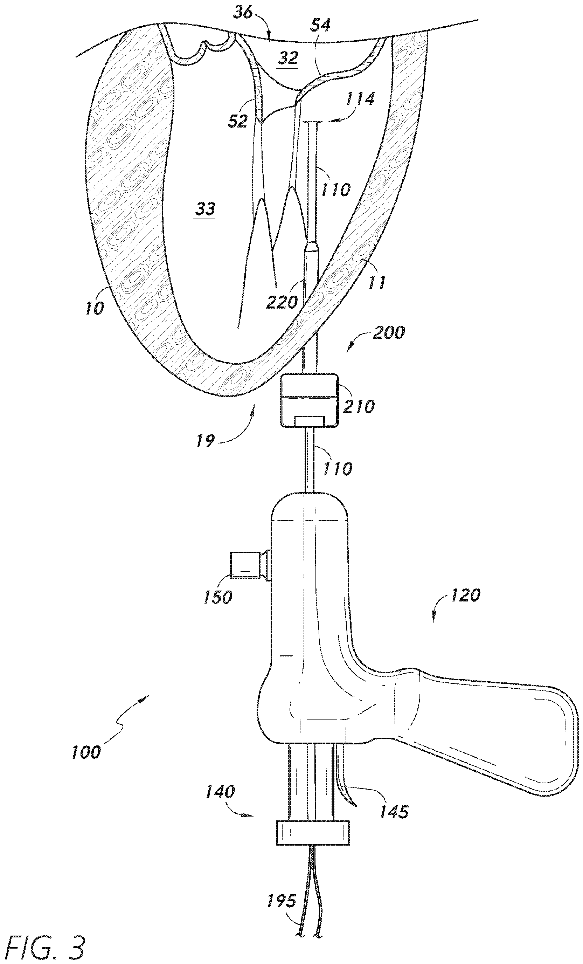

Figure 3 is a cutaway view of a tissue anchor delivery device 100 disposed at least partially within a chamber of a heart in accordance with one or more examples. According to some implementations of valve-repair procedures, an incision into the apical region 39 of the appropriate ventricle 33 of the heart is made. For instance, an introducer port device 200 containing a one or more fluid-retention valves to prevent blood loss and/or air entry into the ventricle 33, may be inserted into the site of entry. Once inside the chamber 33, the shaft 110 of the delivery device 100 may be advanced through the lumen 220 of the introducer 200. In some examples, a sheath may be inserted through the introducer 200, through which one or more other instruments are advanced. For instance, an endoscope may first be advanced into the chamber 33 to visualize the ventricle, the valve 36, and/or the sub-valvular apparatus. By use of an appropriate endoscope, a careful analysis of the malfunctioning valve 36 may be performed. Each segment of each leaflet may be carefully assessed to determine its pliability, integrity, and motion. Based on this assessment, the practitioner can determine whether the valve can indeed be repaired or must be replaced. The motion of the leaflets 52, 54 can be classified as slightly dysfunctional, prolapsed, or restricted and based on this classification, the necessary steps of the repair can be determined.

Mitral valve regurgitation generally increases the workload on the heart and may lead to very serious conditions if left untreated, such as decreased ventricular function, pulmonary hypertension, congestive heart failure, permanent heart damage, cardiac arrest, and ultimately death. Since the left heart is primarily responsible for circulating the flow of blood throughout the body, malfunction of the mitral valve 36 is particularly problematic and often life threatening. Methods and devices are provided herein, as well as in the '761 PCT Application and the '170 PCT Application, for performing non-invasive procedures to repair a cardiac valve, such as a mitral valve. Such procedures include procedures to repair regurgitation that occurs when the leaflets of the mitral valve do not coapt properly at peak contraction pressures, resulting in an undesired backflow of blood from the ventricle into the atrium. As described in the '761 PCT Application and the '170 PCT Application, after the malfunctioning cardiac valve has been assessed and the source of the malfunction verified, a corrective procedure can be performed. Various procedures can be performed in accordance with the methods described therein to effectuate a cardiac valve repair, which will depend on the specific abnormality and the tissues involved.

After a minimally invasive approach is determined to be advisable, one or more incisions may be made proximate to the thoracic cavity to provide a surgical field of access. The total number and length of the incisions to be made depend on the number and types of the instruments to be used as well as the procedure(s) to be performed. The incision(s) may advantageously be made in such a manner as to be minimally invasive. As referred to herein, the term "minimally invasive" means in a manner by which an interior organ ortissue may be accessed with relatively little damage being done to the anatomical structure through which entry is sought. For example, a minimally invasive procedure may involve accessing a body cavity by a small incision of, for example, approximately 5 cm or less made in the skin of the body. The incision may be vertical, horizontal, or slightly curved. If the incision is located along one or more ribs, it may advantageously follow the outline of the rib. The opening may advantageously extend deep enough to allow access to the thoracic cavity between the ribs or under the sternum and is preferably set close to the rib cage and/or diaphragm, dependent on the entry point chosen.

In one example method, the heart may be accessed through one or more openings made by one or more small incision in a portion of the body proximal to the thoracic cavity, such as between one or more of the ribs of the rib cage of a patient, proximate to the xyphoid appendage, or via the abdomen and diaphragm. Access to the thoracic cavity may be sought to allow the insertion and use of one or more thorascopic instruments, while access to the abdomen may be sought to allow the insertion and use of one or more laparoscopic instruments. Insertion of one or more visualizing instruments may then be followed by transdiaphragmatic access to the heart. Additionally, access to the heart may be gained by direct puncture (e.g., via an appropriately sized needle, for instance an 18-gauge needle) of the heart from the xyphoid region. Accordingly, the one or more incisions should be made in such a manner as to provide an appropriate surgical field and access site to the heart in the least invasive manner possible. Access may also be achieved using percutaneous methods, further reducing the invasiveness of the procedure. See, e.g., "Full-Spectrum Cardiac Surgery Through a Minimal Incision Mini-Sternotomy (Lower Half) Technique," Doty et al., Annals of Thoracic Surgery 1998; 65(2): 573-7 and "Transxiphoid Approach Without Median Sternotomy for the Repair of Atrial Septal Defects," Barbero-Marcial et al., Annals of Thoracic Surgery 1998; 65(3): 771-4.

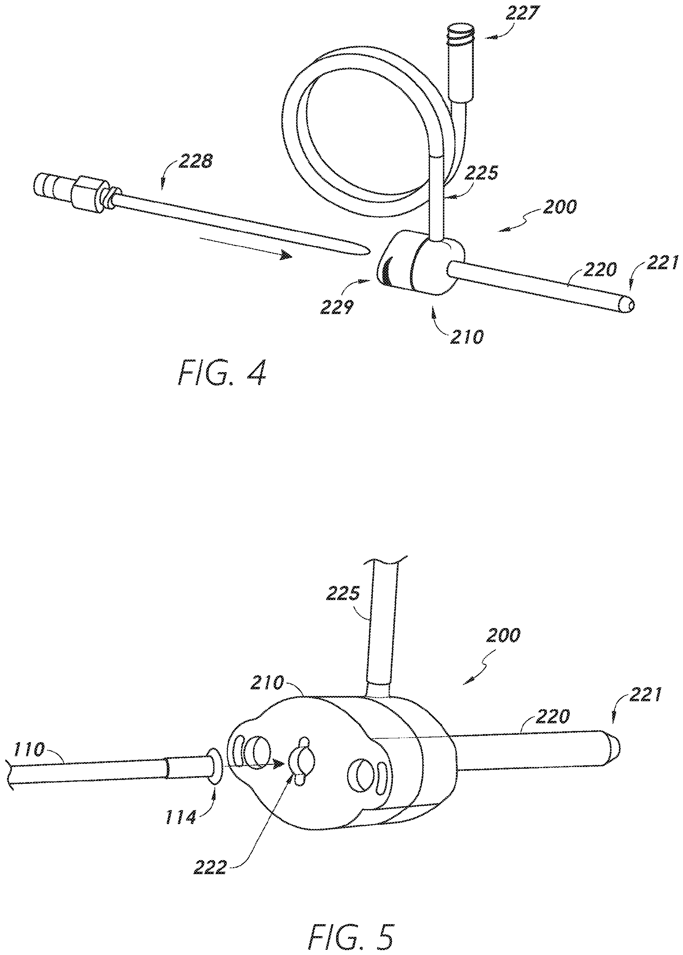

Figure 4 shows a perspective view of a dilator 228 and introducer 200 that may be used in valve-repair procedures in accordance with one or more examples. Figure 5 shows the introducer device 200 and a tissue anchor delivery device shaft 110 in accordance with one or more examples.

The hemostatic introducer 200 may be inserted into the target ventricle at a tip 221 associated with a lumen 220 of the introducer 200. The lumen 220 of the introducer 200 may be used to guide the shaft 110 of a tissue anchor delivery device in accordance with examples of the present disclosure during a valve-repair procedure. The body or hub 210 of the introducer 200 may be used to secure the introducer 200 to the pericardium of the heart for stable entry of the shaft 110 of the tissue anchor delivery device and/or to control the amount of bleed-back during the valve-repair procedure. In some instances, a female Luer may be used to de-air the introducer 200 through a port 225 prior to use and/or to connect a fluid flush, such as a heparin flush, during a valve-repair procedure. The dilator 228 may be used to guide the introducer into the target ventricle. For example, the dilator 228 may be used to guide the introducer 200 into the left ventricle off-apex, as described in detail herein. In some implementations, a tie-down eyelet is used to secure the introducer 200 during the valve-repair procedure.

The introducer lumen 220 provides a conduit into a target surgical area or chamber, such as a ventricle of a heart. In some instances, the introducer 200 comprises one or more hemostasis valves associated with a channel/lumen port 222. Such hemostasis valve(s) may comprise silicone or other flexible material configured to keep blood from flowing out of the channel/lumen port 222. The port 222 may serve as a tissue anchor delivery device lumen insertion port, wherein an inserted delivery device shaft may pass through the lumen 220 of the introducer 200 and out the distal end 221 thereof for access to the target chamber. The port 222 may further be dimensioned to accommodate insertion of the dilator device 228 used to guide the introducer into the target chamber (e.g., left ventricle, off-apex). The distal end 221 of the introducer 200 may have a tapered shape to seal against the delivery system lumen.

The shaft 110 may present a relatively low-profile delivery device, which may be dimensioned to fit within the lumen 220 of the introducer 200. For example, the shaft 110 may be an about 3 mm (9 Fr) shaft. Furthermore, the tip (e.g., end effector) 114 may advantageously be flexible to allow for insertion into the lumen 220 even where the lumen 220 has a smaller diameter than the extended diameter of the tip 114.

Once a suitable entry point has been established, the surgeon can use one or more sutures to make a series of stiches in one or more concentric circles in the myocardium at the desired location to create a "purse-string" closure. The Seldinger technique can be used to access the left ventricle in the area surrounded by the purse-string suture by puncturing the myocardium with a small sharp hollow needle (a "trocar") with a guidewire in the lumen of the trocar. Once the ventricle has been accessed, the guidewire can be advanced, and the trocar removed. The valved introducer 200 (e.g., the introducer 200 with dilator 228 extending through the lumen 220 of the introducer) can be advanced over the guidewire to gain access to the left ventricle. The guidewire (not shown) and dilator 228 can be removed while the introducer 200 maintains hemostasis, with or without a suitable delivery device inserted therein, throughout the procedure. Alternatively, the surgeon can make a small incision in the myocardium and insert the introducer 200 into the heart via the incision. Once the introducer is properly placed, the purse-string suture can be tightened to reduce bleeding around the lumen of the introducer.

A suitable tissue anchor delivery device, such as a delivery device as described in the '761 PCT Application and/or the '170 PCT Application, may be advanced into the body and through the valved introducer in a manner to access the left ventricle. The advancement of the device may be performed in conjunction with echo imaging and/or direct visualization (e.g., direct transblood visualization). For example, the delivery device may be advanced in conjunction with transesophageal echocardiogram (TEE) guidance or intracardiac echo (ICE) to facilitate and direct the movement and proper positioning of the device for contacting the appropriate apical region of the heart. Typical procedures for use of echo guidance are set forth in Suematsu.

In some implementations, after prepping and placing the subject under anesthesia, echo imaging, such as involving TEE (two-dimensional (2D) and/or three-dimensional (3D)), transthoracic echocardiography (TTE), ICE, and/or cardio-optic direct visualization (e.g., via infrared vision from the tip of a 7.5 F catheter), or other imaging modality, may be performed to assess the heart, heart valves, and/or tissue anchor delivery device component(s). For example, echo imaging can be used to guide positioning of tissue anchor(s) (e.g., suture knots) onto a target valve leaflet. However, guidance of an anchor delivery device shaft to the proper position on a mitral valve leaflet as performed under echo guidance can be difficult due, for example, to the level of resolution of the echo guidance technology, and/or equipment or software limitations. As certain mitral valve leaflet repair procedures using tissue anchors, as described herein, can be performed on a beating heart, motion of the beating heart, including the mitral valve leaflets, can also interfere with desired visualization under echo.

Although the procedures described herein are with reference to repairing a cardiac mitral valve or tricuspid valve by the implantation of one or more leaflet anchors and associated cord(s), the methods presented are readily adaptable for various types of tissue, leaflet, and annular repair procedures. The methods described herein, for example, can be performed to selectively approximate two or more portions of tissue to limit a gap between the portions. That is, in general, the methods herein are described with reference to a mitral valve but should not be understood to be limited to procedures involving the mitral valve.

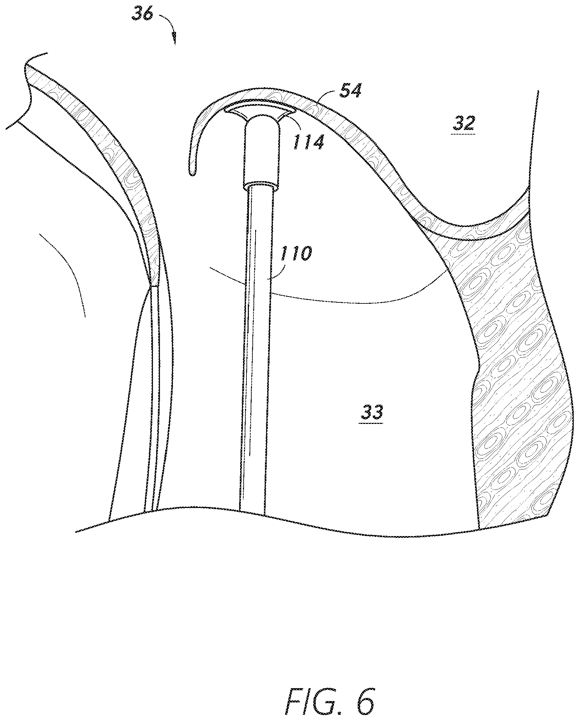

Figure 6 shows a close-up view of a shaft 110 of a tissue anchor delivery device 100 inserted into a ventricle 33 (e.g., left ventricle) and approximated to a target valve leaflet 54 in connection with a valve-repair procedure in accordance with one or more instances of the present disclosure. For example, the valve 36 may be a mitral valve. The anchor delivery device shaft 110 can be configured to deliver a tissue anchor (not shown; see, e.g., Figures 7-9), such as a bulky knot to the valve leaflet 54. As an example, Figure 6 shows a valve leaflet 54, which may represent a posterolateral leaflet of a mitral valve. It will be understood that the anchor delivery device shaft 110 can also deliver a tissue anchor to the anteromedial mitral valve leaflet. Although the description of Figures 6-9 below is presented in the context of a mitral valve, it should be understood that the principles disclosed herein are applicable to other valves or biological tissues, such as a tricuspid valve.

With reference to Figures 3 and 6-9, the anchor delivery device shaft 110 can comprise one or more elongate lumens configured to allow delivery of the anchor 190 to the valve leaflet 54. The shaft 110 can be configured to facilitate performance of one or more functions, such as grasping, suctioning, irrigating, cutting, suturing, or otherwise engaging a valve leaflet. The distal end, or tip, 114 of the shaft 110 can be configured to contact the mitral valve leaflet 54 without substantially damaging the leaflet to facilitate repair of the valve 36. For example, during a valve-repair procedure, a handle (e.g., handle 120) coupled to the shaft 110 can be manipulated in such a manner so that the leaflet 54 is contacted with the functional distal portion of the shaft 110 and a repair effectuated.

Echo imaging guidance, such as transesophageal echocardiogram (TEE) (2D and/or 3D), transthoracic echocardiogram (TTE), and/or intracardiac echo (ICE), may be used to assist in the advancement and desired positioning of the anchor delivery device shaft 110 within the ventricle 33. The distal end 114 of the shaft 110 can contact a proximal surface (e.g., underside surface with respect to the illustrated orientation of Figures 3 and 6-9) of the mitral valve leaflet 54, without or substantially without damaging the leaflet 54. For example, the end/tip portion or component 114 can have a relatively blunt form or configuration. The end/tip portion or component 114 can be configured to maintain contact with the proximal side of the valve leaflet 54 as the heart beats to facilitate reliable delivery of the anchor 1911190 to the target site on the leaflet 54.

In some instances, one or more perforation devices 130 (e.g., needle(s)) can be delivered through a working lumen (not shown) of the shaft 110 to the valve leaflet 54 to puncture the valve leaflet 54 and project a sutureform 191 including a plurality of winds of suture about a distal portion of a needle 130 into the atrium 32 (see Figure 7), wherein the sutureform is deployed to form the bulky knot tissue anchor 190 shown in Figures 8 and 9. For example, as shown in Figure 7, a slotted needle 130 can be deployed from the distal end of the shaft 110, thereby puncturing the leaflet 54 and projecting into the atrium 32, wherein the slotted needle 130 is wrapped with a sutureform 191 (e.g., PTFE suture) in a particular configuration (See the '761 PCT Application for further detail regarding example suture wrapping configurations and needles for use in suture anchor deployment devices and methods). In some instances, a pusher or hollow guide wire (not shown) is provided on or at least partially around the needle 130 within the shaft 110, such that the needle may be withdrawn, leaving the pusher and wound sutureform 191. When a withdrawal force is applied to the sutureform 191 using the pusher, the sutureform 191 may form a bulky-knot-type anchor (e.g., the anchor 190), after which the pusher may be withdrawn, leaving the permanent knot 190 to anchor the suture(s) 195 to the leaflet 54.

Figure 6 shows the shaft 110 of the tissue anchor delivery device 100 positioned on the target valve leaflet 54 (e.g., mitral valve leaflet). For example, the target site of the valve 54 may be slowly approached from the ventricle side thereof by advancing the distal end of the shaft 110 along or near to the posterior wall of the ventricle 33 (e.g., left ventricle) without contacting the ventricle wall. Successful targeting and contacting of the target location on the leaflet 54 can depend at least in part on accurate visualization of the shaft 110 and/or tip/end effector 114 throughout the process of advancing the tip 114 to the target site. Generally, echocardiographic equipment may be used to provide the necessary or desired intra-operative visualization of the shaft 110 and/or tip 114.

Once the tip 114 is positioned in the desired position, the distal end of the shaft 110 and the tip 114 may be used to drape, or "tent," the leaflet 54 to better secure the tip 114 in the desired position, as shown in Figure 6. Draping/tenting may advantageously facilitate contact of the tip 114 with the leaflet 54 throughout one or more cardiac cycles, to thereby provide more secure or proper deployment of leaflet anchor(s). The target location may advantageously be located relatively close to the free edge of the target leaflet 54 to minimize the likelihood of undesirable intra-atrial wall deployment of the anchor. Navigation of the tip 114 to the desired location on the underside of the target valve leaflet 54 may be assisted using echo imaging, as described in detail herein. Echo imaging may be relied upon to confirm correct positioning of the tip 114 prior to anchor/knot deployment.

With the shaft 110 positioned against the target leaflet 54, the plunger 140 of the tissue anchor delivery device 100 can be actuated to move the needle 130 and a pusher disposed within the shaft 110, such that the coiled sutureform portion 191 of the suture anchor slides off the needle 130. As the plunger 140 is actuated, a distal piercing portion of the needle 130 punctures the leaflet 54 and forms an opening in the leaflet. Figure 7 shows a close-up view of the distal portion of the delivery device shaft 110 showing the needle 130 and tissue anchor sutureform 191 projected therefrom through the target leaflet 54 in accordance with one or more examples. In some instances, the needle 130 is projected a distance of about 5-8 mm (about 0.2-0.3 inches), or less, distally beyond the distal end of the shaft 130 (e.g., beyond the tip 114). In some instances, the needle 130 is projected a distance of about 4-10 mm (about 0.15-0.4 inches). In some instances, the needle 130 is projected a distance of about 25 mm (about 1 inch), or greater. In some instances, the needle 130 extends until the distal tip of the needle and the entire coiled sutureform 191 extend through the leaflet 54. While the needle 130 and sutureform 191 are projected into the atrial side 32 of the leaflet 54, the shaft 110 and tip 114 advantageously remain entirely on the ventricular side 33 of the leaflet 54.

As the pusher (not shown) within the tissue anchor delivery device shaft 110 is moved distally, a distal end of the pusher advantageously moves or pushes the distal coiled sutureform 191 (e.g., pre-deployment coiled portion of the suture anchor) over the distal end of the needle 130 and further within the atrium 32 of the heart on a distal side of the leaflet 54, such that the sutureform extends distally beyond a distal end of the needle 130. For example, in some instances, at least half a length of the sutureform 191 extends beyond the distal end of the needle 130. In some instances, at least three quarters of the length of the sutureform 191 extends beyond the distal end of the needle 130. In some instances, the entire coiled sutureform 191 extends beyond the distal end of the needle 130.

After the sutureform 191 has been pushed off the needle 130, pulling one or more of the suture tail(s) 195 (e.g., suture strands extending from the coiled portion of the suture) associated with the tissue anchor 190 proximally can cause the sutureform 191 to form a bulky knot anchor 190, as shown in Figure 8, which provides a close-up view of the formed suture anchor 190 on the atrial side 32 of the leaflet 54. For example, the bulky knot suture anchor 190 may be formed by approximating opposite ends of the coils of the sutureform 191 (see Figure 7) towards each other to form one or more loops. After the sutureform 191 has been formed into the bulky knot 190, the delivery device 100 can be withdrawn proximally, leaving the tissue anchor 190 disposed on the distal atrial side of the leaflet 54, as shown in Figure 9. In some instances, two suture tails 195 may extend from the proximal/ventricle side 33 of the leaflet 54 and out of the heart 1. For example, the delivery device shaft 110 can be slid/withdrawn over the suture tail(s) 195.

Figure 9 shows a cutaway view of the deployed leaflet anchor 190 accordance with one or more examples of the present disclosure. The suture tails 195 coupled to the anchor 190 may be secured at the desired tension using a pledget 71 or other suture-fixing/locking device or mechanism on the outside of the heart through which the suture tails 195 may run. Furthermore, a knot or other suture fixation mechanism or device may be implemented to hold the sutures at the desired tension and to the pledget 71. With the suture tail(s) 195 fixed to the ventricle wall 11, a ventricular portion 195a of the suture tail(s) 195 may advantageously function as replacement leaflet cords (e.g., chordae tendineae) that are configured to tether the target leaflet 54 in a desired manner.

In certain examples, the pledget 71 is a low-porosity and relatively stiff pledget. Such a pledget may advantageously allow for the desired tension of the suture tails 195a to be sustained over an extended post-operative period of time. In some examples, suture tying or fixation may be implemented using one or more soft tissue retractors and/or right-angle clamps, which may be rubber-shod to reduce the risk of damage to the replacement cords.

In certain implementations, testing of location and/or tension of the anchor 190 and/or suture tail(s) 195 may be performed by gently tensioning the suture tails until leaflet motion is felt and/or observed. Echo imaging technology may be used to view and verify the anchor placement and resulting leaflet function. The steps and processes outlined above for placing a suture-knot-type tissue anchor may be repeated as necessary until the desired number of anchors have been implanted on the target valve leaflet. In some implementations, tension adjustment in the suture tail(s)/cord(s) associated with multiple leaflet anchors may be performed simultaneously. The appropriate number of leaflet anchors may advantageously be determined to produce the desired coaptation of the target valve leaflets 54, 52. All deployed leaflet anchors may advantageously be below the surface of coaptation. With respect to posterior mitral valve leaflet repair, the anterior leaflet may advantageously touch the posterior leaflet basal to the leaflet anchor(s). The pledget 71 may be drawn against the epicardial surface, and all the suture tails/cords 195b may be inserted through a tourniquet so that all cords can be tension to the desired effective coaptation together.

In some implementations, one or more leaflet anchors is deployed in each of the mitral valve leaflets, wherein sutures/cords coupled to separate leaflets are secured together in the heart by tying them together with knots or by another suitable attachment device, creating an edge-to-edge repair to decrease the septal-lateral distance of the mitral valve orifice.

With further reference to Figures 2-9, generally, the shaft 110 of the tissue anchor delivery device 100 may be slowly advanced into the introducer 200 until the tip 114 has flushed the introducer 200 and entered the ventricle 33. In so doing, it may be desirable to advance the shaft 110 within the ventricle 33 in such a way as to avoid traversing areas populated by papillary muscles and/or associated chordae tendineae to avoid entanglement therewith. In order to facilitate or ensure avoidance of such anatomy, imaging technology may advantageously be implemented to provide at least partial visibility of the shaft 110 within the ventricle 33, as well as certain anatomical features within the ventricle. With respect to visibility of the shaft 110 in the ventricle 33, echogenic characteristics of the shaft 110 can affect the visibility thereof using echo imaging modalities. Therefore, a shaft having relatively high echogenicity as described in detail herein may advantageously allow for more accurate and/or simplified advancement of the shaft 110, as well as placement of the tip 114 at the target implantation site at the valve leaflet 54 (e.g., an anterior or posterior leaflet of a mitral valve). In some implementations, hybrid imaging technologies may be used, wherein echo imaging is used in combination with a different imaging modality. Multi-imaging modalities may provide improved visibility of anatomical and/or delivery system components.

When relying upon echo imaging, problems and/or difficulties may result from loss of visibility of one or more components of the tissue anchor delivery device, such as loss of visibility of the tip of the anchor-delivery shaft. In such instances, it may be necessary to re-start the anchor-placement procedure. Because the tissue anchor delivery device is inserted through a port in the heart, re-starting delivery of a leaflet anchor can involve additional manipulation on the entry side of the heart, which may result in increased trauma that may have detrimental effects on the cardiac tissue. Examples of the present disclosure advantageously allow for quickly moving the shaft of a tissue anchor delivery device from an introducer to a target location on a target valve leaflet through improved visibility of the delivery shaft and/or other components of the tissue anchor delivery device.

Echo imaging is commonly used with cardiothoracic procedures. For example, for procedures implementing transapical access, rather than intravenous access, in accordance with examples of the present disclosure, fluoroscopy imaging may not provide a practical or tenable solution for imaging. Furthermore, as fluoroscopy can involve exposure of the patient to radiation, such imaging may be undesirable in connection with valve-repair procedures in some situations. Therefore, echo imaging can provide a preferable solution for visibility in valve-repair procedures in accordance with examples of the present disclosure. However, for tissue anchor delivery devices comprising relatively low-echogenicity shafts and/or other components, such components may produce only relatively faint images thereof under echo. Therefore, images of anchor delivery device shafts can become blended or obscured with/by background anatomy and/or images. Therefore, examples of the present disclosure that provide improved echogenicity and/or imaging visibility for components of a tissue anchor delivery device may allow for relatively easy navigation of the delivery shaft from the port of entry into the target ventricle to the target valve leaflet(s) (e.g., mitral valve leaflets). Furthermore, such improved visibility may facilitate avoidance by the operator of anatomy or areas in which the delivery shaft and/or associated components of the tissue anchor delivery device may become entangled or caught. For example, examples of the present disclosure may facilitate avoidance of native chordae tendoneae, thereby reducing the risk of injury or damage to native anatomy/tissue. Furthermore, improved visibility of anchor delivery shafts and/or other components of the tissue anchor delivery device may facilitate improved targeting of the target leaflet, which may result in improved coaptation and/or results from valve repair intervention.

### Echogenic Surfaces/Volumes

Examples of the present disclosure provide echogenic shafts and/or associated components of tissue anchor delivery devices for certain medical procedures, such as mitral valve repair procedures, which may improve health prospects of patients suffering from degenerative mitral regurgitation. As described in detail above, such procedures may involve transapical, off-pump, echo-guided repair, wherein a tissue/leaflet anchor delivery device shaft is inserted in the left ventricle and steered to the surface of the diseased portion of the target mitral valve leaflet for deployment/implantation of a leaflet anchor, such as a suture-knot-type anchor, which may advantageously be coupled to one or more suture tails that provide artificial leaflet cord functionality. Such sutures may comprise expanded polytetrafluoroethylene (ePTFE) in some instances.

In accordance with leaflet anchor deployment processes outlined herein, a delivery device shaft can be passed through an introducer device and advanced inside the ventricle under echo (e.g., ultrasound) guidance. Such imaging implementation may utilize, for example, Transthoracic Echocardiography (TTE) probe device or a Transesophageal Echocardiography (TEE) probe device. However, it should be understood that features disclosed herein may be applicable to any type of imaging devices/modalities.

Figure I0A shows a side view of a TTE imaging probe 69 disposed adjacent to a chest wall 60 of a patient in accordance with one or more examples. Figure I0A shows a medical device shaft 110 inserted into a heart 10 for deployment of a tissue anchor, as described herein. For example, the shaft may be configured to transport and deploy a needle in a lumen thereof, the needle having a distal portion that is wrapped with suture or wire in a coiled preformed knot sutureform/configuration. With the shaft 110 positioned against a diseased prolapsed valve leaflet, or other tissue, the delivery device (not shown) associated with the shaft can be actuated to puncture the leaflet, push the coiled sutureform through the leaflet for deployment thereof on the atrial side of the leaflet. For example, the coiled sutureform may be formed into a relatively large-width/diameter bulky knot on the atrial side of the mitral valve leaflet. The length of the suture tail(s) associated with the knot anchor can be adjusted under real-time echo guidance, and the proximal ends of the suture tails can be secured to the outer ventricular wall of the heart.

In order to reliably navigate through the ventricle and place the leaflet anchor(s) in precise targeted locations on the target segment of the mitral valve, it may be necessary or desirable for the ultrasound image of the anchor delivery device shaft to be clearly visible in relation to the surrounding structures. However, during surgery, the appearance of a medical device shaft can be faint, blurry, and come in-and-out of focus in the field of view, thereby providing a misleading and inaccurate impression of the shape and/or location of the shaft or create confusion between the shaft and artifact(s). Specifically, when the echogenicity of the shaft 110 during its operation is not relatively high, tracking and visualization of the shaft can be very challenging.

Examples of the present disclosure provide medical device shafts having relatively high echogenicity in order to improve visibility during surgical procedures. Examples of the present disclosure may implement any suitable or desirable echogenicity-enhancement features (also referred to herein as "image-enhancement" features) in medical device shafts and/or other devices. For example, devices and surfaces having echogenicity-enhancement features in accordance with the present disclosure can include texturing or roughening of device surfaces, adhering particles to device surfaces, utilization of reflectors, creating indentations or holes in the device/surface, using dissimilar materials in a device, and other image-enhancement mechanisms or techniques.

Echocardiography utilizes sonic imaging to provide sonogram images of the heart, or portion thereof. Generally, echocardiography can utilize two-dimensional, three-dimensional, and/or Doppler ultrasound to create images of the heart, including real-time images. In some implementations, echocardiography can involve the use ultrasonic pulse echo imaging, or other technology. With respect to Figure I0A, the echo imaging transducer or probe 69 may be placed on the chest wall 60 of the patient, wherein images are taken through the chest wall 60. Such echocardiography may be referred to as transthoracic echocardiography. Systems like that shown in Figure I0A may advantageously provide a noninvasive, real-time imaging of the heart 10 during a valve-repair procedure as described herein.

In some implementations, valve-repair imaging in accordance with examples of the present disclosure may incorporate three-dimensional (3D) echocardiography technology, which may implement a matrix array ultrasound probe in combination with appropriate processing system. Such imaging modality may enable detailed anatomical assessment of cardiac pathology, including valvular defects and cardiomyopathies. Real-time 3D echocardiography can be used to guide the location of a tissue anchor delivery device shaft, as described in detail herein, within a chamber of the heart, such as the left ventricle for mitral valve leaflet repair. Therefore, echo imaging may be used to provide intraoperative assessment of cardiac anatomy and repair system components).

In Figure I0A, the inserted shaft 110 is inserted at an angle *Θ* with respect to a surface plane 67 of the probe/transducer 69; sound waves transmitted by the transducer 69, such as the wave 61, may generally propagate orthogonally (e.g., at a right angle) with respect to the surface plane 67. For non-echogenic medical device shafts, the angle of reflection of sound waves may generally equal the angle of incidence with respect to the shaft surface. For example, as shown in Figure I0A, for a non-echogenic shaft, a transmitted sound wave 61 from the transducer 69 may be reflected in accordance with the angle of incidence with respect to the angled shaft orientation shown in Figure I0A, resulting in the reflected wave 65 shown. The reflected wave 65 may be generally directed at least partially away from the transducer 69, thereby producing an undesirably-attenuated reflected signal 65. Imaging visualization may generally be greater when the reflected signal is directed directly back to the transducer, such that the angle of incidence is at or near 0°. In some examples, the present disclosure provides medical device shafts and devices including at least partially fluid/gas-filled chambers or lumens (or surfaces, chambers, and/or lumens having solid media with similar echogenicity characteristics), wherein the fluid/gas serves to provide multifaceted reflectors that reflect a substantial portion of sound waves incident thereon directly back to the signal source (e.g., echo transducer) 69, thereby providing a relatively clear and more defined image, as shown by the reflected signal 63. Such devices may comprise multi-lumen shafts, wherein one or more lumens thereof are at least partially filled with fluid (e.g., air or other gas) or other echogenic media.

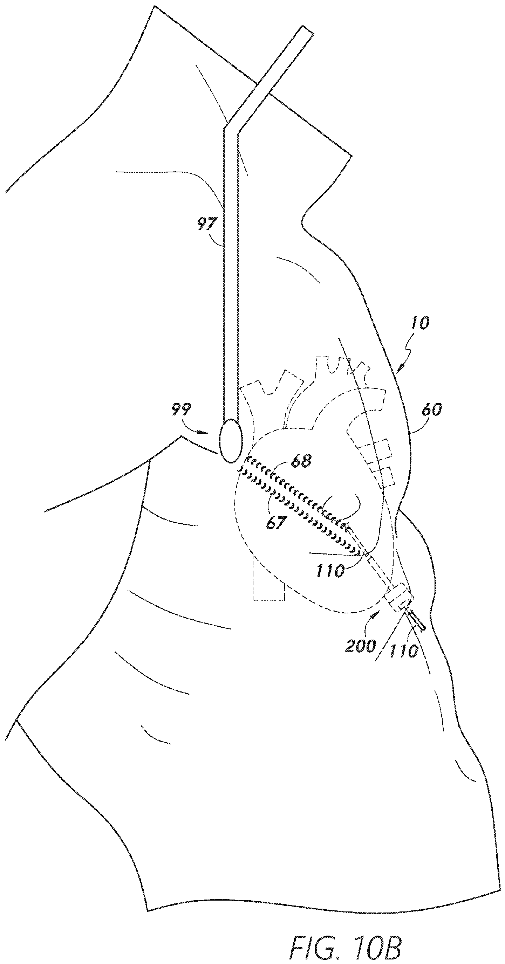

Figure 10B shows a side view of a transesophageal echocardiography (TEE) imaging probe 99 implemented to generate echo image. The TEE implementation shown in Figure 10B represents a relatively more invasive form cardiac ultrasound (e.g., echo), wherein the ultrasound transducer/probe 99 is placed down the throat of the patient and into the esophagus. Image generation from a position within the patient's esophagus may provide a relatively more detailed, clearer imaging of the heart 10 and medical device shaft 110 than may be typical using transthoracic echocardiography (TTE) imaging. For example, the position of the probe 99 may allow for clearer imaging of certain soft tissue that is not visible in some TTE implementations. When the shaft 110 comprises echogenic contrast media and/or image-enhancement lumen(s), as disclosed in detail herein, at least a portion of the transmitted signals 67 may be reflected substantially directly back to the transducer 99, such as shown by the reflected signals 68. Disclosure of echo imaging generation herein may involve TTE, TEE, or any other type of echo imaging generation.

### Multi-Lumen Medical Device Shafts

Multi-lumen shafts in accordance with examples of the present disclosure may be used in association with contrast echocardiography to provide improved imaging and/or visibility during valve-repair procedures, or other medical interventions. For example, in some instances, ultrasound contrast medium, or imaging agent, may be contained within a lumen or chamber of a medical device shaft and/or flow therethrough during a valve-repair procedure to provide improved imaging/visibility.

The degree to which materials and media reflect soundwaves may be referred to as "echogenicity," as described and used herein in accordance with its broad and ordinary meaning, which generally refers to the ability of a material or surface to bounce an echo (e.g., return a sound/ultrasound signal). That is, echogenicity may be considered higher when the surface bouncing the sound echo reflects increased sound waves. Materials/surfaces having relatively higher echogenicity may be referred to as "hypoechogenic," or simply "echogenic," and may usually present visually with lighter colors on echo imaging. In contrast, materials/surfaces with relatively lower echogenicity may be referred to as "hypoechogenic," or "non-echogenic," herein.

Anchor delivery shafts in accordance with instances of the present disclosure may have improved echogenicity characteristics through the use of one or more chambers or lumens having disposed therein certain gas/fluid and/or other media or surface characteristics that provide increased reflection of sound waves. For example, examples disclosed herein provide multi-lumen shafts, wherein one or more lumens or chambers thereof are filled with gas or other contrast media. Such gas/media may provide a density interface between the shaft and the gas/fluid disposed in one or more lumens thereof. For example, a shaft in accordance with examples of the present disclosure may comprise a substantially solid/rigid material, such as stainless steel or the like, that has one or more portions thereof that are filled with fluid or media that is less dense than the solid/rigid material of the shaft, thereby providing a reflection interface between the materials.

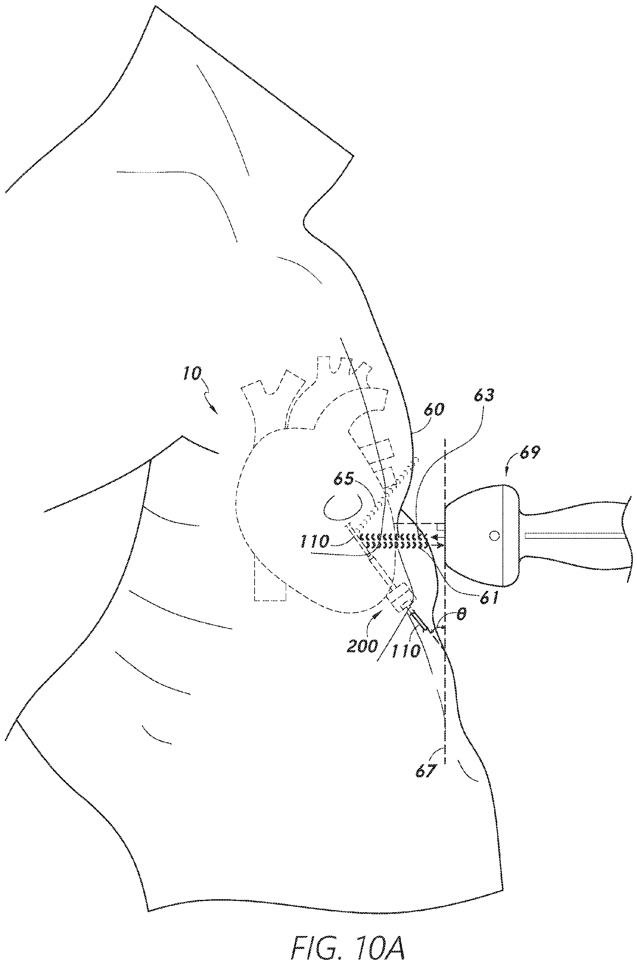

With reference back to Figure 10, for gases having echogenic characteristics, such as air, or microbubble-type or other contrast media, the angle of reflection of soundwaves thereby may be substantially equal to the angle of incidence. Generally, visualization is best when the angle of incidence of the reflected wave 63 with respect to the transducer surface 67 is 0°, whereas visibility may be relatively less with respect to signals reflected away from the transducer. The use of gas or other contrast media in medical device shafts in accordance with examples of the present disclosure may advantageously provide improved imaging, wherein gas or other contrast media disposed inside one or more lumens or chambers of the shaft act as multifaceted reflectors that continuously and perpendicularly reflect sound waves (e.g., ultrasound waves) back to the wave source (e.g. ultrasound probe 69), thereby providing a relatively clear and more-defined images. Therefore, as shown in Figure 10, a shaft 110 being disposed at an angle fJ with respect to an angle of incidence of an ultrasound wave 61 provided by the ultrasound probe 69 may comprise air/gas or other contrast media that reflects the signal 61 at an angle substantially equal to the angle of incidence of the ultrasound waves on the amorphous media, which may provide improved reception by the ultrasound transducer 69 compared to signals 65 reflected by the surface of the shaft 110, which may generally reflect at an angle equal to the angle of incidence on the shaft. That is, regardless of what angle the shaft 110 is presently oriented at with respect to the ultrasound probe 69, certain reflected echoes may be reflected back directly to the source of the signal, which advantageously includes the receiving functionality in addition to the transmission functionality. Such reflective qualities may serve to reduce or eliminate the presence of blind spots in echo images with respect to portions of the shaft 110 that are associated with gas or other contrast media as described herein. Although examples of the present disclosure describe gas-filled chambers or lumens of anchor delivery device shafts, it should be understood that liquid media may be used in addition to, or as an alternative to, gas.

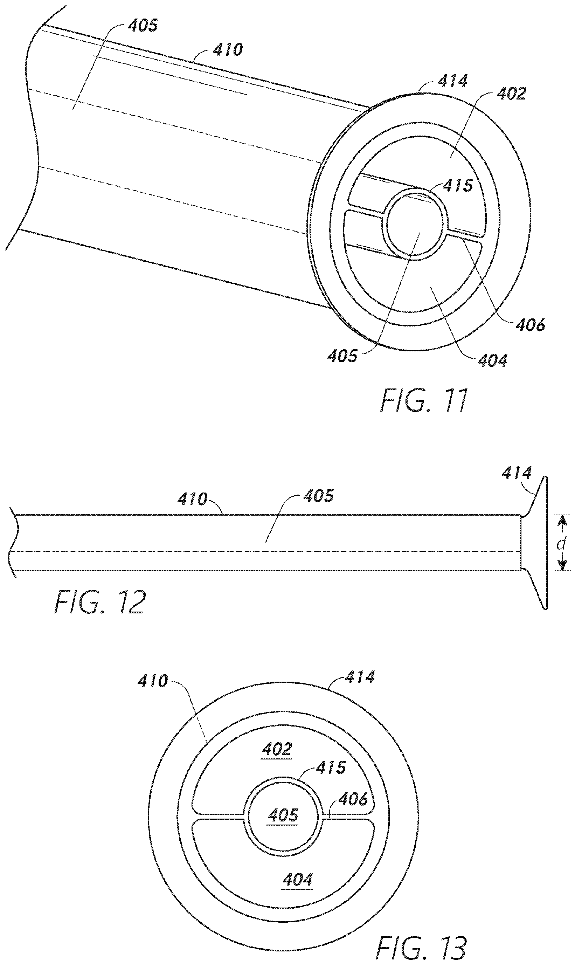

Examples of echogenic medical device shafts in accordance with the present disclosure may advantageously comprise one or more fluid-/media-filled image-enhancing lumens or chambers, as well as one or more working lumens for transporting repair devices and/or tools in connection with a medical procedure. Some examples of the present disclosure relate to multi-lumen anchor-delivery shafts that provide improved echogenicity characteristics compared to certain other tissue anchor deployment solutions. Figure 11 shows a perspective view of a distal portion of a multi-lumen medical device shaft 410 in accordance with one or more examples. The shaft 410 may comprise a rigid elongate tube forming at least one internal lumen, as shown. Figure 12 shows a side view of the multi-lumen medical device shaft of Figure 11, while Figure 13 shows a front axial view of the multi-lumen medical device shaft 410 of Figure 11. The shaft 410 of Figure 11 may comprise one or more image-enhancement lumens/chambers designed to continuously reflect ultrasonic energy back to the signal source (e.g., echo transducer). For such purpose, the shaft 410 includes fluid-filled lumens 402, 404, in addition to the working lumen 405. The fluid-filled lumens 402, 404 can act as resonators along at least a portion of the longitudinal length of the shaft 410. The fluid in the lumens 402, 404 may advantageously optimize the angle of incidence between ultrasound waves incident on the shaft 410, allowing the sound waves to continually reflect back to the signal source orthogonally/perpendicularly regardless of the angle at which the shaft is oriented with respect to the signal source. The enhanced echogenicity provided by multi-lumen shafts as disclosed herein can provide improved safety and efficacy of, for example, heart valve repair procedures.

The working lumen 405 may be used to encapsulate needle and pusher components of a tissue anchor delivery device, as described in detail above. In some examples, the working lumen 405 may be disposed generally centrally about a longitudinal central axis of the shaft 410, whereas one or more of the image-enhancement lumens 402, 404 may be dispose in the periphery around at least a portion of the working lumen 405 and adjacent thereto, as shown in Figures 11-13.

The shaft 410 is a multi-lumen shaft, wherein one or more lumens associated with the shaft are to be shaped and/or configured to receive and/or contain echogenic contrast media/gas, as described in detail herein. The shaft 410 of the illustrated embodiment of Figure 11 includes a working lumen 405, through which a leaflet anchor may be advanced to a target implantation site. Furthermore, the working lumen 405 may be used to hold and/or advance sutures of, or associated with, a leaflet anchor, as well as a needle and/or other tool(s) for facilitating deployments of one or more leaflet anchors. In some examples, the central lumen 405 is configured to encapsulate one or more of a needle, a coiled sutureform anchor, and a pusher component of the tissue anchor delivery device, as described above. The lumens 402, 404 may be configured to contain air, or other gas or media contrast fluid, as described in detail herein. In some examples, the proximal and/or distal ends of the lumens 402, 404 may be closed-off, such that the lumens 402, 404 are effectively fluid-tight. Therefore, the lumens 402, 404 may hold substantially stagnant fluid therein, and may advantageously be at least partially fluid-tight, such that blood and/or other environmental fluids or solids cannot enter therein during performance of a surgical operation.

The shaft 410 may be associated with or coupled to a substantially atraumatic leaflet-contact tip 414, also referred to herein as an "end effector." The tip 414 may advantageously be at least partially flexible, such that during insertion of the shaft 410 through an introducer device to gain access to the target heart ventricle, the tip 414 may be configured to flex backward in the direction of the proximal end of the shaft 410, such that the channel of the introducer through which the shaft 410 is advanced need not necessarily be as large in diameter as the tip 414. Through use of a flexible leaflet-contact tip 414 and appropriately dimensioned lumens 402, 404, 405, the shaft 410 of Figure 11 may advantageously be relatively small-profile, which may allow for a relatively greater range of movement of the shaft 410 within the target ventricle and/or other anatomical chamber(s). As called-out in Figure 12, which shows a side view of the distal portion of the shaft 410 illustrated in Figure 11, the diameter of the shaft 410 may be approximately 3.048 mm (.120 inches). Figure 13 shows a straight-on view of the distal end of the shaft 410 and associated component(s) shown in Figures 11 and 12.

With one or both of the lumens 402, 404 having disposed therein air or other gas or contrast media that has relatively high echogenicity characteristics, the shaft 410 may appear relatively brighter visually in an echocardiography image compared to a similar shaft not having such additional lumen(s). The shaft 410 may have any size or shape. In some examples, the multi-lumen shaft may be formed using an extrusion process.

The embodiment of Figures 11-13 represents an example of a multi-lumen shaft with two kidney-shaped outer lumens 402, 404 positioned on the outside of a central working lumen 405. However, fluid-filled echogenic lumens in accordance with examples of the present disclosure may have any suitable or desirable shape, form, or configuration relative to a working lumen. In some examples, such as in the embodiment shown in Figures 11-13, outer echogenic lumens 402, 404 can be located in the periphery around the central working lumen 405. In some examples, the echogenic lumens 402, 404 are not open to external fluid. There may generally be no fluid communication between the fluid-filled echogenic lumen(s) and the working lumen. Therefore, in some examples, trapped air or other fluid in the lumens 402, 404 is fully contained and does not leak, or penetrate through and crossover to the working lumen 405. The multi-lumen shaft 410 of Figure 11-13 can be made of stainless steel or any other medically-compatible metals, such as titanium, steel (DUMOXEL^{®}, DUMOSTAR^{®}; Dumont, Montignez, Switzerland) any other metal alloys, or resins or polymers used to make surgical instruments.

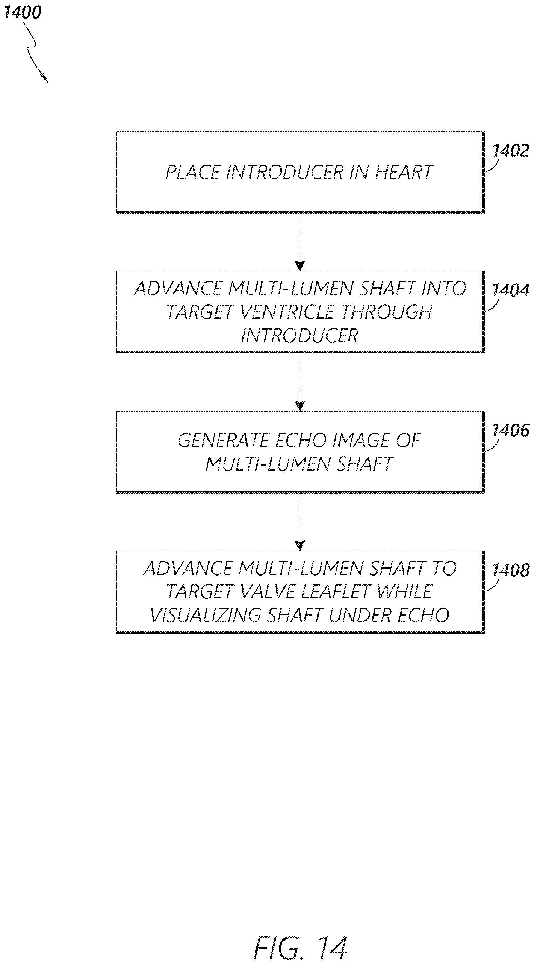

Figure 14 is a flow diagram illustrating a process 1400 for targeting a heart valve leaflet using a multi-lumen shaft in accordance with one or more examples. The process 1400 may be implemented to advance a multi-lumen shaft of a tissue anchor delivery device to a target heart valve leaflet in accordance with examples of the present disclosure. At block 1402, the process 1400 involves placing an introducer device with a lumen thereof projecting through a heart wall and into a ventricle of the heart. The introducer device may advantageously have one or more valves for preventing backflow of blood from the ventricle through the introducer device lumen.

At block 1404, the process 1400 involves advancing a multi-lumen shaft of the tissue anchor delivery device through the lumen of the introducer device to thereby introduce a distal portion of the multi-lumen shaft into the ventricle of the heart. The multi-lumen shaft may advantageously comprise one or more echogenic at least partially fluid-filled lumens. Such lumens may be closed-off to outside fluids, such that the lumens are fluid-tight, or may allow for fluid flow through and out the echogenic lumen(s).

At block 1406, the process 1400 involves generating an image of the multi-lumen shaft using echo imaging, as described in detail herein. For example, such imaging may be generated using an echo transducer/probe, which may be placed or disposed at or near the chest of the patient. Compared to traditional shafts, which can be associated with echo images that are faint, blurry, and/or come in-and-out of focus in the field of view, and thereby mislead the surgeon and echo cardiographer, the multi-lumen shaft may provide a clearer echo image. At block 1408, the process 1400 involves further advancing the multi-lumen shaft while visualizing the shaft on the echo image to contact a target valve leaflet, as described in detail herein.

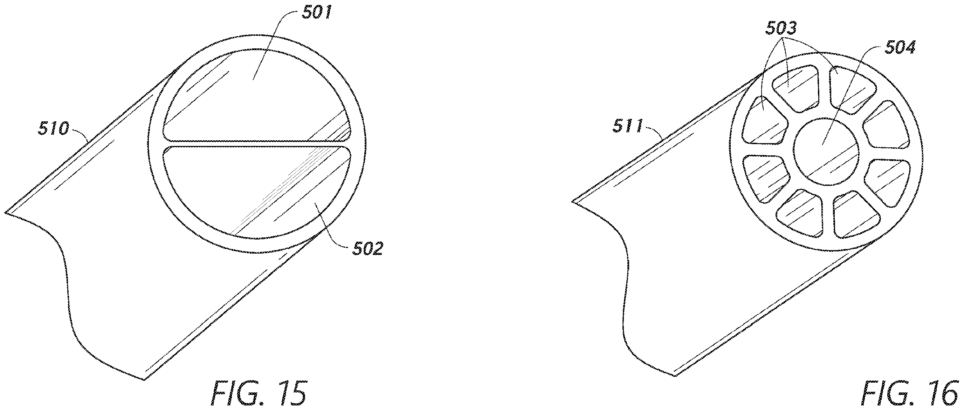

Figures 15-18 illustrate example embodiments of multi-lumen echogenic shafts in accordance with the present disclosure. Although various shapes and configurations of multi-lumen shafts are illustrated in Figures 15-18, it should be understood that embodiments of the present disclosure may comprise any shape, configuration, and/or number of fluid-filled image-enhancement lumens and/or working lumens. Although the fluid-filled image-enhancement lumens shown in Figures 15-18 are illustrated as being open, it should be understood that in some instances, such lumens may be closed off, such that fluid may neither enter nor exit the lumens when disposed in a target ventricle or other anatomy or chamber. Furthermore, although working lumens of the shafts in Figures 15-18 are illustrated as being generally centrally disposed or positioned with respect to the cross-sectional area of the shafts, it should be understood that in some instances, working lumen(s) may be disposed generally on the outside of the cross-sectional area of the shaft, or in any area that is not in-line or concentric with the central longitudinal axis of the shaft. Furthermore, addition fluid-filled image-enhancement lumens may be disposed generally in an outer area of the cross-sectional area of the shaft or may be centrally disposed. With respect to each of the examples of Figures 15-18, it may be advantageous for fluid-filled image-enhancement lumens associated therewith to be configured such that there is no fluid communication between such lumen(s) and any working lumens associated therewith over the length of the shaft.

Figure 15 is a perspective view of a distal portion of a multi-lumen medical device shaft 510 in accordance with one or more examples. The shaft 510 may comprise a rigid elongate tube forming at least one internal lumen, as shown. Figure 15 illustrates an embodiment of a shaft 510 comprising two lumens 501, 502. In some examples, one of the two lumens 501, 502 may be utilized as a working lumen, as described herein, while the other of the lumens may be used as a fluid-filled image-enhancement lumen. The lumens 501, 52 may have semicircular shapes, as illustrated, or may have any other shape. In some examples, the lumens 501, 502 are substantially equal in size or volume, whereas in other examples, one of the lumens 501, 502 may be greater in size or volume than the other. As shown, the image-enhancement lumen may generally be positioned adjacent to the working lumen.

Figure 16 is a perspective view of a distal portion of a multi-lumen medical device shaft 511 in accordance with one or more examples. The shaft 511 may comprise a rigid elongate tube forming at least one internal lumen, as shown. Figure 16 illustrates an embodiment of a shaft comprising a central working lumen 504 and a plurality of outer fluid-filled image-enhancement lumens 503. For example, a multi-lumen shaft in accordance with the present disclosure may comprise a substantially circular-cross-sectional working lumen 504, whereas fluid-filled image-enhancement lumens 503 may have a non-circular cross-sectional shape. Such a configuration may advantageously efficiently utilize the cross-sectional area of the lumen 511 to thereby allow for a greater volume of image-enhancement fluid/media. As shown, the image-enhancement lumens 503 may generally be positioned adjacent to the working lumen 504.

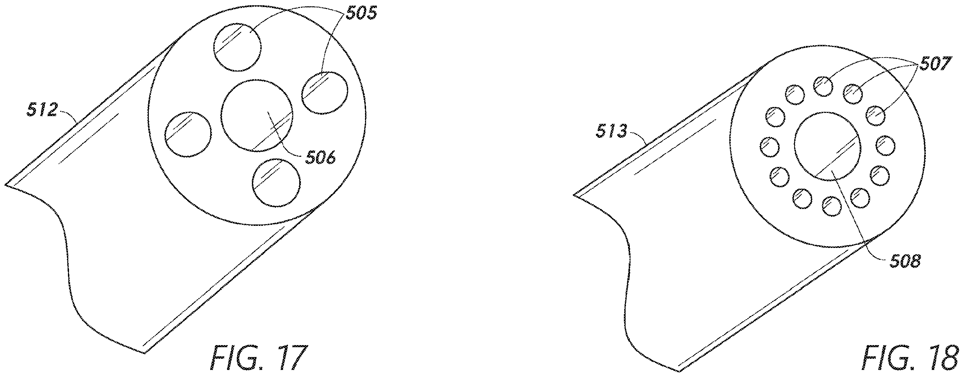

Figure 17 is a perspective view of a distal portion of a multi-lumen medical device shaft 512 in accordance with one or more examples. The shaft 512 may comprise a rigid elongate tube forming at least one internal lumen, as shown. The shaft 512 comprises a central working lumen 506 and a plurality of circular image-enhancement lumens 505. In the embodiment of Figure 17, the shaft 512 comprises four circumferentially spaced image-enhancement lumens 505. With respect to Figure 18, the illustrated shaft 513 comprises a central working lumen 508 and a plurality of image-enhancement lumens 507, which may include more than four image-enhancement lumens in some examples. With respect to the examples of Figures 17 and 18, the image-enhancement lumens 505, 507 may generally be smaller in cross-sectional area than the central working lumens 506, 508, respectively. As shown, the image-enhancement lumens 505, 507 may generally be positioned adjacent to the working lumen 506, 508.

The shafts of Figures 15-18 may be manufactured or formed in any suitable or desirable way. For example, in some implementations, a multi-lumen shaft similar to those represented in Figures 15-18 may be made using an extrusion process. For example, such shafts may be made using any material, such as polymer or other material suitable for single-form extrusion. Specifically, an extrusion process may be utilized to produce a continuous line of tubing having a plurality of lumens running therethrough. Such tubing may be cut into discrete units that are used for valve-repair system shaft components. In some examples, it may be desirable for a central working lumen to be more rigid and/or have characteristics that are not generally characteristic of extruded tubing. Therefore, a stainless steel or other relatively rigid lining or tube may be incorporated in an extruded multi-lumen structure. That is, in some examples, a multi-lumen shaft can include different sections or portions that comprise different materials. For example, a stainless-steel liner or tube may be used in connection with the working lumen and/or one or more other lumens, while one or more lumens may be formed using an extrusion process designed to form a sleeve or other component(s) that can slide over or otherwise engage with one or more other lumens/shafts.

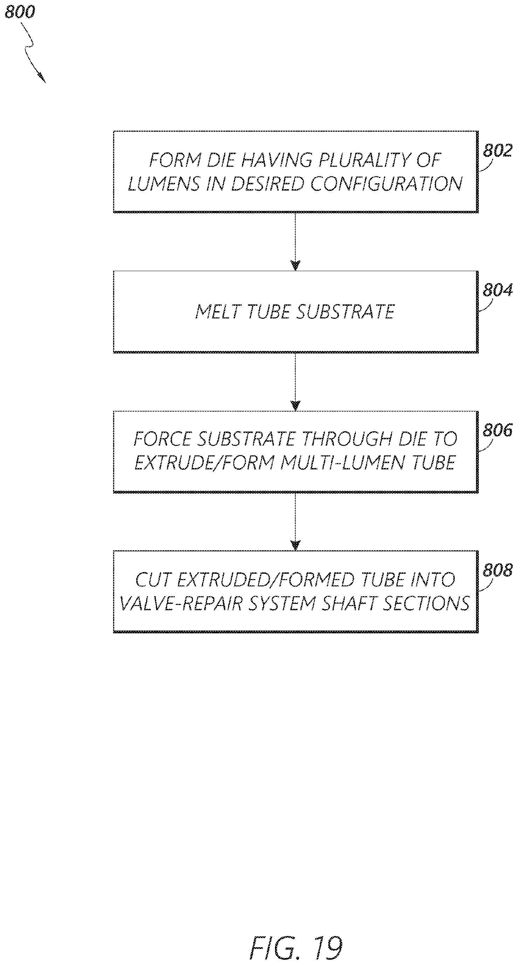

Figure 19 is a flow diagram illustrating a process 800 for manufacturing a multi-lumen medical device shaft using an extrusion process in accordance with one or more examples. At block 802, the process 800 involves forming a die having a plurality of lumens in a desired configuration. For example, the die may include a plurality of axially-arranged lumens in a substantially-circular arrangement about the axis. Additionally or alternatively, the die may include one or more lumens having any suitable or desirable non-circular cross-sectional shape.

At block 804, the process 800 involves melting the material that is used as the substrate for the multi-lumen shaft. For example, a polymer/plastic (e.g., thermoplastic), or any other suitable or desirable material may be melted using a hopper or other type of machinery or mechanism. At block 806, the process 800 involves forcing the melted material through the die to form the desired multi-lumen shaft/tube. At block 808, the process 800 involves cutting the extruded/formed tube into one or more sections, each of which may serve as a shaft component of a tissue anchor delivery device in accordance with examples of the present disclosure. The extruded tube may be permitted to cool prior to cutting in some implementations. The process 800 may optionally further involve coupling the extruded tube section to a relatively-rigid shaft/lumen, such as a stainless-steel shaft. For example, the extruded tube may be slid or otherwise engaged around the stainless-steel shaft. Certain shafts disclosed herein may advantageously comprise stainless steel, which may provide suitable or desirable strength and/or density, as well as echogenicity characteristics. Furthermore, stainless-steel shafts and/or components thereof may advantageously provide a relatively stable platform for deploying leaflet anchors (e.g., bulky knot anchors), and furthermore, stainless steel may have desirable biocompatibility characteristics as well.

Although various examples are disclosed herein of multi-lumen shafts that comprise a single integrated form, in some examples, a multi-lumen shaft may comprise a plurality of lumens or shafts having separate tubes that are coupled together in some manner, wherein one or more of the tubes is used as a working lumen and one or more of the tubes is used as a fluid-filled image-enhancement lumen.

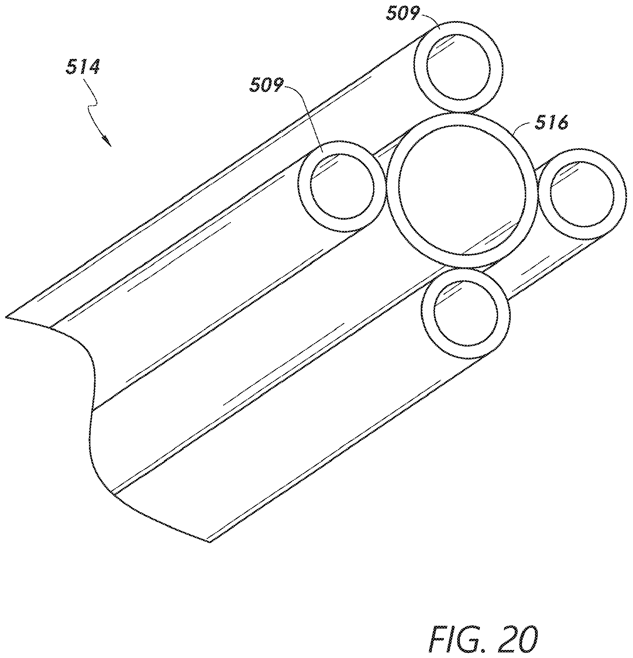

Figure 20 is a perspective view of a distal portion of a multi-lumen medical device shaft 514 in accordance with one or more examples. In particular, Figure 20 illustrates a multi-lumen shaft 514 comprising a central working lumen 516 and one or more outer lumens 509. In some implementations, the central lumen 516 is utilized as a working lumen, through which anchor deployment devices and/or components may be advanced and deployed whereas the outer lumens 509 are fluid-filled image-enhancement lumens. In some examples, the central lumen 516 and outer lumens 509 may comprise separate lumens, such as stainless-steel lumens, polymer lumens, and/or the like, which are joined together in some manner, such as through welding, adhesion, or through the use of one or more mechanical fasteners or straps. As shown, the image-enhancement lumens 509 may generally be positioned adjacent to the working lumen 516.

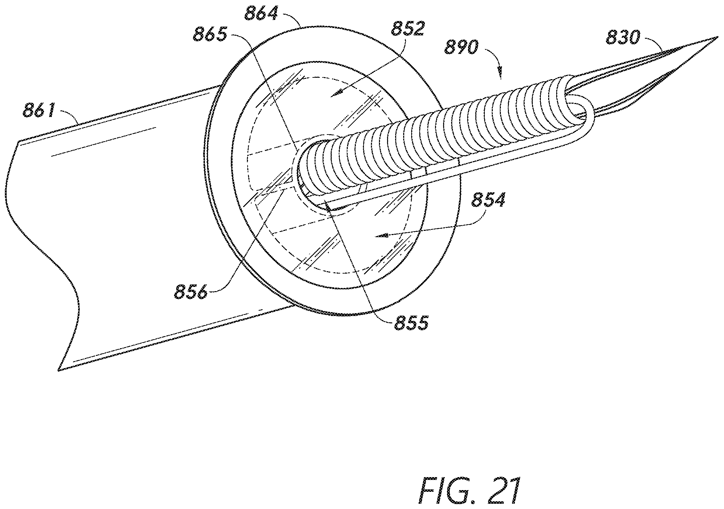

Figure 21 is a perspective view of a distal portion of a multi-lumen medical device shaft 861 having a needle 830 and tissue anchor 890 projected from a working lumen 855 thereof in accordance with one or more examples. For example, the tissue anchor 890 may be configured in a coiled sutureform configuration about the needle 130, which may advantageously be a slotted needle. As shown, the anchor 890 may comprise a plurality of winds of the suture around at least a portion of the needle 830. The lumen 855 may further be used to hold at least a portion of a pusher device (not shown; described above), which may be used to deploy the needle 330 and/or deploy the anchor 890 from the needle 830. In addition to the central working lumen 855, the shaft 861 includes one or more outer fluid-filled image-enhancement lumens 852, 854. As in some examples, the image-enhancement lumens 852, 854 are shown as closed-off, such that the lumens 852, 854 are effectively fluid-tight, and divided by a divider wall 856, which may run at least a portion of the length of the shaft 861.

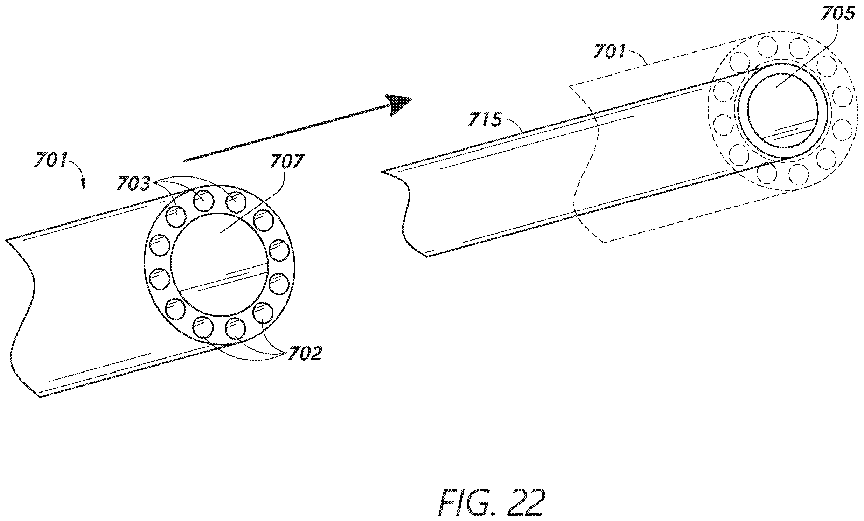

As described briefly above, according to some examples, an image-enhancement tube, sleeve, or component may be manufactured as a separate component from a working lumen/shaft used for a tissue anchor delivery procedure. For example, Figure 22 illustrates an image-enhancement sleeve 701, which may have any suitable or desirable form or shape. The image-enhancement sleeve 701 may comprise a tube or sleeve having one or more lumens, chambers, or channels formed in a wall thereof. The use of an image-enhancement outer sleeve that is separate from the working shaft can optimize the angle of incidence between sound waves and the wave-reflecting media. For example, the circumferentially-arranged fluid-filled compartments 703 can provides a unique surface area for reflection of the sound waves off the fluid in the compartments 703. The fluid-filled compartments/lumens 703 may advantageously be closed-off on one or more ends of the sleeve 701, such that the compartments/lumens 703 are effectively fluid-tight.

The sleeve 701 may have an elongated tubular shape, wherein the image-enhancement lumens/compartments 703 run longitudinally through at least a portion of the length of the tube. The sleeve 701 may further include a working-shaft-receiving lumen 707 through which a working shaft 715 may be inserted. For example, in some examples, the sleeve component 701 is formed of a polymer or plastic, whereas the working shaft 715 is formed of a more rigid material, such as stainless steel or the like. Such a configuration may be desirable from an echogenicity standpoint when the solid structure of the sleeve 701 (as opposed to the fluid contained in the lumens 703) is formed of a material that is less reflective of sound waves than that of the working shaft 715 (e.g., stainless steel). As shown, with the sleeve 701 disposed on the shaft 715, the image-enhancement lumens 703 may generally be positioned adjacent to the working lumen 707. The shaft 715 may comprise a rigid elongate tube forming at least one internal lumen, as shown.

The working shaft 715 may be used to advance various anchor-deployment devices/tools, as described herein, whereas the sleeve component 701 may be incorporated to provide improved imaging due to enhanced echogenicity of the fluid-filled lumens 703. Although image-enhancement sleeves and components are described herein as being coupled with tissue anchor delivery device shafts, it should be understood that image-enhancement sleeves and lumens in accordance with examples of the present disclosure may be utilized in connection with any type of medical device shaft, or non-medical device shafts that can benefit from improved visibility under echo. Furthermore, although the sleeve 701 is illustrated as a tubular sleeve, it should be understood that image-enhancement components having features similar to those shown and described with respect to the sleeve component 701 may have any suitable or desirable form, including a wrap, clip, band, and/or the like. Furthermore, the image-enhancement component 701 may be configured to fit over any size shaft. With respect to wrappable image-enhancement devices, such devices may be able to provide image-enhancement by fitting around shafts or devices having different diameters and/or sizes.

### Shaft with Outer Helical Wire

Various examples disclosed above relate to medical device shafts having image-enhancement lumens or chambers that run substantially longitudinally along the axis of the shaft. In certain examples, at least partially circumferentially- or horizontally-running lumens or chambers may be utilized to provide improved imaging with respect to a medical device shaft. For example, any of the examples disclosed above having image-enhancement lumens within a tubular form may be implemented with such lumens winding circumferentially around the tube as opposed to strictly longitudinally. Such circumferential/horizontal lumens may be generally helical in shape, wherein at least a longitudinal segment of the shaft is traversed by a helical lumen forming a plurality of winds of coil/channel about the shaft. Alternatively or additionally, the lumens/channels may be generally circular, wherein the shaft is associated with a plurality of separate concentric hoop-shaped lumens/channels.

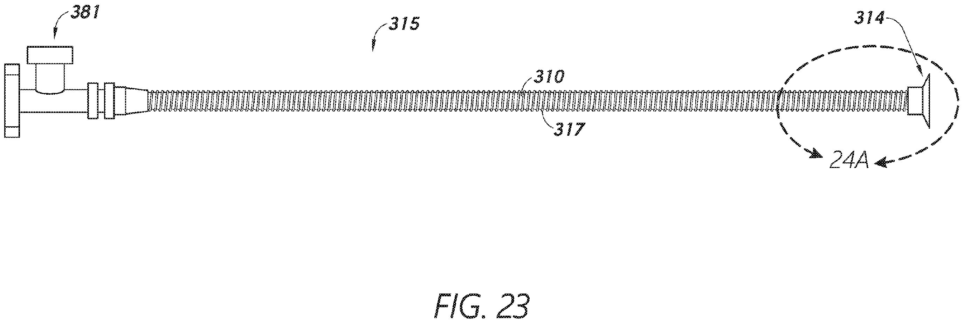

In some examples, a helical coil or tube may be wrapped around an outer working shaft to thereby provide improved imaging visibility as described herein. For example, Figure 23 illustrates a medical device shaft assembly 315 having a helical coil/wire 317 wrapped around a shaft 310 thereof. For example, as shown, the helical coil/wire may be wrapped around an exterior portion of the shaft 310. The shaft 310 may comprise a rigid elongate tube forming at least one internal lumen, as shown. In some examples, a helical coil (e.g., spring-type wireform) can be slid or disposed over the outer tube 310 of the shaft 310. A covering (not shown for clarity in Figure 23), such as shrink tubing or the like, may cover the shaft 310 and coil 317, which may serve to hold the components together and to trap fluid between the coils, such as air.

Although helically-shaped, and other geometrically-shaped, forms, patterns, and devices are disclosed for enhancing echogenicity in accordance with certain examples disclosed herein, such forms, patterns, and devices may be irregularly-shaped and/or non-geometrical in shape. For example, the shaft assembly 315 may have forms, shapes, members, devices, and/or features/components that are non-geometric in shape and/or irregular in shape or form that are configured to trap or hold fluid (e.g., air) and/or provide uneven surfaces designed to increase echogenicity. Furthermore, such forms, members, devices, and/or features/components may be associated with any other type of device or assembly other than the shaft 310 and assembly 315, including other types of medical device shafts or non-medical devices.

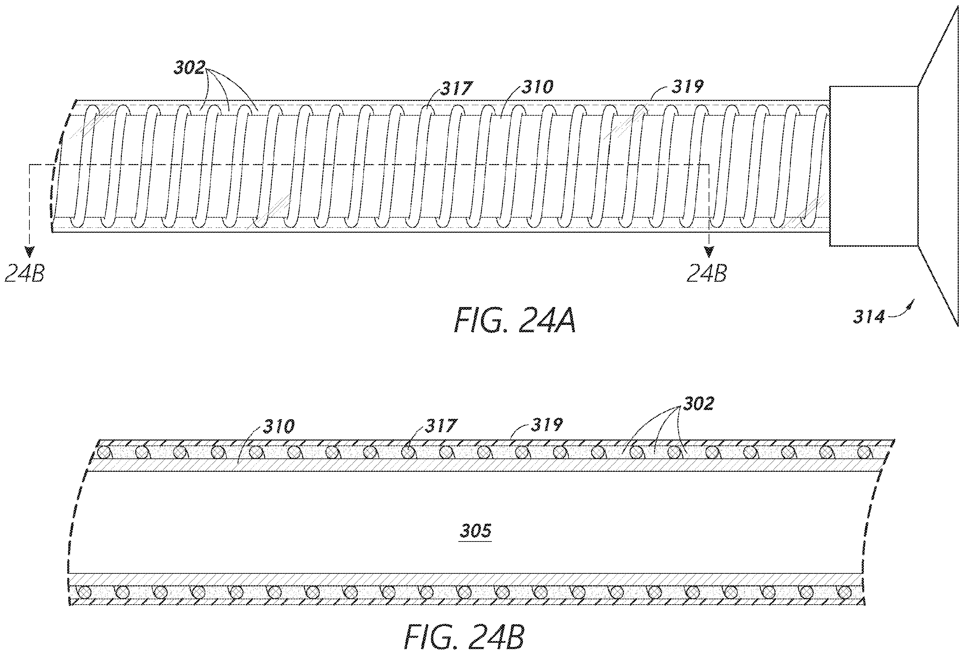

Figure 24A provides a close-up view of the distal portion of the shaft assembly 315 shown in Figure 23. Figure 24B is a cross-sectional view of a portion of the shaft assembly 315 shown in Figure 23. As shown in Figures 24A and 24B, the coil 317 may have shrink tubing or other covering 319 disposed or wrapped on the outside thereof, such that air or other fluid or contrast media is trapped in spaces 302 or at least partially inhibited from flowing. The fluid disposed between the adjacent coils of the coil/wire 317 may advantageously increase the echogenicity of the assembly 315, as described in detail herein. The wire 317 may be wrapped around the shaft 310 in a generally helical fashion. As shown, the image-enhancement spaces 302 may generally be positioned adjacent to the working lumen 305.

The shaft assembly 315 of Figures 23 and 24, which may include a spring-type coil slipped or wrapped over and/or around a shaft 310 and laminated with shrink tubing or the like, as described above, can incorporate image-enhancement features by maintaining echogenic fluid (e.g., air) in the spacings 302 between the coils or wraps of the coil 317, which may be visible under echo imaging. Additionally or alternatively, the coil 317 may comprise a hollow wire itself, wherein echogenic contrast media, such as air or other gas, maintained within the hollow conduit of the wire can provide enhanced imaging for the shaft assembly 315. In examples comprising a hollow wire wrapped around the shaft 310, there may or may not be space between wraps/coils of the wire. That is, the spacings 302 illustrated in Figures 24A and 24B may not be present in some examples, wherein the wire 317 comprises a hollow interior lumen containing image-enhancing fluid, such that the wraps/coils of the hollow wire may substantially abut/contact one another along the longitudinal dimension of the shaft 310. Conversely, as in the illustrated embodiment of Figures 23, 24A, and 24B, the wire/coil 317 can comprise a solid wire.

The outer cover 319 may comprise a polymer tube, which may be slid or otherwise positioned or disposed over the wire/coil 317 and/or shaft 310. In some implementations, heat may be used to cause the cover 3 19 to shrink and/or adhere to the wire 317 to provide lamination thereof. Such process may result in air or other fluid being trapped between the wraps/coils of the wire 317, such as in the spaces 302 illustrated in Figures 24A and 24B. In examples in which the wire/coil 317 comprises a hollow tube having an internal lumen filled with image enhancing fluid, such shaft assemblies may not include an outer covering or laminate.

As with all examples disclosed herein, the chambers, gaps, channels, lumens, and/or other non-working (and possibly fluid-sealed) compartments associated with the shaft 310 and/or shaft assembly 315 can be gas-filled (e.g. air, perfluorocarbon, nitrogen, or the like) or liquid-filled. For example, such gas- or other fluid-filled compartments may have no fluid communication with the working (e.g., central) lumen, which may primarily serve as a housing for a needle, pusher, and tissue anchor assembly, as described herein. In some examples, the shaft assembly 315 comprises alternating bands of uniform raised metals with gas trapped in between the bands. Certain of these examples, like the embodiment of Figures 23, and 24, can result in a distinct banding image of the shaft assembly under echo, resulting in improved clarity of the echo image that enables the surgeon to more precisely target the target valve leaflet(s) to achieve desirable results.

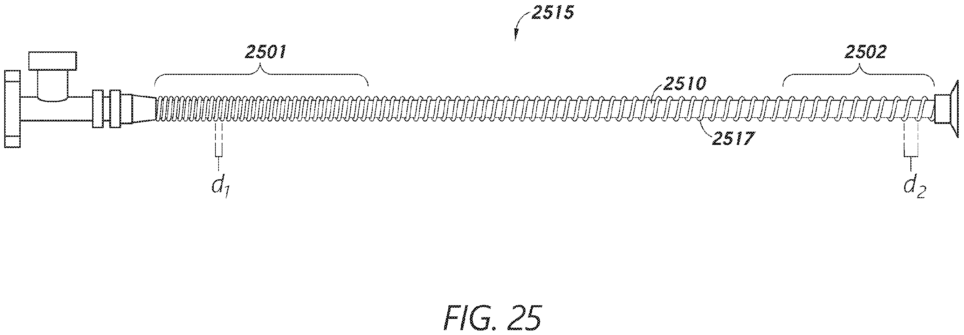

Figure 25 shows a medical device shaft 2515 having one or more coils with non-uniform spacing around a medical device shaft in accordance with one or more examples. The shaft 2510 may comprise a rigid elongate tube forming at least one internal lumen, as shown. As shown in Figure 25, the spacing between the adjacent coils/wraps of a wire/coil 2517 disposed around a medical device shaft 2510 may have varying and/or graduated spacings or elevations. That is, over a length of a medical device shaft, a wire or coil 2517 wrapped around the shaft 2510 may have a different spacing distance di between winds of the coil 251 7 at at least a portion of a first longitudinal area or portion 2501 of the shaft 2510 and a second spacing distance d₂ at at least a portion of another area portion 2502 of the shaft, as shown in the illustrated medical device shaft assembly 2515 of Figure 25. Such variation in spacing may result in an image under echo imaging that allows for differentiation and/or identification of different areas/portions of the shaft 2510 relative to one another. For example, as shown in the embodiment of Figure 25, the wire 2517 may have a smaller coil-to-coil spacing d₁ at or near a proximal end or area 2501 of the shaft assembly 2515 compared to a relatively wider/greater spacing distance d₂ between the coils of the wire 2517 at or near a distal end or area 2502 of the shaft assembly 2515.

Variation in pitch between adjacent coils may allow for simplified location/identification of the distal end or portion 2502 of the shaft assembly 2515 relative to the proximal end or portion 2501 of the shaft assembly. In some examples, the spacings between coils along the length of the shaft 2510 are substantially uniform across certain lengths or sections of the shaft. Alternatively, the spacings may increase continuously/gradually from one area to another area of the shaft 2510. Although Figure 25 shows greater spacing d₂ at or near the distal portion 2502 of the shaft assembly 2515 than the spacing di at or near the proximal and portion 2501, in some examples, the spacing may be greater at the proximal portion 2501 and narrower at or near the distal portion 2502. In some examples, the spacing may be substantially uniform over a proximal area and/or over a distal area. In some examples, the spacing may be uniform and/or different at the distal area portion 2502. The pattern of spacings between coils in a medical device shaft assembly as described herein may be any suitable or desirable pattern and may advantageously be designed to promote relatively easily visible and/or identifiable patterns or characteristics under echo. Variation in spacing may advantageously allow the operator to identify where one or more portions of the shaft are within a patient in real time.

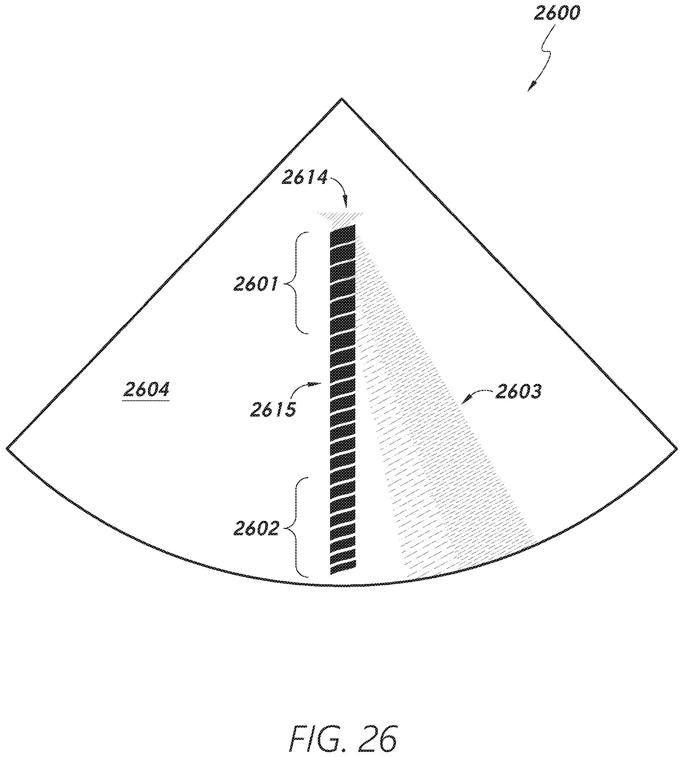

Figure 26 shows an example echo imaging window 2600 showing an image of a medical device shaft assembly 2615 in accordance with certain examples of the present disclosure. Particularly, the image window 2600 shows an image 2615 of a shaft having a spaced-coil design, as shown in Figures 23-25 and described above, or another shaft assembly having circumferential banding in accordance with the present disclosure. Although the image 2615 is shown as black and the background 2604 of the window 2600 is shown represented as white, it should be understood that such presentation is for descriptive purposes, and according to some imaging modalities, echogenic matter may present as white or other relatively-lighter shade on a substantially black or dark background. In some examples, the tip of a shaft assembly is outfitted with echogenic material or features to thereby improve the visibility thereof under echo and produce an image 2614 representative thereof.

The shaft image 2615 may present as relatively highly detectable compared to medical device shafts not including image-enhancement characteristics as described herein. The image of the shaft 2615 may show bands of visibility corresponding to the coil banding described above with respect to Figures 23-25. In some implementations, the atraumatic tip 2614 or other distal end component may further be visible in the image window 2600, as well as a comet tail artifact image 2603, which may occur in echo imaging due to reverberation of the echo from within the shaft 2615. For example, inside the tube(s) of the shaft, the walls of the tube may act as hollow reflectors, which can reflect back to the transducer in the form of an artifact, as shown.

Generally, the image of the shaft 2615 and the artifact 2603 may converge at or near the tip 2614 of the shaft. Therefore, the tip 2614 may serve to provide a true positioning of the tip of the shaft 2615, as it is the location of the tip of the shaft 2615 as well as the artifact 2603. In addition to the image-enhancement provided by the fluid-filled spaces between coils of the wire disposed around the shaft 2615, such outer coil embodiments may further provide improved imaging/visibility due to the irregular outer surfaces associated with the shaft and coil. In some embodiments, the working shaft may have an etched outer surface to form an irregular and relatively more reflective outer surface.

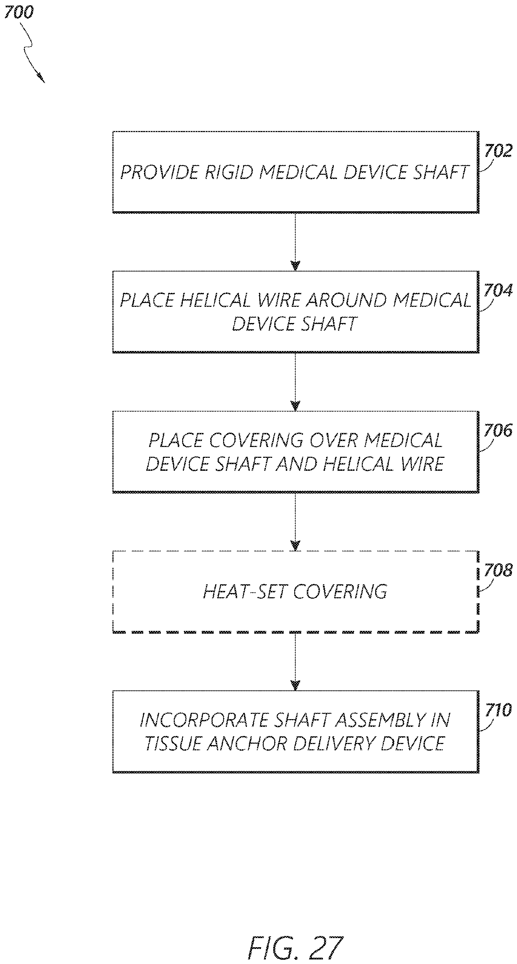

Figure 27 is a flow diagram illustrating a process 700 for manufacturing an echogenic shaft assembly in accordance with one or more embodiments of the present disclosure. At block 702, the process 700 involves providing a medical device shaft having a substantially rigid tubular form. For example, the medical device shaft may comprise a stainless-steel working lumen used in a medical procedure. The medical device shaft may be similar in certain respects shaft 110 of Figure 2 described above.

At block 704, the process 700 involves sliding, wrapping, or otherwise disposing a helical wire around at least a portion of the outer surface of the medical device shaft. The helical wire may be a substantially-solid wire or may alternatively comprise a tubular wire having an internal lumen configured and/or designed to receive and/or maintain fluid, such as air, therein. In some embodiments, the wire is disposed about the medical device shaft in such a way as to produce spacing or gaps between adjacent coils/turns of the wire. In some embodiments, substantially no spacing is present between adjacent coils/turns of the wire.

At block 706, the process 700 involves sliding, wrapping, or otherwise disposing a covering about the medical device shaft and helical wire. The covering may advantageously be substantially fluid-tight, such that air or other fluid present between adjacent coils of the wire may be substantially trapped therein, and further fluid external to the shaft assembly may not be permitted to enter within the covering. In some embodiments, disposing the covering about the shaft assembly may comprise laminating the medical device shaft and helical wire. For example, the covering may comprise a polymer sleeve or tube that may be heat-set to fit and/or adhere the covering to the helical wire and/or shaft. At block 708, the process 700 may involve heat-setting the covering to seal the covering and/or fluid in the wire gaps/spacings. At block 710, the process 700 involves incorporating the shaft assembly including the medical device shaft, the wire, and the covering in a tissue anchor delivery device or system, or other type of device or system used for performing a medical or non-medical procedure.

### Echogenic Recesses/Channels in Medical Device Shafts

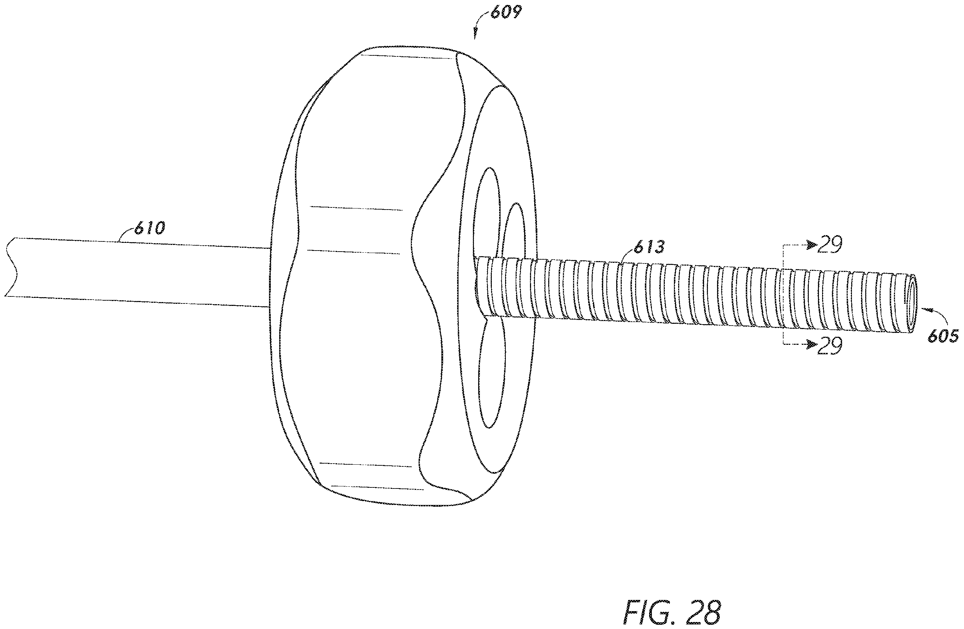

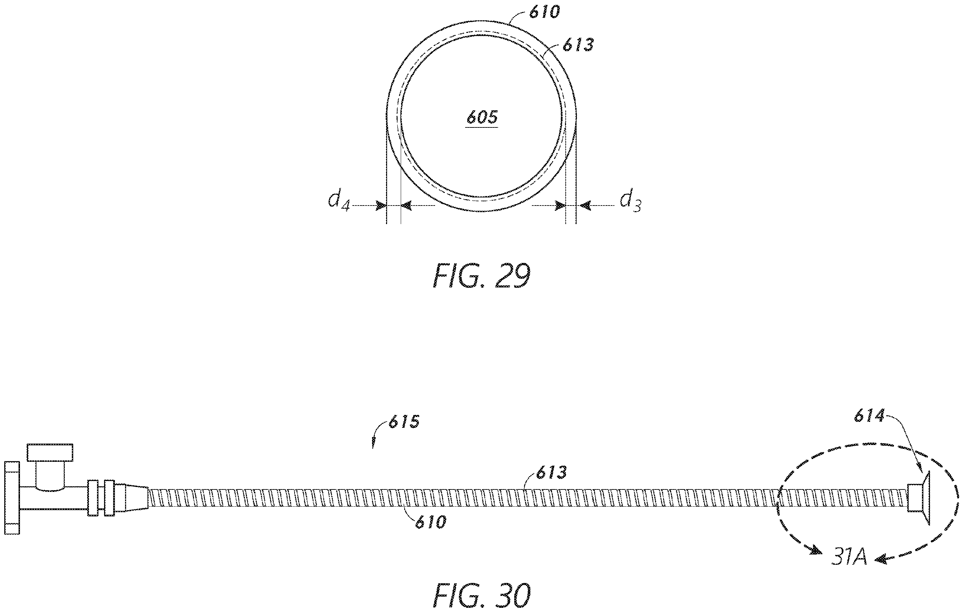

Examples disclosed above relate to helical and/or horizontal echogenic fluid channels in medical device shaft assemblies, wherein the fluid channels are in the form of spacings between adjacent coils of a wire or structure and/or within a lumen of a tubular wire wrapped around a medical device shaft. Alternatively, echogenic fluid channels in a medical device shaft assembly may take the form of recesses cut or etched into a tubular shaft to form channels/grooves. For example, Figure 28 shows a perspective view of a portion of a medical device shaft 610 and a channel-/groove-cutting die or tool 609 configured to cut image-enhancement helical grooves/channels in the shaft 610. Figure 29 is an axial view of a portion of the medical device shaft shown in Figure 28 in accordance with one or more examples. The die 609 represents one means of creating a groove/channel in helical pattern in a wall of a tubular shaft, wherein the die 609 is configured to cut deep enough external threads around the shaft 610 to allow for the presence of fluid or other contrast media therein. Devices and methods involving cutting image-enhancement channels/grooves in a shaft, as opposed to adding a coil or other structure on the outside of the shaft, can provide desirable image-enhancement functionality without substantially increasing the outer diameter/size of the shaft 610.

In some examples, the die 609 may be operated by rotating the die 609 about the shaft 610, wherein rotation relative to the shaft 610 causes a groove or channel of the desired depth and dimensions to be cut into the outer surface of the shaft 610 in a helical fashion. Figure 29 shows a straight-on view of the shaft 610 showing the depth d₃ of the groove 613. In some examples, the depth d₃ of the channels/grooves is greater than or equal to half, or even two-thirds, of the total thickness d₄ of the wall of the shaft 610. The channels/grooves are advantageously shallow enough to avoid piercing through the wall of the shaft 610. Furthermore, it may be desirable for the channels/grooves to be shallow enough such that the structure of the shaft 10 is not substantially weakened. That is, one potential downside of implementing image-enhancement by cutting channels/grooves into the wall of the shaft of a medical device is that doing so may undesirably weaken the mechanical strength/stability of the shaft. As shown, the image enhancement spaces 613 may generally be positioned adjacent to the working lumen 605.

Although Figure 28 shows grooves/channels cut using a die or other tool 609, channels or grooves in a medical device shaft may be created or formed in any suitable or desirable manner. For example, such grooves or channels may be molded in the shaft using a metal or non-metal molding technique. In some examples, the shaft may be three-dimensionally (3D) printed with the desired channels/grooves. Furthermore, although helical grooves/channels are shown and described, it should be understood that such grooves/channels may have any shape or form, including interrupted coils or banding.

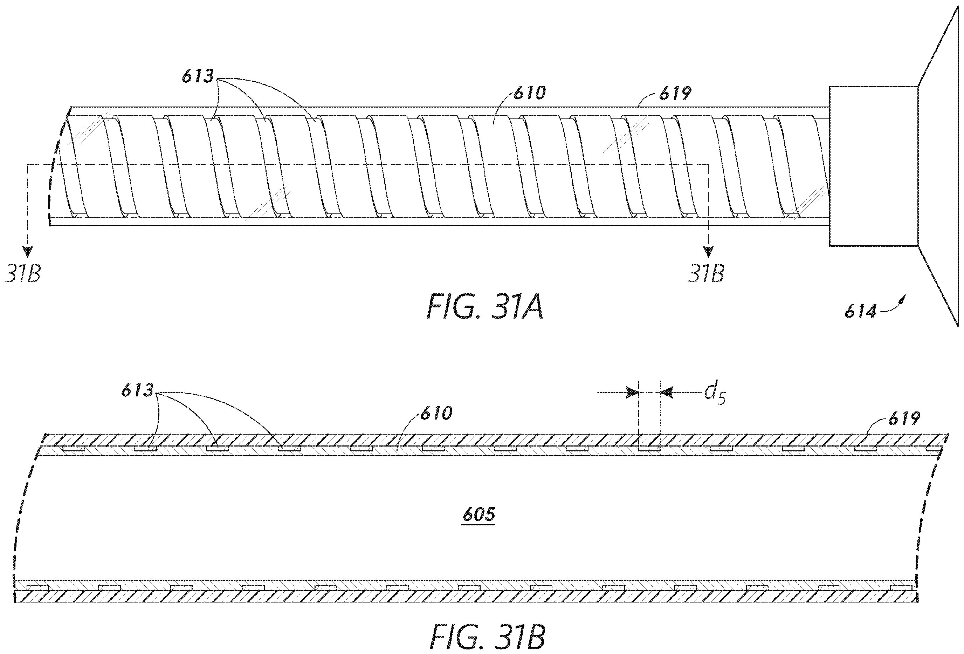

Figure 30 shows a side view of a medical device shaft assembly 615 having one or more image-enhancement channels 613 formed in a shaft thereof in accordance with one or more examples of the present disclosure. For example, the channel(s) 613 may be formed in an exterior portion of the shaft 610, as shown. The shaft 610 may comprise a rigid elongate tube forming at least one internal lumen, as shown. Figure 31A is a close-up view of a distal portion of the medical device shaft of Figure 30, whereas Figure 31B is a cross-sectional view of a portion of the medical device shaft portion shown in Figure 31A in accordance with one or more examples. The channel(s) 613 may be helical around the exterior of the shaft 610 and may traverse, in a helical configuration, a length of the shaft 610, as shown. The channel(s) 613 may wind/run around the circumference of the shaft 610 in a number of winds as they helically span/traverse a length of the shaft 610. In some example, the channel(s) 610 are formed in one or more rings that circumscribe a longitudinal/axial segment of the shaft 610. Such rings may not be helical, but rather circular, such that an individual ring does not axially traverse the shaft outside of the width of the channel(s) and/or the

Although helically-shaped, and other geometrically-shaped, channels, grooves, and/or recesses are disclosed for enhancing echogenicity in accordance with certain examples disclosed herein, such channels, grooves, and/or recesses may be irregularly-shaped and/or non-geometrical in shape. For example, the shaft assembly 615 may have channels, grooves, and/or recesses that are non-geometric in shape and/or irregular or without continuous pattern (e.g., non-contiguous channels or divots/recesses) in shape or form that are configured to trap or hold fluid (e.g., air) and/or provide uneven surfaces designed to increase echogenicity. Furthermore, such channels, grooves, and/or recesses may be associated with any other type of device or assembly other than the shaft 610 and assembly 615, including other types of medical device shafts or non-medical devices. In some examples, the channels, grooves, and/or recesses are knurled in shape/form or circumferential, longitudinal grooves, or some other pattern of removed material from a shaft or other device.

In some examples, the outside of the shaft 610, including the grooves/channels 613, may have shrink tubing or other covering 619 disposed or wrapped thereon, such that air or other fluid or contrast media is trapped and/or disposed within the grooves/channels 613 between the walls of the grooves/channels and the covering 619. The grooves/channels 613 may run around the circumference of the shaft 610 in a generally helical fashion. The shaft assembly 315 of Figures 30-31, can incorporate image-enhancement features by maintaining echogenic fluid (e.g., air) within the grooves/channels 613, which may be visible through echo imaging. The outer covering 619 may comprise a polymer tube, which may be slid or otherwise positioned or disposed over the shaft 610. In some implementations, heat may be used to cause the covering 619 to shrink and/or adhere to the outside of the shaft 610 to provide lamination thereof. Such process may result in air or other fluid being trapped within the grooves/channels 613. By using channels/grooves in the medical device shaft 610 itself, as opposed to a helical wire disposed thereon, examples in accordance with Figures 28-31 advantageously provide image-enhancement functionality without the need for additional assembly components and/or without increasing the outer diameter or size of the medical device shaft.

### Medical Device Shafts incorporating Echogenic Contrast Media

In some examples, the present disclosure relates to multi-lumen medical device shafts configured to have echogenic contrast media injected therein. For example, echogenic microbubbles, or other contrast media, may be injected and/or ejected at or near the tip of a medical device shaft, which may allow for marking identification of the tip of the shaft at different points in time. Such contrast media may be injected, and/or flow or be maintained within, a lumen similar to lumens disclosed herein, or alternatively may be used in balloon-type lumens, as described in detail below.

In some examples, the contrast medium may comprise microbubbles, which may be filled with a gas core and/or have a protein shell. Such microbubbles may advantageously be highly reflective of sound waves and return the sound waves from an echo (e.g., ultrasound) probe to create a highly-reflective image. In certain examples, microbubbles of a contrast medium may be substantially benign when introduced into the cardiovascular system, and therefore may be allowed to flow from a tissue anchor delivery device shaft into the heart and cardiovascular system during a valve-repair procedure.

As described in detail herein, echogenicity of tissue anchor delivery device shafts can be increased by adding gas-filled microbubble contrast media within one or more lumens or chambers of the shaft, to thereby provide contrast-enhanced visibility of the shaft. Microbubbles can provide a relatively high degree of echogenicity due to their ability to compress, oscillate, and reflect a characteristic echo of an ultrasonic frequency, thereby providing an enhanced sonogram of components containing such contrast media. Contrast media in accordance with examples of the present disclosure may comprise air and/or heavy gases, such as perfluoro carbon or nitrogen, or the like. For example, relatively heavy gases may be less water-soluble than air, and may therefore be less likely to leak out from the microbubble to impair echogenicity. Therefore, microbubbles with heavy gas cores may be preferable in some implementations.

In addition to the use of contrast media within valve repair shafts, echogenicity of such shafts may be increased in accordance with examples of the present disclosure through use of interior or exterior surfaces of the shaft that have certain echogenic characteristics. For example, the outside surface of a shaft in accordance with examples of the present disclosure may have certain texturing, roughening, and/or particle-adhesion characteristics to provide a relatively highly echogenic surface of the shaft to improve visibility thereof under echo imaging. Furthermore, surfaces and components of an anchor delivery device shaft in accordance with examples of the present disclosure may have improved echogenicity characteristics by presenting interfaces between materials of different densities, such as between stainless steel or other metal or hard surface of the shaft and air or other gas or medium disposed or contained adjacent thereto.

In some examples, moving lipid-coated echogenic microbubbles are incorporated in some manner into the shaft of a device. In connection with certain medical procedures, clinicians can use ultrasound contrast agents comprised of lipid-coated echogenic microbubbles filled with octafluoropropane gas to aid in the view of, for example, the left ventricle during diagnosis. In some implementations, such echogenic microbubble media can be used to aid in the echo navigation of a device by ejecting them through the inner lumen of the device to locate the distal tip or by injecting them into a balloon that at least partially surrounds the device shaft.

With respect to tissue anchor delivery devices in accordance with examples of the present disclosure, in some implementations, by injecting echogenic microbubbles through an inner lumen of a delivery device shaft, and either out of the atraumatic tip or out of a side port, a surgeon and/or echocardiographer can quickly locate the distal tip or portion of the shaft. For example, when the shaft of an anchor delivery device is draped/tented against a target leaflet, as described above, it can become difficult to distinguish the distal tip of the shaft from the leaflet tissue in some situations. Examples of the present disclosure can involve the ejecting of echogenic microbubbles from a tissue anchor device shaft before/during deployment of a tissue anchor therefrom to facilitate relatively easy location of the tip of the shaft based at least in part on the contrast in echo signature created thereby.

In some implementations, the present disclosure relates to medical device shafts having a balloon-type material or structure laminated or otherwise coupled to the outside of the device shaft, wherein echogenic contrast media can be flushed in and out of the balloon to agitate the contrast media (e.g., microbubbles of the contrast media) and create a brightened echo signature. This brightened echo signature can aid in the navigation of the delivery device shaft by making it more easily distinguishable from the surrounding blood and/or anatomy. During the insertion and removal of a balloon-type echogenic shaft in accordance with the present disclosure, the balloon feature(s) may be compressed to provide a relatively smaller shaft outer profile for insertion/withdrawal and/or navigation of the shaft.

Examples disclosed herein may utilize any suitable or desirable echogenic contrast media. For example, microbubble contrast media may be desirable in some situations. Contrast media in accordance with examples of the present disclosure may comprise saline, indocyanine green, hydrogen peroxide, dextrose, renografin, autologous blood injections, cyanoacrylate and/or other synthetic polymer(s). Generally, contrast media in accordance with examples of the present disclosure may advantageously provide alterations in the reflection pattern of sound waves. For example, such contrast media may increase the backscattered signal. In addition, contrast media may resonate sound waves in a relatively linear manner.

In some implementations, the contrast media may comprise an inner gas encapsulated by a shell that promotes desirable viscoelastic properties, including stability and durability. The type of gas used in microbubble contrast media may determine at least in part solubility and/or acoustic properties of the bubbles. Microbubbles, such as perfluoro carbon microbubbles may range from approximately 1-10 µm in size, which may be suitable for propagation in the systemic circulation. Softshell microbubble examples can comprise phospholipids or other surfactants, whereas protein-shelled microbubble examples can comprise an albumin shell around perfluoro propane gas. Although microbubble contrast media is described herein, it should be understood that the examples disclosed herein may utilize nano-bubble contrast media, which may generally refer to contrast media comprising bubbles having a size of between 400-800 nm.

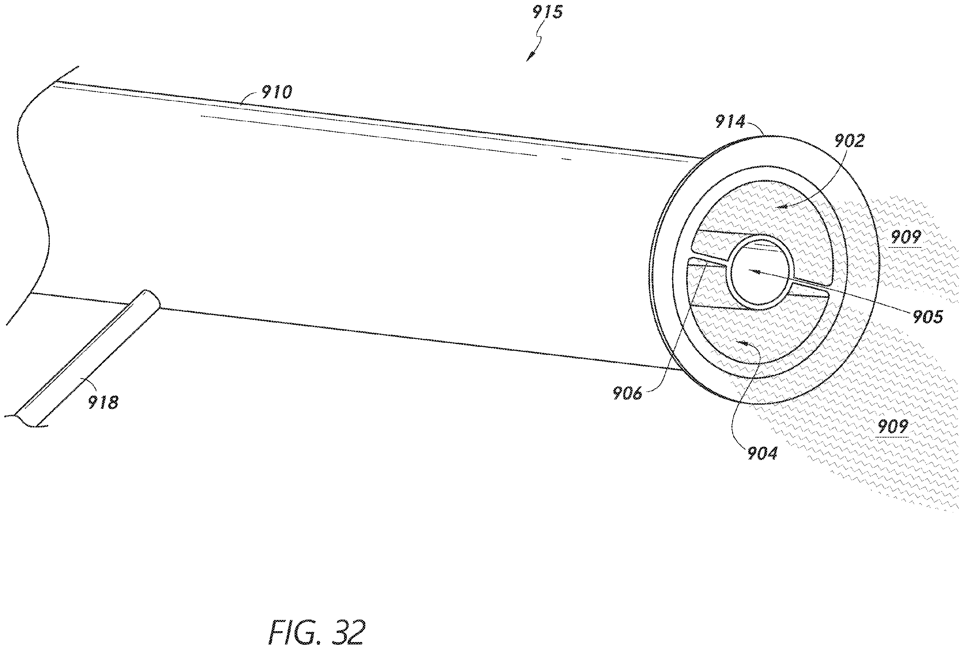

In some implementations, the sonic reflection of contrast media in connection with delivery device shafts may provide improved echogenicity characteristics through the use of flow or movement of the contrast media. That is, for microbubble contrast media, reflection off the microbubbles may be enhanced by fluid motion, causing images to appear brighter in reverberation. Figure 32 provides a perspective view of a multi-lumen shaft assembly 915 having one or more non-working lumens 902, 904, which may be utilized for the injection and propagation/flow of high-contrast fluid 909. For example, the shaft assembly 915 and/or shaft 910 includes lumens 902, 904 that are not closed off at the distal ends thereof, such that fluid contained therein may be permitted and/or inclined to escape the shaft assembly 915 at or near a distal end thereof, particularly in relatively high fluid pressure conditions. The shaft 910 may comprise a rigid elongate tube forming at least one internal lumen, as shown.

In some examples, the shaft assembly 915 includes a port 918, such as a side port, which may be utilized to inject contrast media fluid 909, such as microbubble fluid, which may in turn be ejected from the shaft assembly 915 at or near a distal end thereof. By ejecting the fluid 909 from the shaft 915, it may be easier to visualize and/or identify the tip or distal portion of the shaft assembly 915. The fluid-injection port 918 can be considered to be fluidly coupled to, or in fluid communication with, the first image-enhancement lumen in that fluid may flow between the port 918 and the lumen(s) 902, 904.

By containing the fluid 909 within the shaft 910, a contrasting image between the shaft 910 and the surrounding blood within the ventricle and/or other tissue/anatomy may be apparent. For example, under certain echo imaging modalities, the fluid-containing lumens 902, 904 may present as significantly brighter than the surrounding environment. Although Figure 32 shows an open-ended shaft assembly 915 that permits the contrast fluid 909 to be ejected from the distal tip of the shaft assembly 915, as described above, in some examples, contrast media may be contained within one or more closed-off (e.g., fluid-tight) lumens.

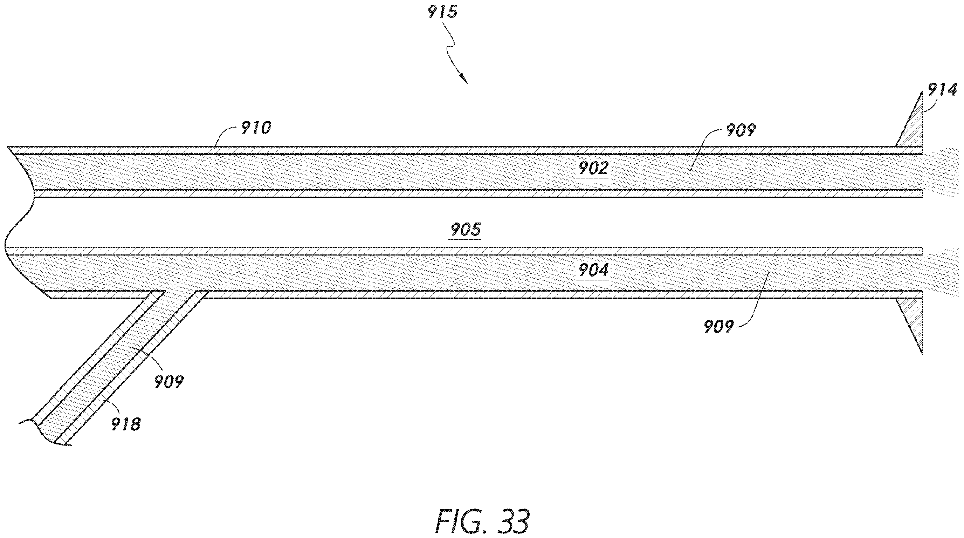

Figure 33 is a cross-sectional side view of the portion of the multi-lumen medical device shaft assembly 915 shown in Figure 32 in accordance with one or more examples of the present disclosure. Specifically, Figure 33 shows the internal working lumen 905, which may be surrounded by one or more image-enhancement lumens or lumen portions 902, 904, which may comprise contrast fluid 909. The lumen portions 902, 904 may be separate areas of a single circumferential lumen. In some embodiments, the contrast fluid 909 may be introduced into the lumen(s) 902, 904 through the injection port 918. Although the lumens 902, 904 may be separated by one or more structures or components, such as the dividing wall 906 shown in Figure 32, in some examples, the lumen(s) 902, 904 are in fluid communication at one or more portions or areas of the shaft assembly 915. Injecting the contrast fluid 909 (e.g., microbubble contrast fluid, or other ultrasound contrast agent/media) into and/or through the shaft assembly 915 can advantageously facilitate navigation of the shaft assembly 915 under echo guidance.

Compared to certain other imaging solutions incorporating and relying solely on relatively dense materials like stainless steel in order to create an echo signature that contrasts sufficiently with environmental blood and surrounding anatomy, image-enhancement principles disclosed herein can provide relatively higher echogenicity properties by using materials with a density significantly different from blood or tissue, thereby manipulating how sound attenuates through the material and creating desirable contrasting echo signatures. For microbubble-utilizing embodiments, the relatively heavy gas molecules that may be used/trapped inside such echogenic microbubbles can offer a relatively greater magnitude of difference in density compared to blood and tissue than some denser materials like stainless steel. In addition, when such microbubbles are caught in an ultrasonic frequency field, they can be inclined to compress and oscillate to create an even brighter echosigna-ture. Echo image brightness can further be supplemented by ejecting the microbubbles out of the shaft/device or flushing them through a balloon that surrounds the device, as described in detail below. Furthermore, the microbubble contrast fluids disclosed herein are generally advantageously safe for use in certain medical procedures. Although the term "shaft assembly" is used herein to refer to medical devices comprising a shaft associated with one or more additional features or components, it should be understood that such assemblies may be referred to simply as "shafts," rather that shaft assemblies. That is, references herein to a "shaft," which is used herein according to its broad and ordinary meaning, should be understood to apply to a shaft component alone or to a shaft assembly incorporating, or associated with, one or more additional features or components as described herein. Therefore, use of the term "shaft" may refer herein to a shaft assembly.

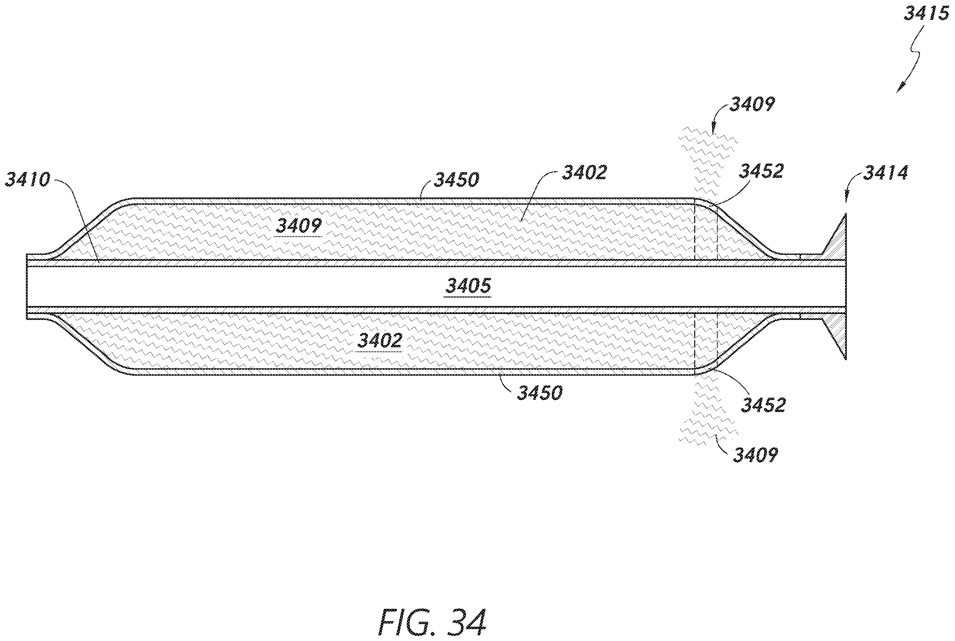

Figure 34 is a cross-sectional side view of a medical device shaft assembly 3415 having one or more image-enhancement balloon features 3450 in accordance with one or more embodiments. The shaft assembly 3415 may further include one or more side ports 3452 to eject contrast media 3409 contained in the balloon feature(s) 3450. In some implementations, the balloon feature(s) 3450 may comprise a tube or other form implemented over the working lumen 3405 and/or shaft 3410. The shaft 3410 may comprise a rigid elongate tube forming at least one internal lumen, as shown. In some implementations, the contrast fluid 3409 may be injected into the balloon feature(s) 3450 from the proximal end/area of the balloon feature(s) 3450 and/or shaft assembly 3415 and may flow at least a portion of the length of the shaft 3410 to at least partially fill the balloon feature(s) 3450. In some procedures/implementations, the contrast fluid 3409 may be continuously ejected from the balloon feature(s) 3410, such as out of one or more ports 3452 associated with a distal portion of the shaft assembly 3415. As shown, the image-enhancement lumen(s) may generally be positioned adjacent to the working lumen 3405.

Compared to embodiments incorporating rigid image-enhancement lumens, incorporation of balloon-type image-enhancement features in medical device shafts may advantageously be expandable and/or collapsible in certain respects. Furthermore, the flexibility of certain balloon-type image-enhancement features may allow for gas bubbles in contrast fluid to move relatively freely within the balloon(s) without obstruction that may be present with thicker and/or more rigid fluid channels, thereby providing improved visibility under echo. In some embodiments, the balloon feature(s) 3450 have a wall thickness of approximately 70-100 µm (about 0.003 to 0.004 inches). In some implementations, the balloon feature(s) 3450 may allow for insertion of the shaft assembly 3415 into an anatomical chamber in a deflated state, thereby providing a relatively low profile for the shaft assembly 3415. The balloon feature(s) 3450 may subsequently be inflated at least in part after the shaft 3415 has been inserted into the patient. Therefore, embodiments of the present disclosure may advantageously allow for devices providing additional image-enhancement fluid volume when necessary without requiring accommodation for such volume during device introduction.

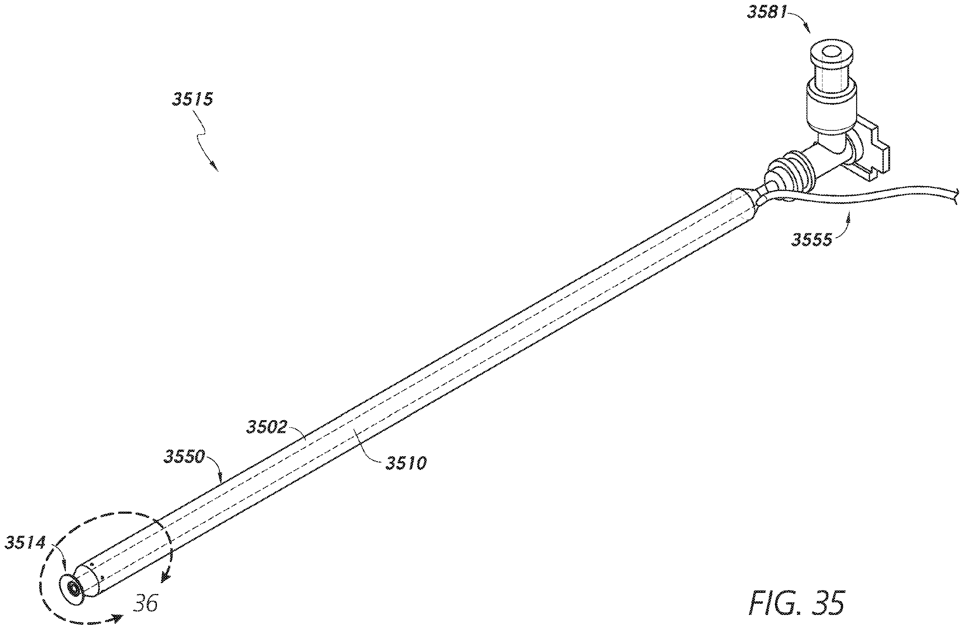

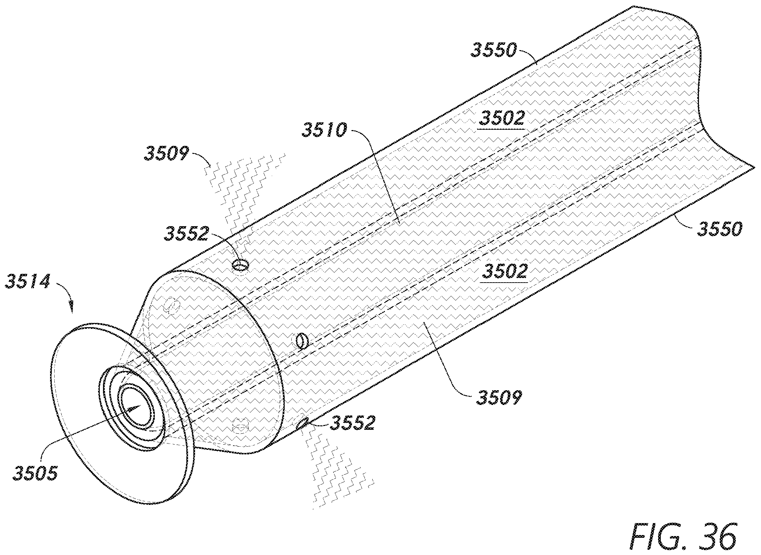

Figure 35 shows a perspective view of a medical device shaft assembly 3515 having one or more image-enhancement balloon features 3550 in accordance with one or more embodiments. Figure 36 is a close-up view of a distal portion of the medical device shaft of Figure 35 in accordance with one or more embodiments. In some implementations, contrast fluid 3509 (see Figure 36) may be introduced into the balloon-type lumen 3502 through the injection port 3555. Injecting the contrast fluid 3509 (e.g., microbubble contrast fluid, or other ultrasound contrast agent/media) into and/or through the lumen 3502 can advantageously facilitate navigation of the shaft assembly 3515 under echo guidance. The fluid-injection port 3555 can be considered to be fluidly coupled to, or in fluid communication with, the first image-enhancement lumen in that fluid may flow between the port 3555 and the lumen(s) 3502.

The shaft assembly 3515 may further include one or more side ports 3552 to eject contrast media 3509 contained in the balloon feature(s) 3550. In some implementations, the balloon feature(s) 3550 may comprise a tube or other form implemented over the working lumen 3505 and/or shaft 3510. In some implementations, the contrast fluid 3509 may be injected into the balloon feature(s) 3550 from the proximal end/area of the balloon feature(s) 3550 and/or shaft assembly 3515 and may flow at least a portion of the length of the shaft 3510 to at least partially fill the balloon feature(s) 3550. In some embodiments, the contrast fluid 3509 may be continuously ejected from the balloon feature(s) 3510, such as out of one or more ports 3552 associated with a distal portion of the shaft assembly 3515.

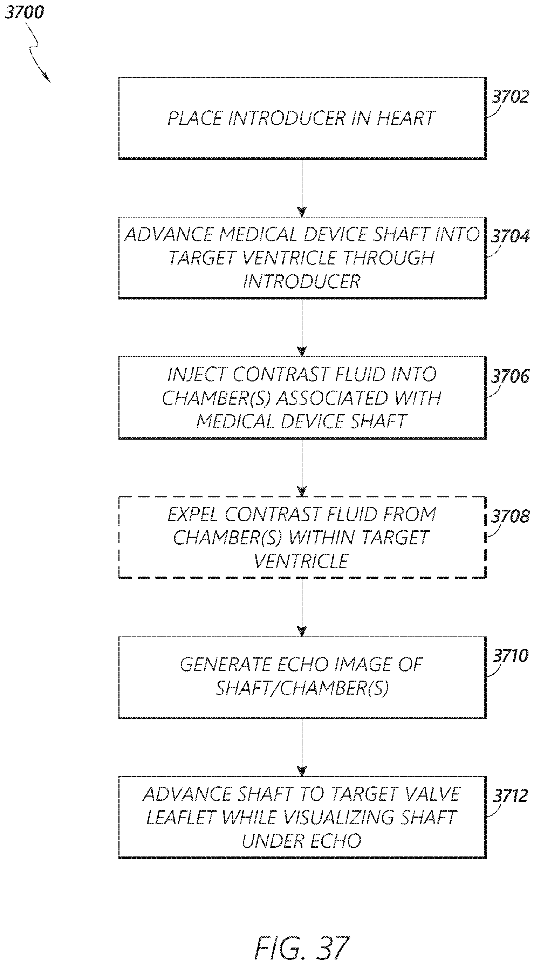

Figure 37 is a flow diagram of a process 3700 for targeting a valve leaflet using a multi-lumen/chamber medical device shaft containing echogenic contrast media in accordance with one or more embodiments. At block 3702, the process 3700 involves placing an introducer into a target ventricle (e.g., left ventricle), or other chamber or blood vessel, of a heart of a patient. At block 3704, the process 3700 involves advancing the medical device shaft into the target ventricle through the introducer, as described in detail herein. At block 3706, the process 3700 involves injecting echogenic contrast fluid into one or more chambers or lumens associated with the medical device shaft. For example, such chamber(s)/lumen(s) may be substantially rigid lumens/chambers or may be inflatable balloon-type chamber(s)/lumen(s).

At block 3708, the process 3700 may optionally involve expelling or ejecting contrast fluid from chamber(s)/lumen(s) within the target ventricle, such as through one or more ejection ports associated with the medical device shaft. At block 3708, the process 3700 involves generating echo imaging of the medical device shaft and/or the fluid-containing chamber(s)/lumen(s) associated therewith, wherein such imaging may provide guidance for the medical procedure. At block 3710, the process 3700 involves advancing the shaft to a target tissue anchor deployment site, such as a target valve leaflet, while visualizing the shaft under echo.

### Echogenic Guidelines

As described in detail herein, navigation of medical device shafts under echo guidance can be challenging in some situations and can require relatively extensive training and/or coordination between the surgeon and the electrocardiographer. In many instances, the echo signature of the medical device is less than optimal due to, for example, the various functional design requirements for the device. In particular, with respect to tissue anchor delivery device shafts, as disclosed herein, the tips of such devices can suffer from reduced visibility under echo when contacting target tissue (e.g., mitral valve leaflet tissue). Furthermore, lack of visibility can contribute to tissue damage and/or entanglement with neighboring anatomy when attempting to advance a medical device shaft within a target cardiac chamber. In addition, according to some implementations, navigation of medical device shafts may necessarily be repeated for subsequently-deployed tissue anchors or implantation of other types of devices.

In some implementations, the present disclosure relates to medical device shafts that are associated with a guideline (e.g., guidewire) that the shaft can be deployed alongside that is relatively more visible than the shaft alone. In some embodiments, the guideline may comprise a relatively small hollow tube having fluid, such as air, disposed in the lumen thereof, similarly to certain features disclosed above.

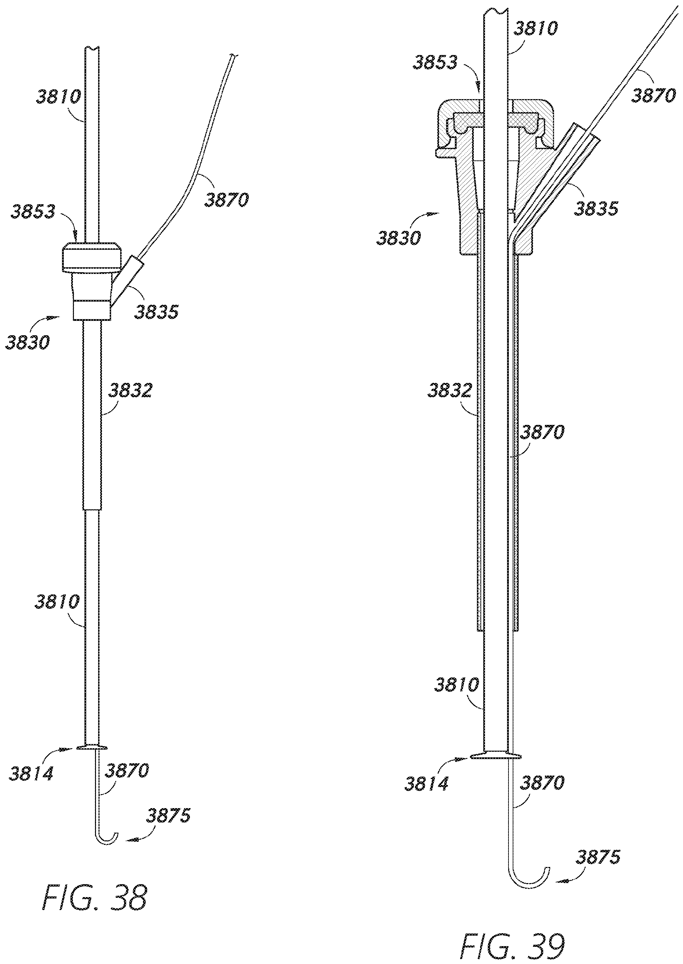

Figure 38 shows a tissue anchor delivery device shaft 3810 and introducer 3830 including a guideline port 3870 in accordance with one or more embodiments. Figure 39 shows a cutaway view of the introducer shown in Figure 38 in accordance with one or more embodiments. The guideline 3870 may advantageously be relatively echogenic compared to the shaft 3810 and/or tip 3814. Therefore, pre-deployment of the guideline 3870 to the target tissue anchor deployment site may advantageously produce a relatively highly visible path from introducer lumen 3832 to the target site under echo. For example, the guideline 3870 may comprise a solid echogenic wire/rod and provide a rail-type system for guiding the shaft 3810 to the target anatomy/location.

The introducer 3830 is a multi-port introducer, including a port 3853 for inserting the shaft 3810 as well as an additional port 3835 for inserting and advancing the guideline 3870. Use of a guideline port and echogenic guideline may help provide simplified navigation of devices under echo guidance. In some embodiments, the guideline 3870 is atraumatic and highly visible under echo. For example, the guideline 3870 may have a J-tip form 3875 at a distal end thereof, or any other feature that reduces the risk of tissue damage by the guideline. The guideline port may be oriented at a deflected angle with respect to the lumen 3832 of the introducer 3830. The dual ports of the introducer 3830 can allow the echogenic guideline 3870 and the shaft 3810 to be inserted at the same site. Furthermore, the dual ports may serve to align the guideline 3870 and the shaft 3810 so the shaft 3810 runs next to and parallel to the guideline. The introducer 3830 may advantageously be a hemostatic introducer.

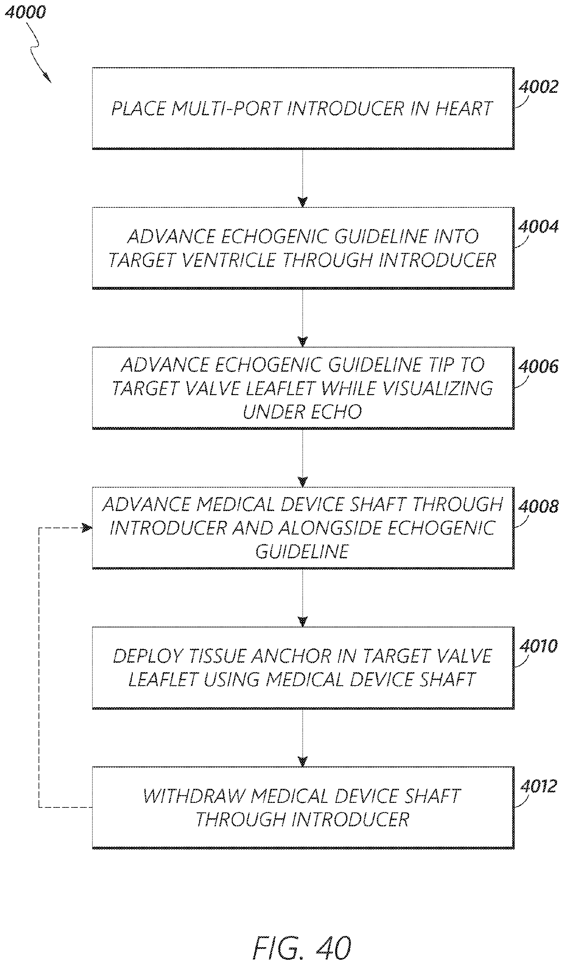

Figure 40 is a flow diagram illustrating a process for advancing a medical device shaft using a guidewire in accordance with one or more embodiments. At block 4002, the process 4000 involves placing a multi-port introducer, such as a hemostatic introducer as described herein, in a heart to provide access via one or more lumens thereof to, for example, a target ventricle or other chamber/vessel of the heart. The introducer advantageously includes a main port configured to have a medical device shaft inserted therethrough, as well as a secondary port configured to have a guideline (e.g., guidewire) inserted therethrough. In some implementations, the introducer is inserted at or near the apex of the heart through a small incision in the chest.

At block 4004, the process 4000 involves advancing an echogenic guideline into the target ventricle via the secondary port of the introducer and/or one or more lumens or other valved access ports of the introducer. At block 4006, the process 4000 involves advancing a tip of the echogenic guideline to a target location, such as on a ventricular side of a target heart valve leaflet (e.g., mitral leaflet) while visualizing the echogenic guideline under echo imaging. The echogenic guideline may be advanced to the target location in any suitable or desirable way.

At block 4008, the process 4000 involves advancing a medical device shaft through the main port of the introducer and/or one or more lumens of the introducer into the target ventricle, and further advancing the shaft to the target location alongside the echogenic guideline. The medical device shaft may generally follow the guideline to the proper target location. It may be beneficial to advance the guideline prior to advancing the medical device shaft in order to avoid masking/obscuring the echogenicity of the guideline in view of the medical device shaft. At block 4010, the process 4000 involves deploying a tissue anchor in the target valve leaflet or other target anatomy using the medical device shaft.

At block 4012, the process 4000 involves withdrawing the medical device shaft from the heart through the introducer. After tissue anchor deployment, the guideline may also be removed, or if additional tissue anchors are to be subsequently deployed, the guideline may be left in place at or near the target implantation site to allow the guideline to be reused to guide a shaft to the target implantation site. Where additional anchor(s) are to be deployed, the process 4000 may loop back to block 4008, as shown. Therefore, the process 4000 may allow for placement/deployment of multiple tissue anchors without requiring additional echo navigation.

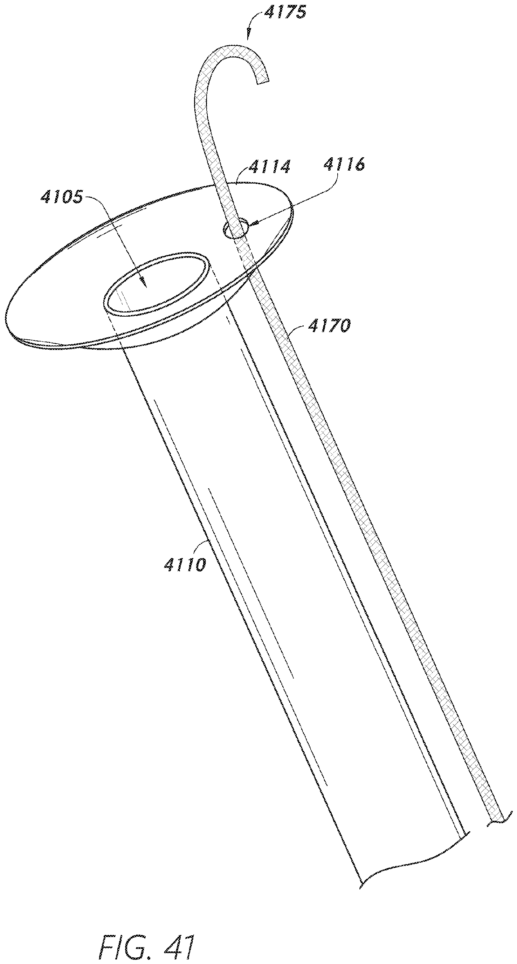

In some situations, using a guideline rail system as disclosed herein without having the guideline be attached or coupled to the shaft in some way may result in confusion of moving components due to the thin structure of the guideline and/or the pulsatile flow of the beating heart. In some embodiments, a port or engagement feature can be integrated with the medical device shaft, such as in the atraumatic tip thereof, to assist in the guidance of the medical device shaft along the guideline. Figure 41 shows a perspective view of a distal portion of a medical device shaft 4110 including a guideline-engagement feature 4116 in accordance with one or more embodiments. Generally, the tip of the guideline is atraumatic to avoid damage to the target tissue (e.g., leaflet tissue) it contacts. For example, the guideline 4170 may have a J-tip form 4175, as shown, or may have any other feature impeding tissue damage, such as a ball or other rounded feature. The tip 4114 of the shaft 4110 may be engaged with the guideline by the engagement feature 4116 prior to insertion of the shaft 4110 into the target ventricle, such that no separate introducer port is necessary for introduction of the guideline.

The device assembly of Figure 41 may provide relatively clearer echogenic signature than certain echogenically-enhanced instruments, which may not generally be fully optimized due to their additional design requirements. For example, the echogenic guideline may have a highly/completely optimized echogenic profile. Furthermore, due to the atraumatic characteristics of the guideline, it may be easily navigated within a heart chamber without fear of damaging tissue.

The engagement feature 4116 facilitates attachment of the guideline 4170 to the tip 4114 of the shaft 4110 for improved echogenic visibility and ease of guidance up to the target implantation site (e.g., mitral valve). The tip 4114 of the shaft 4110 can comprise a flexible plastic form (also referred to herein as an "end effector") that is generally circular in shape and allows atraumatic tenting of the shaft 4110 on the target valve leaflet. The engagement feature 4116 may comprise a relatively small hole or port to allow insertion of the guideline 4170, which can act as a rail system for the shaft 4110 during guidance from the introducer site up to the ventricular side of the target valve leaflet.

In some embodiments, the guideline 4170 can be inserted and removed independently from the delivery device shaft 4110. Once the hemostatic introducer is placed in the heart, the guideline 4170 may be inserted into the target ventricle through the introducer. In some implementations, the guideline 4170 may be engaged with the engagement feature 4114 and inserted engaged with the engagement feature 4116. The shaft 4110 may be passed through introducer into the target ventricle using the echogenic guideline as a rail system to navigate up to the target leaflet. In some implementations, the guideline 4170 may be removed prior to tissue anchor deployment.

Having the guideline 4170 attached to the shaft tip 4114 can facilitate relatively precise imaging of the shaft 4110 for the entirety of guidance from the insertion site up to the target leaflet. Furthermore, after confirmation that the shaft tip 4114 is positioned on the target leaflet, the guideline 4170 can be removed to allow normal deployment of the tissue anchor. With the guideline 4170 actually attached to the tip 4114 during navigation, the device of Figure 41 can allow for relatively faster procedure times and more accurate de-ployments. Furthermore, attaching the guideline 4170 to the shaft 4110 can stabilize the guideline 4170 and/or result in less deflection of the guideline 4170 in the ventricle, as well as tighter alignment between the guideline 4170 and the shaft 4110.

Although the engagement feature 4116 is shown as a circular hole, it should be understood that the engagement feature may have any suitable or desirable shape or form. In some embodiments, the engagement feature allows for disengagement of the guideline 4170 within the ventricle. For example, the engagement feature may have a hook-type form with a radial disengagement path through which the guideline can be drawn out of the engagement feature 4114 radially with respect to the axis of the shaft 4110.

Using a removable, atraumatic guideline rail system as disclosed herein can provide a relatively simple solution to improve echogenic guidance while maintaining the integrity of certain tissue anchor delivery devices. Attaching the guideline to the shaft can also facilitate maintenance of the guideline in a parallel alignment with the shaft. Although the engagement feature 4116 of Figure 41 is shown as being integrated with the tip portion 4114, it should be understood that the engagement feature may be couple to or otherwise associated with any portion of the shaft 4110. Furthermore, in some embodiments, more than one engagement feature may be used.

### Echogenically-Enhanced End Effectors and Shafts

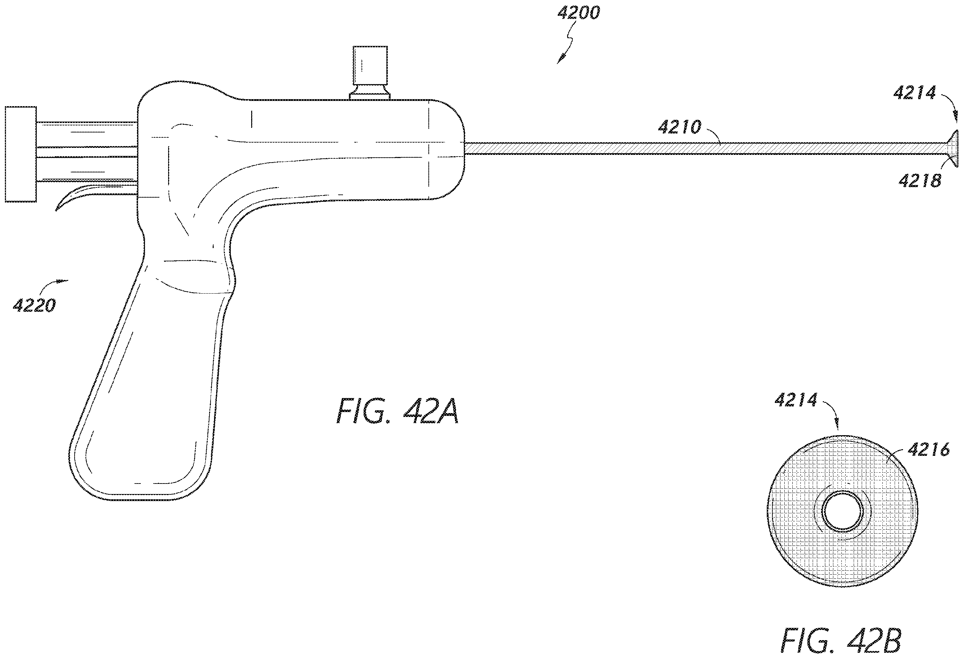

Figure 42A provides a side view of a medical instrument 4200 including a rigid, elongate shaft 4210 and an end effector 4214 associated with a distal end portion of the shaft 4210 in accordance with one or more embodiments of the present disclosure. Figure 42B provides an end view of the end effector 4214 of the medical instrument 4200 shown in Figure 42A, including a distal face/surface 4216 thereof, in accordance with one or more embodiments of the present disclosure. As described above, the medical instrument 4200 and may be a heart valve repair device used to deliver/deploy suture anchors and/or the like. However, it should be understood that the principles disclosed herein with respect to enhanced echogenicity characteristics of medical instruments and/or components thereof are applicable to any type of medical instrument.

In some embodiments, the end effector 4214 may comprise a generally circular tip having a diameter of, for example, approximately 6.35 mm (1/4 of an inch). In some embodiments, the shaft 4210 comprises an elongate tubular form of stainless steel. The shaft 4210 can have, for example, an outer diameter of approximately 2 mm (about 0.083 inches) and/or an inner diameter of approximately 1.6 mm (about 0.063 inches). In some embodiments, the shaft 4210 is coated in urethane or other (e.g., polymeric) material, which may advantageously be designed to enhance echogenicity of the shaft 4210 based at least in part on the density interface between the shaft and the coating.

In some embodiments, the shaft 4210 and/or end effector 4214 can comprise materials with certain echogenic additives added thereto. For example, the forms of the shaft 4210 and/or end effector 4214, and/or coatings/coverings applied thereto, can comprise embedded particles and/or air pockets therein, which can create nonhomogeneous coating/surfaces that increase the reflection coefficient of the material, thereby increasing echogenicity. Generally, end effectors and/or shaft coverings in accordance with embodiments of the present disclosure may present discontinuity in material densities, such as between the flexible form/material of the end effector and/or shaft covering (e.g., thermoplastic rubber or the like) and certain additives associated therewith, such as air-filled and/or hollow glass spheres (e.g., gas-filled microspheres), metal particulates, or any of the other implementations disclosed herein.

The shaft 4210 and/or end effector 4214 may have features or characteristics that provide relatively high echogenicity for such components when compared to a stainless-steel shaft with a homogeneous/uniform thermoplastic rubber end effector. With respect to the diagrams of Figures 42A and 42B, the relatively high-echogenicity characteristics of the shaft 4210 and/or end effector 4214 are represented by the respective fill patterns shown filling the shaft 4210 and end effector 4214, respectively. Although substantially the entire shaft 4210 and end effector 4214 are shown with highechogenicity fill patterns, it should be understood that such components/portions of the medical instrument 4200 may have high-echogenicity characteristics associated with only portions thereof in some embodiments. Furthermore, although different highechogenicity fill patterns are shown for the shaft 4210 and the end effector 4214, it should be understood that in some embodiments, similar echogenicity features/characteristics may be implemented with respect to the shaft 4210 and the end effector 4214. In some embodiments, high-echogenicity features may be implemented in connection with either the shaft 4210 or the end effector 4214, but not both.

Description herein of shafts having enhanced echogenicity characteristics may relate to other types of devices, including flexible catheters, sheaths, and/or other tubular and/or lumen-forming devices. Therefore, the various embodiments disclosed herein should be understood to be applicable to any type of medical device for which enhanced echogenicity may be desired. Furthermore, as with other embodiments disclosed herein, description below of shafts and/or end effectors with echogenicityenhancing characteristics are applicable to non-medical shafts and end effectors. With respect to the end effector 4214, high-echogenicity characteristics may be implemented with respect to a distal end surface 4216 thereof, side surface 4218, and/or any other area (e.g., area below the surface(s) of the end effector 4214).

In some embodiments, medical device/instrument shafts comprise tubular forms comprising stainless steel or other at least partially rigid material. In some embodiments disclosed herein, echogenic coverings or coatings may be disposed/applied over at least a portion of a medical instrument shaft to provide enhanced echogenicity for the shaft. For example, with further reference to Figure 42A, a thermoplastic elastomer/rubber (e.g., polyether block amide (e.g., PEBAX^{®} elastomer)) or other compound (e.g., fluorinated ethylene propylene (FEP)) may be extruded in a tubular form and disposed about the shaft 4210, wherein heat shrinking may be implemented to secure and/or bond the tube to the shaft 4210. In cases in which the covering/coating applied to the shaft 4210 has different density characteristics than the shaft 4210, or where the material of the coating/covering includes certain echogenicity-enhancing additives, and/or other echogenic material or surface characteristics as described below with respect to various embodiments of the present disclosure, the echogenicity of the shaft 4210 may be enhanced at least in part through the application/disposition of the coating/covering to/on the shaft 4210. It should be understood that any of the echogenicity-enhancing materials and/or features disclosed herein may be applied to a shaft or end effector in any suitable or desirable way, including through the use of heat-form tubing, and/or other material application process(es). In some implementations, echogenicity-enhancing materials are over-molded over the shaft and/or end effector. The end effector may be formed by over-molding over the shaft as well.

In some embodiments, the end effector 4214 is formed initially as part of an extrusion used for covering at least a portion of the shaft 4210, wherein the illustrated shape of the end effector can be die-cut from the distal portion of the extrusion. In some embodiments, the end effector 4214 comprises thermoplastic rubber (e.g., PEBAX^{®} elastomer). As with any of the embodiments disclosed herein, shaft coatings/coverings may be based on certain medical grade polyurethane and/or similar materials, which may be formed using, for example, blowing agents or the like. In some implementations, such coatings/coverings may become reactive in a relatively moist tissue environment. Examples of materials that may be used for end effectors and/or shaft coverings/coatings in accordance with embodiments of the present disclosure include various types of urethane (e.g., ReoFlex^{™} 20 urethane, Bentley Advanced Materials, Kidderminster, UK), PEBAX^{®} elastomer, silicone, silica aerogel, expanded polytetrafluoroethylene (ePTFE), metal (e.g., washer or wire), or the like.

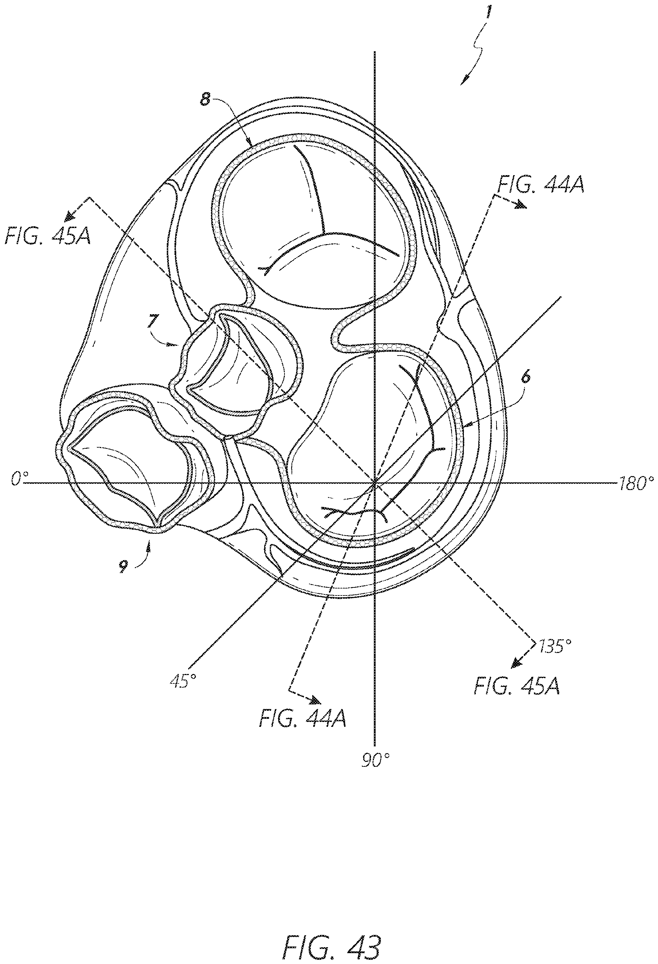

A medical device shaft used for, for example, heart valve repair procedures, may generally be more visible at some angles than others. Embodiments of the present disclosure may advantageously increase the range of angles at which a medical device shaft and/or end effector may be visible, such as within the ventricle of the heart of a patient. Some embodiments of the present disclosure relate to medical device shafts that may be viewable under echo within the ventricle of the patient's heart. In some embodiments, such devices may be particularly visible under echo between approximately 40 and 80° with respect to the x-plane axis in a bicommissural view. The various views from which medical device shafts and/or end effectors associated with embodiments of the present disclosure may be particularly visible and/or particularly suitable for viewing in connection with certain valve-repair procedures are described below with respect to Figures 43-45.

The high-echogenicity shafts and end effector's disclosed herein may be highly-visible under echo imaging, and may advantageously remain visible through the viewing range of between 0° and 90° with respect to the x-plane axis/line, which may cover a range of angles that may be expected and/or associated with certain valve-repair procedures as described herein. Such embodiments may provide a substantial increase in visibility over other solutions in which shafts and/or end effectors may only be visible from angles of approximately 0° to 63°, or less. Visibility over a relatively wider range of angles may be enabled by the increased scattering provided by discontinuity and material densities associated with an end effector and/or shaft.

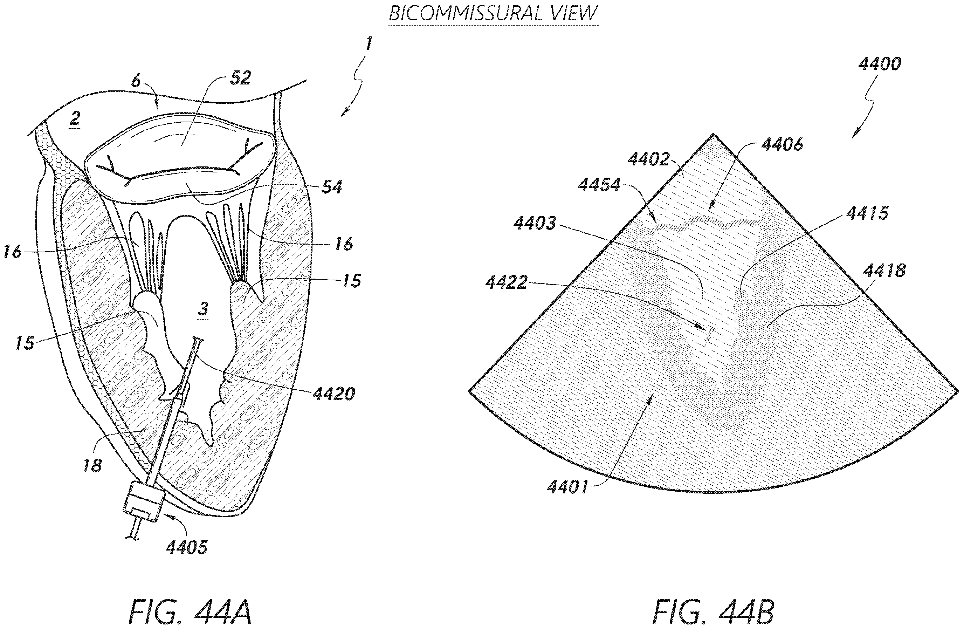

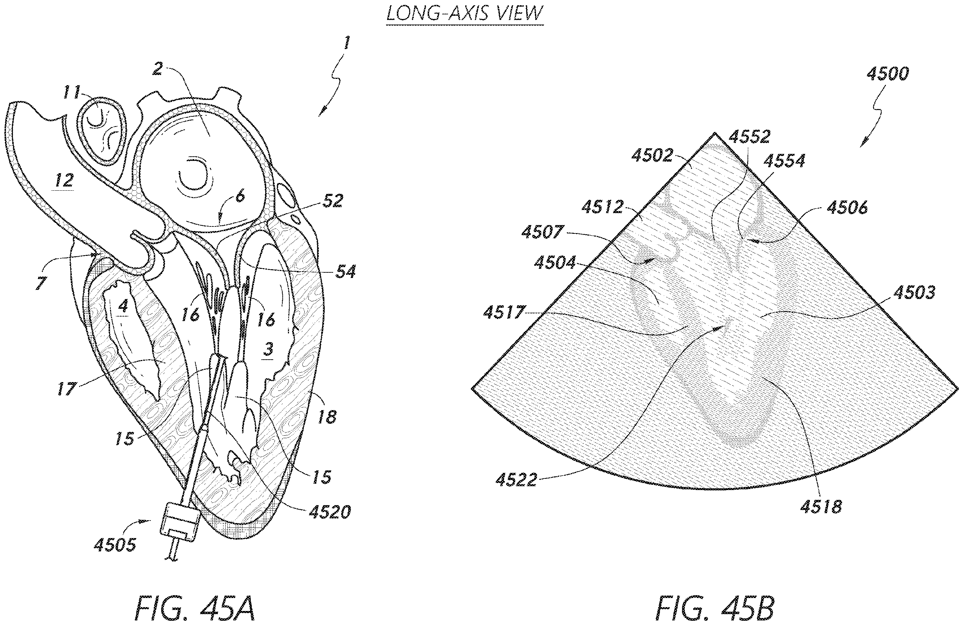

Figure 43 provides a cross-sectional view of certain cardiac anatomy in accordance with one or more embodiments of the present disclosure. In particular, the cross-sectional view provided in Figure 43 shows an axial cross-sectional view of a heart 1 showing the valves of the heart, namely the mitral valve 6, the tricuspid valve 8, the aortic valve 7, and the pulmonary valve 9. When viewing the heart using echo imaging, such as may be implemented in connection with certain valve-repair procedures described herein, it may be desirable to generate echo images between about 40° and 80° with respect to the x-plane access/line to provide a bicommissurral view, and/or at or around approximately 135° to provide a long-axis view. One or both of the bicommissural view and the long-axis view may advantageously provide a suitable viewing configuration to visualize a medical instrument shaft and/or associated end effector for a mitral valve repair procedure. Therefore, Figures 44A/B and 45A/B, described below, may be suitable and/or relevant for/to mitral valve repair. However, it should be understood that echogenicity concepts disclosed herein are applicable to medical instruments used in connection with any suitable or desirable medical procedure.

Figures 44A and 44B show bicommissural cross-sectional and echo imaging views, respectively, of the cardiac anatomy shown in Figure 43 in accordance with one or more embodiments of the present disclosure. The bicommissural view of Figures 44A and 44B may advantageously be between 40° and 80°, which may provide a view of the cardiac anatomy that is substantially in-plane. For example, the bicommissural view may advantageously maintain the left heart and the shaft 4420 in view.

The cross-sectional anatomical view of Figure 44A shows the left ventricle 3 of the heart 1, as well as the mitral valve 6, which provides an interface between the left ventricle 3 and the left atrium 2 above. The bicommissural view of Figure 44A further shows one or more papillary muscles 15, as well as associated chordae tendinea 16, which physically couple the papillary muscles 15 to the leaflet(s) of the mitral valve 6. In the view of Figure 44A, an introducer device 4405 is shown providing the channel through which a shaft 4420 of a medical instrument can access the left ventricle 3.

In the image of Figure 44A, the distal end portion of the shaft 4420, which may have an end effector associated with the distal tip thereof, is shown as projecting into the ventricle 3. In some procedures, the shaft 4420 may be advanced to one or more leaflets of the valve 6 to execute a repair procedure with respect to the mitral valve 6.

Figure 44B shows an echogenic window corresponding to the cross-sectional view shown in Figure 44A. As is apparent from the image 4400, the visibility of the relevant anatomy (e.g., chambers) of the heart 1) may be somewhat obscured and/or relatively difficult to interpret compared to photographic images/representations, or the like. Furthermore, the visibility of the shaft 4420 may be important and/or necessary for effectively executing the relevant medical procedure (e.g., valve-repair procedure). For example, as shown in image 4400, the representation 4422 of the shaft 4420 may generally be relatively difficult to identify and/or ascertain, particularly in the presence of certain image artifacts (not shown) that may appear when using echo imaging modalities.

Figures 45A and 45B show long-axis cross-sectional and echo imaging views, respectively, of the cardiac anatomy shown in Figure 43 in accordance with one or more embodiments of the present disclosure. The long-axis view may provide an effective side view for viewing the valve leaflets 52, 54, and may provide a view that is up to 90° apart from the bicommissural view shown in Figures 44A and 44B. In situations in which the end effector and/or distal ends of the shaft 4520 is visible in the bicommissural view, it may also be visible generally in the long-axis view. Generally, certain artefact representations of the shaft may appear along a center line that is aligned with echo probe (not shown; see Figure I0A). In some embodiments, echogenicity-enhancing features of the present disclosure may improve the ability of the echo technician to differentiate between the true representation of the shaft and/or end effector relative to the any present artifact(s).

The cross-sectional long-axis anatomical view of Figure 45A shows the left ventricle 3 of the heart 1, as well as the mitral valve 6 and aortic valve 7, which are accessible from the left ventricle 3. The long-axis view of Figure 45A further shows the papillary muscles 15 and associated chordae tendinea 16. The view of Figure 45A further shows an introducer device 4505 through which a shaft 4520 of a medical instrument is advanced to access the left ventricle 3. In the image of Figure 45A, the distal end portion of the shaft 4520, which may have an end effector associated with the tip thereof, is shown as projecting into the ventricle 3. In some procedures, the shaft 4520 may be advanced to one or more leaflets of the valve 6 to execute a repair procedure with respect to the mitral valve 6.

Figure 45B shows an echo imaging window 4500 corresponding to the long-axis view shown in Figure 45A. As is apparent from the image 4500, the visibility of the relevant anatomy (e.g., chambers) of the heart 1) may be somewhat obscured and/or relatively difficult to interpret compared to photographic images/representations, or the like. Furthermore, the visibility of the shaft 4420 may be important and/or necessary for effectively executing the relevant medical procedure (e.g., valve-repair procedure). For example, as shown in the image 4500, the representation 4522 of the shaft 4520 may generally be relatively difficult to identify and/or ascertain when the shaft comprises stainless-steel without echogenicity-enhancement features as described in detail herein, particularly in the presence of certain image artifacts (not shown) that may appear in connection with echo imaging modalities.

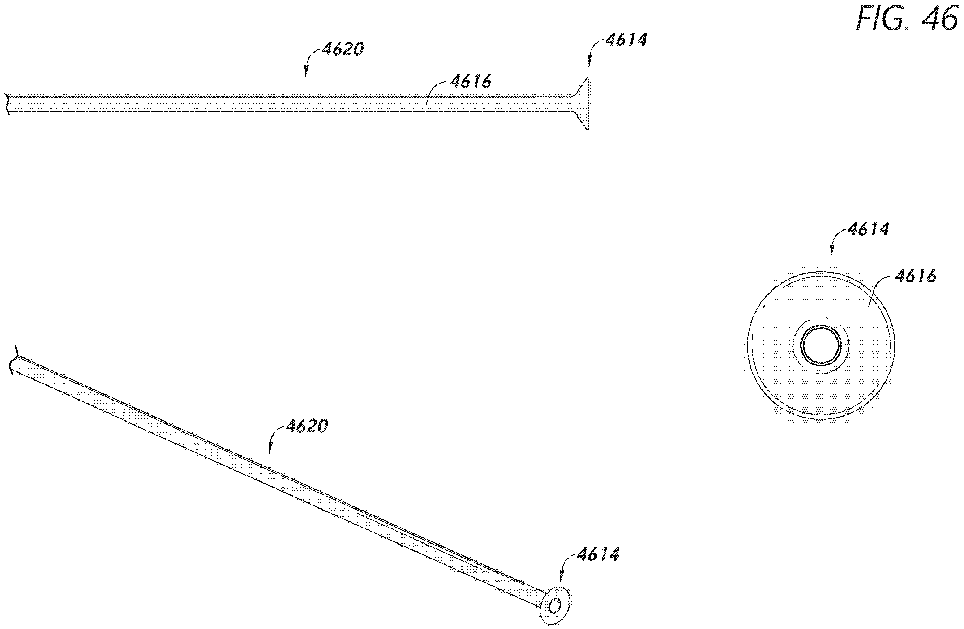

Figure 46 shows side, perspective, and end views of a shaft 4620 and end effector 4614 of a medical instrument having a relatively echogenic coating 4616 applied thereto in accordance with one or more embodiments of the present disclosure. In some embodiments, the coating 4616 may comprise a urethane compound, or the like. In some embodiments, the compound used for the coating 4616 comprises echogenic microbubbles/microspheres, as described in greater detail below.

The coating 4616 may be applied in connection with any step or portion of a manufacturing process associated with the shaft 4620 and/or end effector 4614. In some embodiments, the shaft 4620 and/or end effector 4614 may be dipped in at least partially fluid (e.g., liquid) material configured to adhere at least in part to the surface of the shaft 4620 and/or end effector 4614, such that the coating 4616 may substantially cover one or more surface portions thereof. In some embodiments, the coating 4616 may be configured to harden over time and/or in response to exposure to certain temperature and/or environmental conditions; the coating 4616 may be subjected to such conditions for the purpose of producing a desired hardening or other chemical/material change. In some embodiments, the illustrated "coating" 4616 may not actually be a coating, but may be molded, extruded, or otherwise formed.

The coating 4616 may have any suitable or desirable echogenic characteristics and/or features. For example, the coating 4616 may have any of the features disclosed herein with respect to any of the other embodiments of the present disclosure. In some embodiments, the coating 4616 may comprise and/or have associated therewith certain echogenic particulates, microspheres, bubbles, surfaces, and/or other materials or features that cause echo scattering and/or are more visible under echo than stainless steel or thermoplastic rubber alone. The various materials used in connection with embodiments of the present disclosure to provide echogenic coverings for shafts and/or end effectors may comprise echogenic polymer materials, including, for example, ePTFE, compliant polymeric foams, porous fluoropolymers, polyethylene terephthalate (PET), polyurethane, polyether block amide (e.g., PEBAX^{®} elastomer), and/or composites thereof.

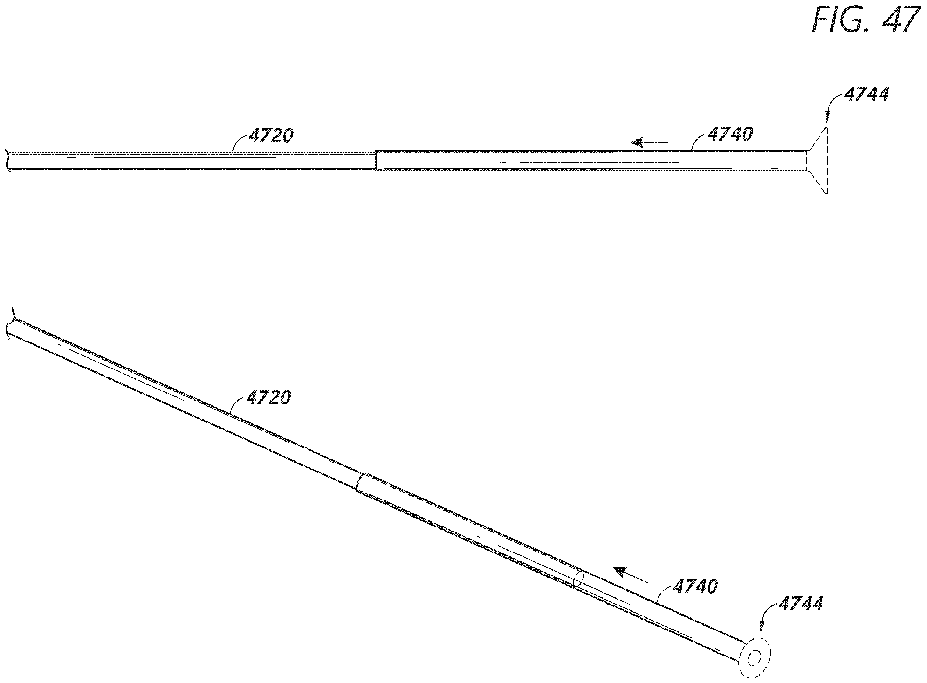

Figure 47 shows side, perspective, and end views of a shaft 4720 of a medical instrument having a relatively echogenic sleeve 4740 disposed thereon in accordance with one or more embodiments of the present disclosure. The sleeve may be formed using an extrusion process, for example.

The shaft 4720 may be any type of shaft, including medical and/or nonmedical device shafts. The sleeve 4740 may be configured and/or shaped to be disposed over at least a portion of the shaft 4720, wherein the sleeve 4740 includes materials, characteristics, and/or constituents that are relatively more echogenic when disposed on the shaft 4720 than the shaft 4720 would be alone. The shaft 4720 may comprise stainless steel or other at least partially rigid material, wherein the sleeve 4740 may be relatively less rigid and/or dense.

The sleeve 4740 may have any of the echogenicity features disclosed herein with respect to any of the other embodiments of the present disclosure. For example, the sleeve 4740 may comprise and/or have associated therewith certain echogenic particulates, microspheres, bubbles, surfaces, and/or other materials or features that are more visible under echo than stainless steel or thermoplastic rubber alone.

In some embodiments, the sleeve 4740 has associated therewith an end effector form 4740, which may be a single integrated form with the tube/lumen portion of the sleeve 4740, or may be attached to or otherwise associated with the tube/lumen portion of the sleeve 4740. As shown in the diagrams of Figure 47, the sleeve 4740 may be configured and/or dimensioned to be slid/passed over at least a portion of the shaft 4720. In some embodiments, the sleeve 4740 is dimensioned to provide a friction fit with the shaft 4720 in order to prevent undesirable sliding of the sleeve 4740 after disposal thereof on shaft 4720. In some implementations, once the sleeve 4740 has been disposed on the shaft 4720, heat or other environmental or mechanical conditions may be applied to the sleeve 4740 to thereby further cinch, adhere, and/or otherwise secure the sleeve 4740 to the shaft 4720. In some embodiments, the sleeve 4740 may comprise polymeric mesh, such as DUALMESH^{®} biomaterial (W.L. Gore, Newark, Delaware), or the like.

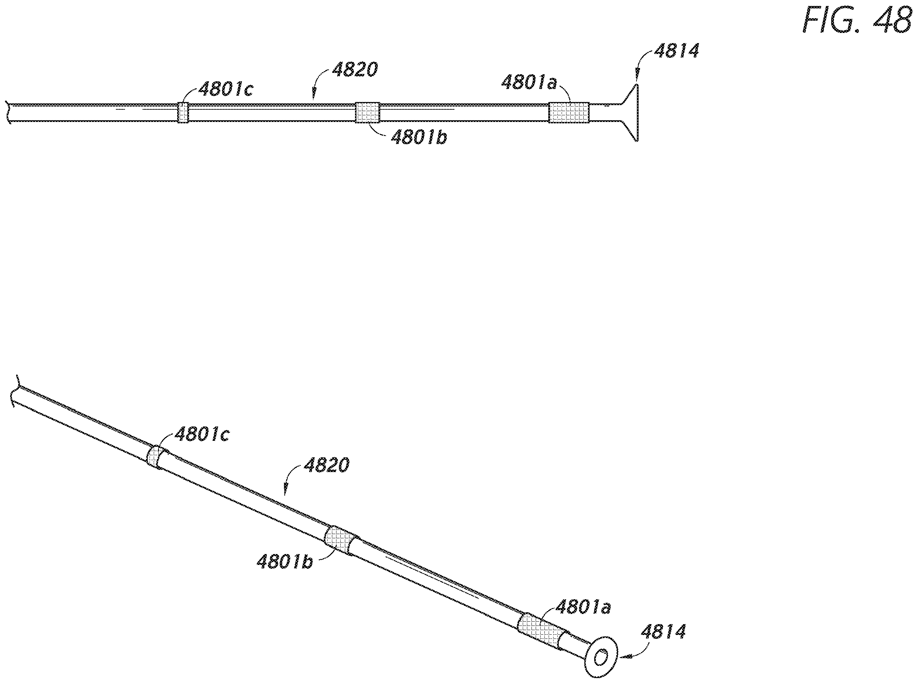

Figure 48 shows side and perspective views of a shaft 4820 of a medical instrument having relatively echogenic bands 4801 associated therewith in accordance with one or more embodiments of the present disclosure. The bands 4801 may advantageously be used as a depth gauge when viewed under echo in some implementations.

The band(s) 4801 may comprise any type of tape, band, strap, wrap, or the like, of relatively echogenic material (e.g., less dense or more dense than the shaft 4820), which can be secured to the shaft 4820 in any suitable or desirable manner. Relatively echogenic materials, as described herein, refer to the material described, itself, or to the echogenic characteristics associated with a difference/interface between the material and another material disposed adjacent thereto and/or in contact therewith. For example, a relatively echogenic material described herein may be a material that, standing alone, does not present relatively echogenic visibility compared to stainless steel and/or thermoplastic rubber. However, such material may provide relatively high echogenicity and/or echogenic characteristics when disposed on and/or in contact with another material having different density characteristics. Therefore, such relatively echogenic materials may provide such relatively high echogenicity only in combination with a shaft, end effector, or other structure on which it is disposed due at least in part to the density interface.

The band(s) 4801 may be wrapped around one or more portions of the shaft 4820 or may be slid/passed over the shaft 4820 and secured or positioned thereon/thereto in any suitable or desirable manner. The band(s) 4801 may have any of the echogenicity features disclosed herein with respect to any of the other embodiments of the present disclosure. For example, the sleeve 4740 may comprise and/or have associated therewith certain echogenic particulates, microspheres, bubbles, surfaces, and/or other materials or features that are more visible under echo than stainless steel or thermoplastic rubber alone.

One or more individual bands 4801 may be implemented on the shaft 4820. For example, where multiple bands are implemented, each band may be disposed at a different axial/longitudinal position of the shaft 4820, as shown in Figure 48. Furthermore, different echogenic bands 4801 may have different axial lengths in some embodiments, as shown in Figure 48. Such different axial lengths may serve to inform the physician or technician viewing echo images thereof as to the position (e.g., depth) of the shaft 4020. For example, identification of relatively longer or shorter bands may inform the user/technician has to what portion(s) of the shaft 4820 is being viewed and/or visible in an echo image. Although the three bands 4801 are shown in Figure 48, it should be understood that any number of bands, including one band, two bands, or more than three bands may be used/implemented in some embodiments.

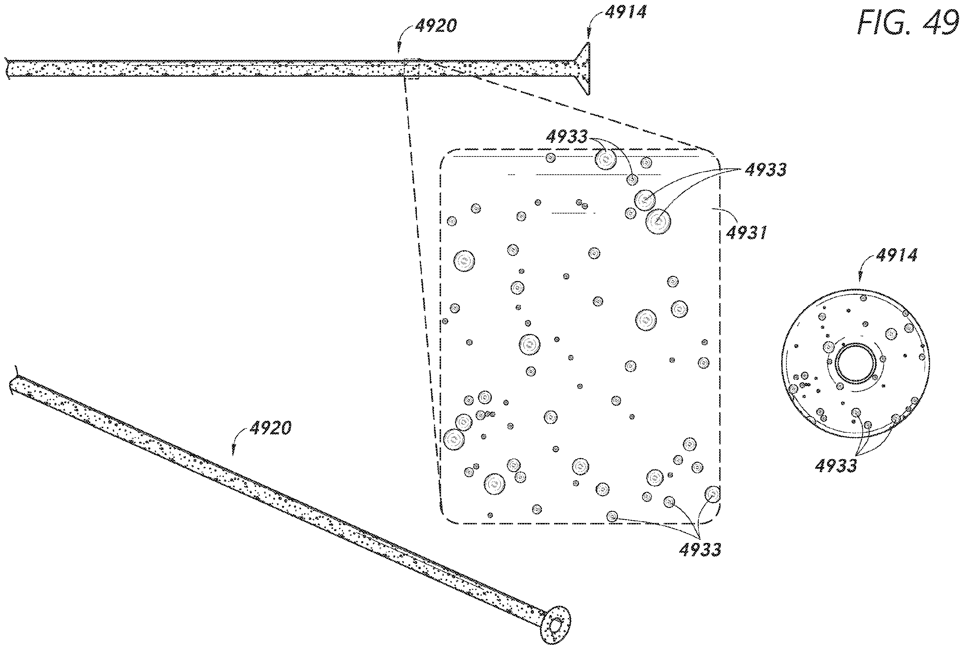

Figure 49 shows side, perspective, and end views of a shaft 4920 and end effector 4914 of a medical instrument having relatively echogenic microsphere additives 4933 associated with one or more portions thereof in accordance with one or more embodiments of the present disclosure. Generally, small air bubbles trapped within the spheres 4933 associated with the shaft 4920 and/or end effector 4914 can increase the echogenicity of such components due at least in part to the non-homogeneous surfaces provided by such spheres/bubbles that enhance the scattering of the echo image. Therefore, the shaft 4920 and/or end effector 4914 may be rendered more easily visible from a relatively wider range of angles.

The microspheres 4933 may be substantially solid, or may be air- or other gas-filled spheres. Although illustrated in the detailed view of Figure 49 has comprising microspheres of non-uniform sizes, it should be understood that in some embodiments, the microspheres 4933 are substantially uniform in size, shape, and/or with respect to one or more other dimensions thereof. The microspheres 4933 advantageously have an appropriate pressure grade with respect to associated manufacturing processes to thereby prevent the instances of breakage or damage to the microspheres, which may result in certain health risks.

In some embodiments, the microspheres 4933 may be embedded and/or mixed-in with a coating 4931, which may comprise any suitable or desirable material. For example, the shaft 4920 may be composed of the material 4931, or the material 4931 may be applied thereto and/or disposed thereon.

In some embodiments, only the end effector 4914 is associated with the micro-spheres 4933, which may be disposed generally on a surface of the end effector 4914 and/or within the structure thereof. With respect embodiments including microspheres 4933 disposed on and/or within the shaft 4920, the microspheres 4933 may be applied to the shaft 4920 and/or integrated with the radial thickness thereof. That is, the shaft 4920 may be formed of material having microspheres 4933 embedded at least partially therein or may be coated or covered with a coating/covering having microspheres associated therewith. As with other embodiments, it may be particularly desirable for the end effector 4914 to have enhanced echogenicity characteristics due to the position of the end effector relative to the deployment of a device from a working channel of the shaft.

The microspheres 4933 may comprise micro balloons (e.g., URE-FIL^{™} 15 filler, Smooth-On, Macungie, Pennsylvania), which may be added to, for example, urethane and other materials to create a relatively lightweight casting for use with the shaft 4920 and/or end effector 4914. The microspheres 4933 may comprise glass spheres in some embodiments, or other small and/or hollow spheres. In some embodiments, the microspheres 4933 are mixed with urethane rubber and poured into a mold to form the end effector 4914.

With reference to the detailed view of the microspheres 4933 in Figure 49, the microspheres 4933 may have a diameter of approximately 0.127 mm (.005 inches) or less in some embodiments. In some embodiments, the microspheres 4933 have a circumference of about 0.254 mm (.01 inches) or less. In some embodiments, the coating of the shaft 4920 may comprise embedded gas bubbles embedded in, for example, polyurethane catheter tubing, which may be achieved by adding gas during the extrusion process associated therewith. In some implementations, gas bubbles may be formed in, for example, rubber or other material used as a coating for the shaft 4920 using chlorine, hydrogen chloride gas, or other gas. Such gas may be sonicated to produce the desired bubbles.

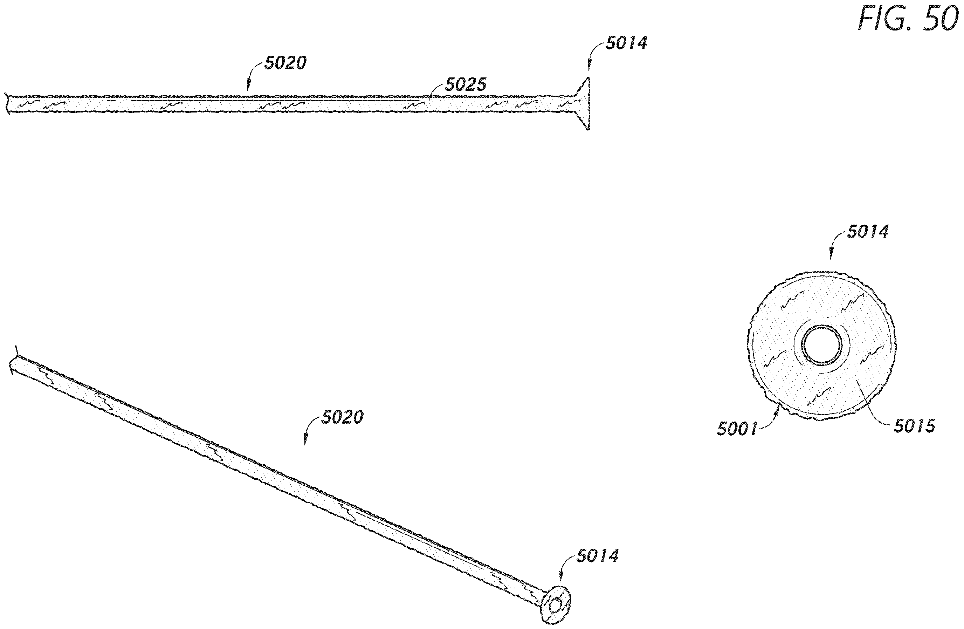

Figure 50 shows side, perspective, and end views of a shaft 5020 and end effector 5014 of a medical instrument having relatively echogenic rough/roughened surfaces 5025, 5015 associated with one or more portions thereof in accordance with one or more embodiments of the present disclosure. The rough surfaces may produce sonic reflection that is more visible under echo than more uniform reflection that may generally be produced by smooth surfaces. That is, the un-smooth/rough surface(s) of the shaft 5020 and/or end effector 5014 can induce relatively large amounts of scattering of sonic waves/signals represented in the echo image, making it easier to locate the shaft and/or end effector under echo guidance, particularly over a relatively wide range of angles.

One or more portions of the shaft 5020 and the end effector 5014 may have exterior and/or interior surfaces associated therewith configured in a rough (e.g., non-smooth) configuration/manner. For example, the surfaces of the shaft and/or end effector 5014 may be treated with an abrasive surface/material in order to produce relatively rough surfaces. In some embodiments, the rough surfaces of the shaft 5020 and/or end effector 5014 may be produced through application of a material to the shaft 5020 and/or end effector 5014. For example, such material may be applied in a non-smooth manner, resulting in uneven and/or at least partially jagged surface topology. In some embodiments, material used to form and/or cover one or more portions of the shaft 5020 and/or end effector 5014 may comprise constituents that are shaped and/or dimensioned to result in uneven surfaces thereof. The rough surface 5025 may have certain grooves, ridges, teeth, or other non-smooth surface features. In some embodiments, such features may have a height that is greater than the relevant wavelength of the echo transducer that is used.

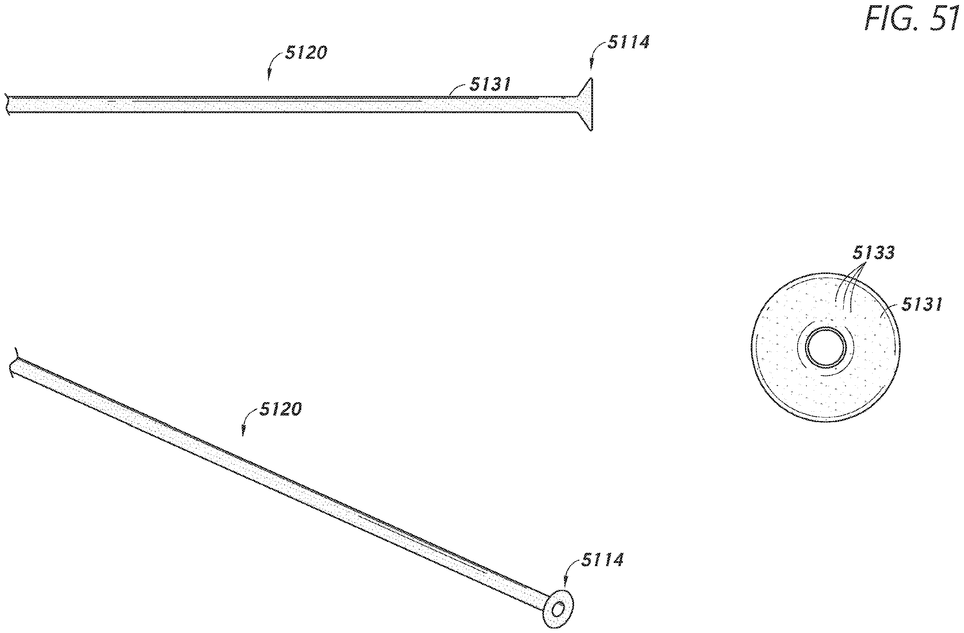

Figure 51 shows side, perspective, and end views of a shaft 5120 and end effector 5114 of a medical instrument having relatively echogenic particulate additives 5133 associated with one or more portions thereof in accordance with one or more embodiments of the present disclosure. The particulates 5133 may be of any suitable or desirable shape, size, and/or type. The particulates 5133 may advantageously be relatively more dense, or less dense, than the surrounding material (e.g., thermoplastic rubber, such as polyether block amide (e.g., PEBAX^{®} elastomer), and/or silicone), thereby resulting in a density interface between the particulates and the surrounding material that can increase sonic reflection.

Examples of particulates/additives that may be implemented in connection with embodiments of the present disclosure include, but are not limited to, white pigment, micro balloons, glass beads, or the like, which may be embedded at least partially within silicone or other similar material, which may be used as a covering and/or form for a shaft and/or end effector. In some embodiments, the particulates 5133 comprise particles of metal, zinc oxide, iron oxide, titanium dioxide, platinum oxide, silver oxide, or the like. In some embodiments, the particulates 5133 comprise stainless steel and a fine powder-type form. The particulates 5133 may comprise white pigment, such as in the form of titanium dioxide, which may represent a relatively dense material providing desirable echogenicity characteristics.

The particulates 5133 may have any chemical or structural characteristics. Furthermore, the particulates 5133 may have any size, and may have uniform or non-uniform size throughout at least a portion of the shaft 5 120 and/or end effector 5114. In some embodiments, the particulates 5133 may be embedded and/or mixed-in with a coating 5131, which may comprise any suitable or desirable material. For example, the shaft 5120 may be composed of the material 5131, or the material 5131 may be applied thereto and/or disposed thereon. A particulate solution may be provided in liquid form and mixed with rubber or other material to produce an echogenic material. The echogenic material may be mixed within and/or added to a mold/die when forming the end effector 5114 and/or covering 5131 of the shaft 5120.

In some embodiments, only the end effector 5114 is associated with the particulates 5133, which may be disposed generally on a surface of the end effector 5114 and/or within the structure/form of the end effector 5114. With respect embodiments including particulates 5133 disposed on and/or within the shaft 5120, the particulates 5133 may be applied to the shaft 5120 and/or integrated within the radial thickness thereof. That is, the shaft 5120 may be formed of material having particulates 5133 embedded/mixed at least partially therein, or may be coated or covered with a coating/covering having particulates associated therewith.

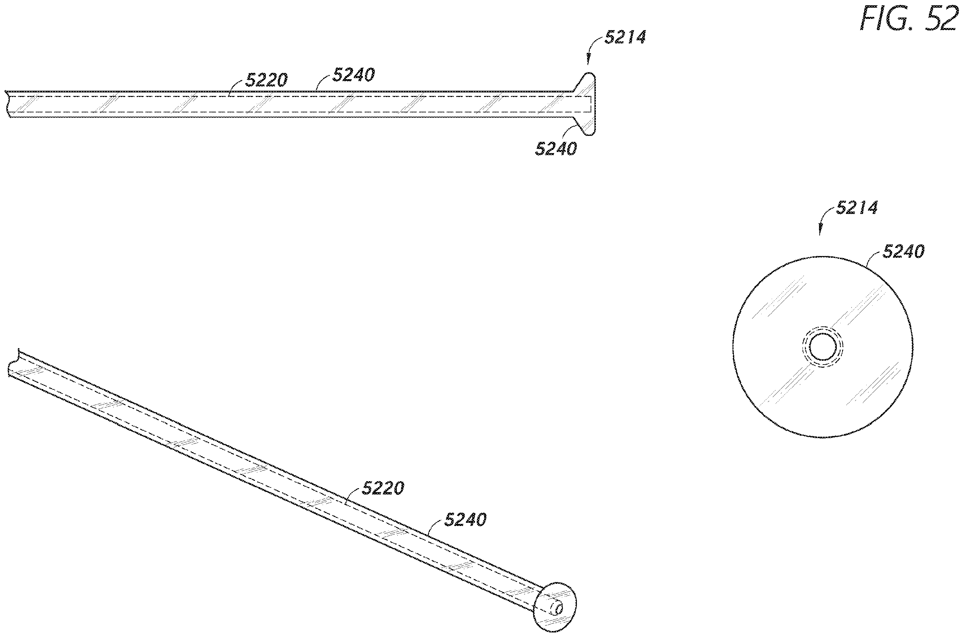

Figure 52 shows side, perspective, and end views of a shaft 5220 and end effector 5214 of a medical instrument having relatively echogenic aerogel material 5240 associated with one or more portions thereof in accordance with one or more embodiments of the present disclosure. Although described as aerogel material, it should be understood that the material 5240 may be any type of porous material. Generally, porous materials may contain air pockets that increase the scattering of echo signals, thereby producing enhanced echogenicity as described in detail herein. Example types of porous material that may be used include, for example, silica aerogel, ePTFE, various foams, and the like.

The aerogel material 5240 may be integrated with the structure of the shaft 5220, and/or may be applied as a coating thereon. Furthermore, the aerogel material 5240 may be applied as a coating to the end effector 5214, or the end effector form 5214 may be formed substantially of the aerogel material 5240, as shown in Figure 52.

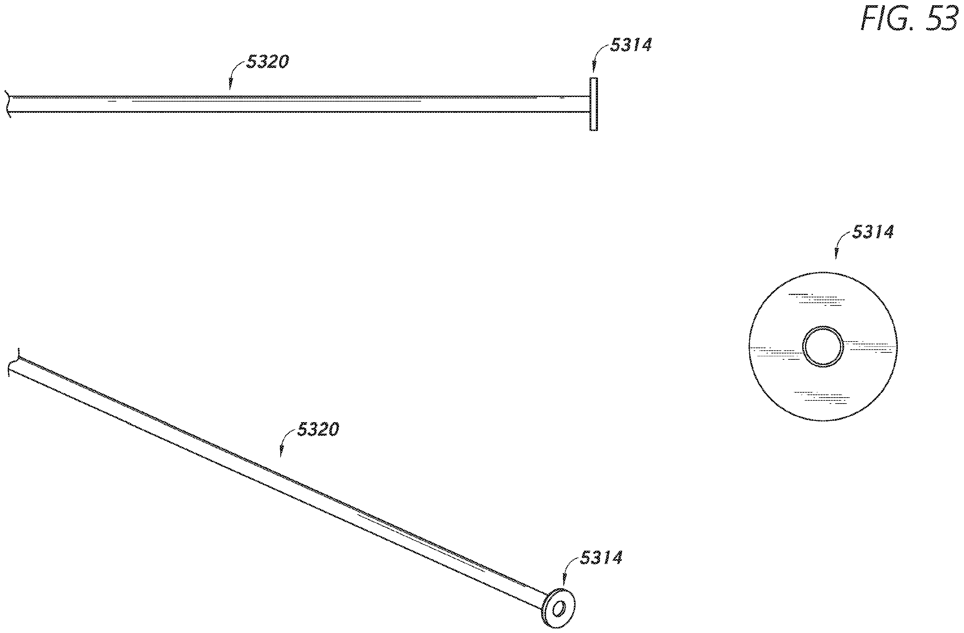

Figure 53 shows side, perspective, and end views of a shaft 5320 of a medical instrument having a relatively echogenic, washer-type end effector 5314 in accordance with one or more embodiments of the present disclosure. In some embodiments, the washer-type end effector 5314 may comprise metal, or other substantially rigid and/or conductive (e.g., thermally and/or electrically) material.

The washer-type end effector 5314 may be secured or attached to the shaft 5320 in any suitable or desirable manner, such as with an adhesive and/or mechanical fastening mechanism. In some embodiments, the end effector 5314 may be of a unitary form with the shaft 5320 and/or with at least a distal portion thereof.

Although the washer-type end effector 5314 is shown as a substantially flat disc-type form having a substantially annular or torus-type shape, it should be understood that washer-type end effectors in accordance with aspects of the present disclosure may have any suitable or desirable shape. Furthermore, such end effectors may have any radial thickness. For example, the radial thickness of the end effector 5314 may be determined to produce a desired level of echogenic visibility.

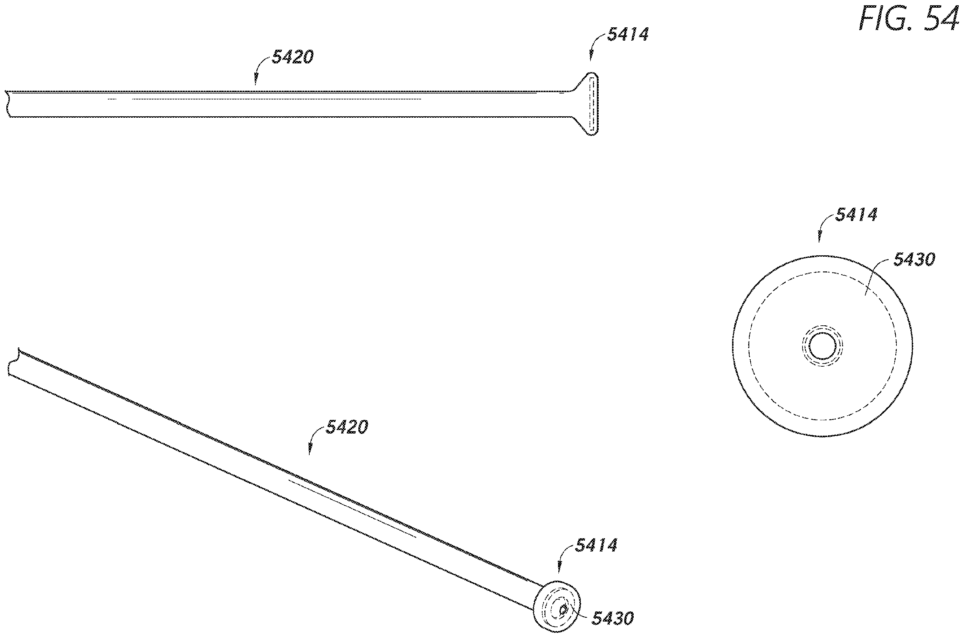

Figure 54 shows side, perspective, and end views of a shaft 5420 of a medical instrument having an end effector 5414 with a relatively echogenic, washer-type component 5430 embedded at least partially therein in accordance with one or more embodiments of the present disclosure.

In some embodiments, the end effector 5414 can comprise thermoplastic rubber and/or another at least partially flexible material. The washer 5430 may be attached to and/or embedded in the material (e.g., rubber) of the end effector 5414, at least in part. For example, the washer 5430 may be substantially covered on the distal end of the end effector 5414 by the material of the end effector, or may be at least partially exposed on one more ends or sides of the end effector 5414.

In some embodiments, the end effector 5414 may be formed using a die or mold, wherein the washer 5430 is disposed in the die/mold when the end effector 5414 is formed, to thereby secure the washer 5430 within/to the end effector 5414. In some embodiments, the washer 5430 is embedded at least partially within a thermoplastic polymer end effector, which may comprise, for example, polyaryletherketone (e.g., polyether ether ketone (PEEK)), or other semi-crystalline thermoplastic having desirable mechanical and/or chemical resistance properties. For example, the thermoplastic polymer may be melted around the washer (e.g., metal washer) 5430, thereby providing an effector having two different densities through which sonic waves may pass in one or more dimensions/axes.

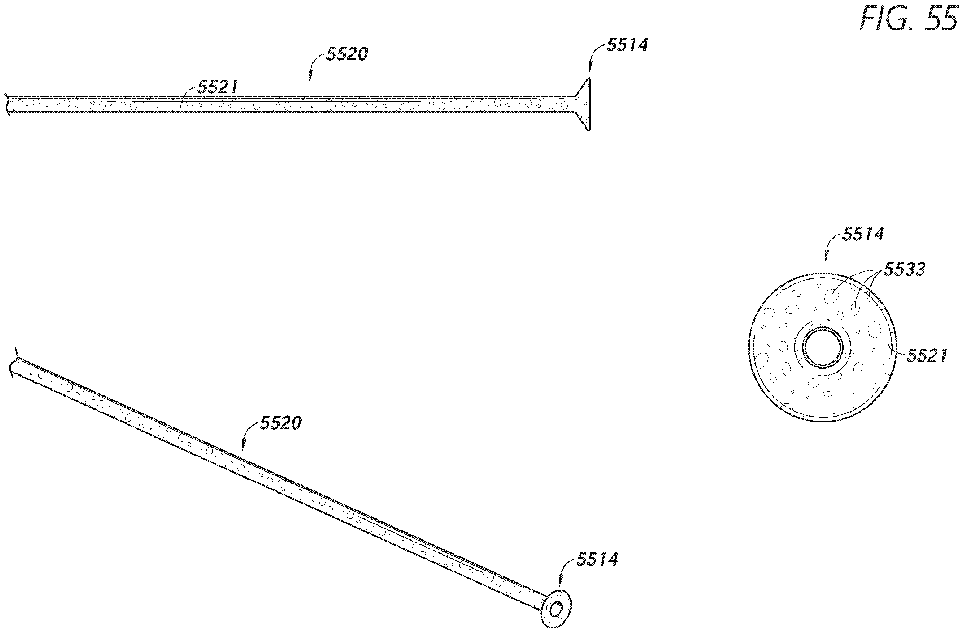

Figure 55 shows side, perspective, and end views of a shaft 5520 and end effector 5514 of a medical instrument having certain relatively echogenic air pockets and/or surface imperfections 5533 associated with one or more portions or surfaces thereof in accordance with one or more embodiments of the present disclosure. The uneven surfaces associated with the air pockets and/or surface imperfections 5533 may result in sonic reflection/scattering that increases visibility of the shaft 5520 and/or end effector 5514 under echo imaging. Molds and/or other components can be sprayed or brushed with water or other fluid, producing air pockets/divots in the polymer material of the end effector and/or shaft covering when the material is applied to the mold/component, wherein the pockets/divots create a difference in density between the air occupying such space and the polymer that produces echogenic scattering.

The air pockets and/or surface imperfections 5533 may be formed in some implementations by brushing, spraying, or otherwise disposing water or other fluid on a surface of a shaft, end effector, or mold(s) in which one or more components associated therewith his formed. For example, by brushing water on a mold/die prior to use thereof for formation of the end effector 5514 and/or shaft covering 5520, application of the material (e.g., urethane) from which the end effector 5514 and/or shaft covering 5520 is formed to the water/fluid-brushed surface of the mold/die and/or the shaft 5520 and results in pocket formation within the material and/or crater/divot formation and one or more portions or surfaces of the material. For example, air pockets may form in a nonuniform pattern between the covering material 5521 and the shaft 5520 in positions associated with water/fluid droplets present on the shaft 5520 at the time of application of the covering material 5521. Furthermore, in implementations in which water/fluid is brushed or otherwise applied to the inside surface of a mold prior to application thereto of material to be formed into the end effector 5514 and/or shaft covering 5521, air pockets may form between the molded material and the inner surface of the mold, wherein after removal of the mold/die product from the mold/die, such air pockets may present as crater-/ divot-type imperfections in the exterior surface of the molded product (e.g., end effector 5514 and/or shaft covering 5521).

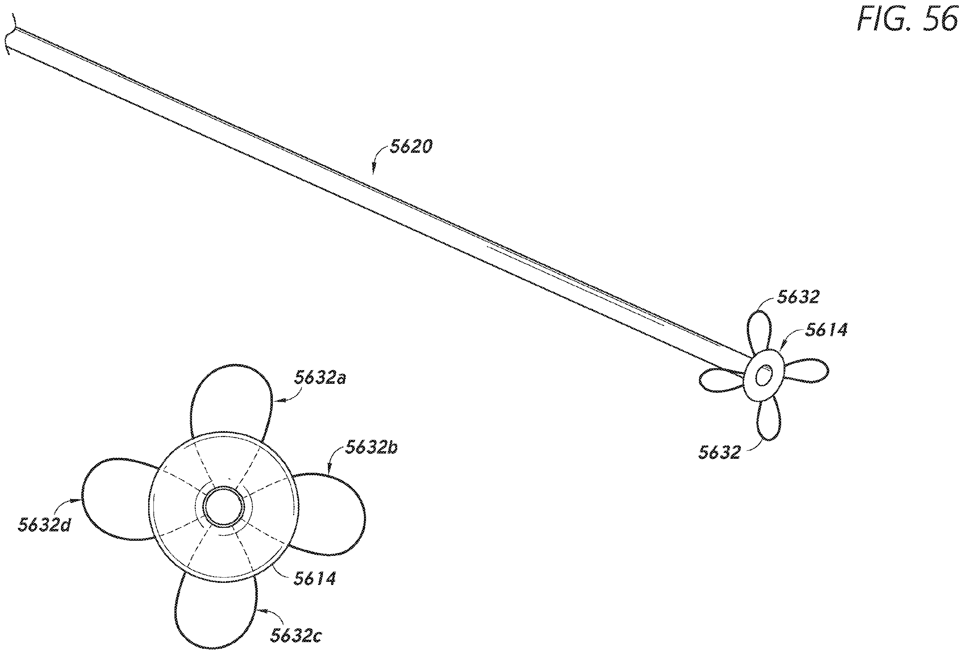

Figure 56 shows side, perspective, and end views of a shaft 5620 of a medical instrument including an end effector 5614 with one or more relatively echogenic wires and/or loops 5632 associated therewith in accordance with one or more embodiments of the present disclosure. For example, the wires 5632 may comprise metal or other relatively dense material, wherein the material of the wires 5632 is advantageously more dense (or less dense) than the material of which the end effector 5614 is comprised.

The wires/loops 5632 may be attached to the end effector 5614 and/or shaft 5620 in any suitable or desirable way. For example, in some embodiments, the wires 5632 are glued or attached in some manner to a proximal/back side of the end effector 5614, as illustrated in Figure 56. In some embodiments, the wires 5632 may be attached to a distal side of the end effector 5614, or to one or more longitudinal portions of the shaft 5620. Although the wires 5632 are shown in Figure 56 as being loop-shaped, it should be understood that any type of wire shape or form may be implemented in accordance with aspects of the present disclosure.

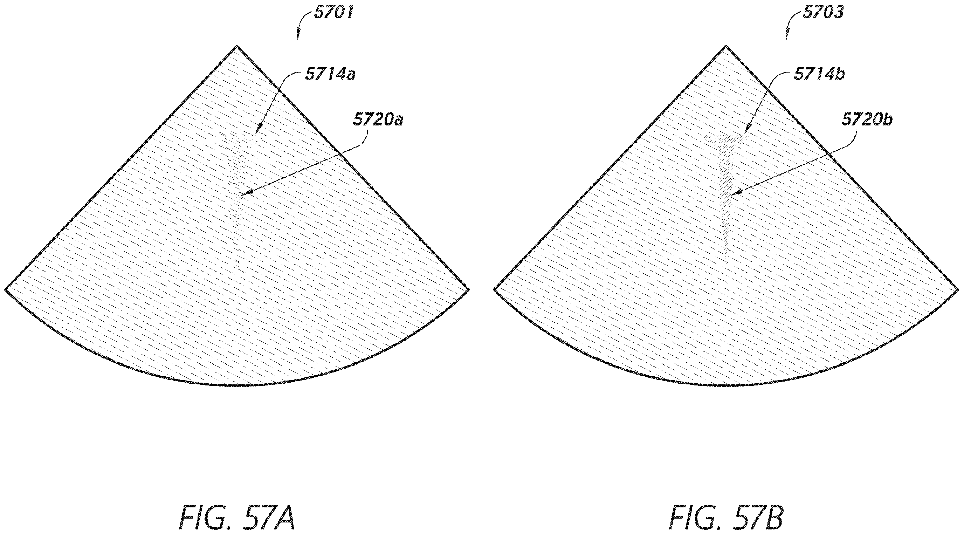

Figures 57A and 57B show echo images of relatively low- 5701 and high-echogenicity 5703 shafts 5720 and/or end effectors 5714 associated with a medical instrument in accordance with one or more embodiments of the present disclosure.

For example, the image 5701 of Figure 57A may represent the visibility of a representative image 5720a of a shaft and/or and end effector 5714a associated therewith, wherein such shaft and/or end effector does not include echogenicity enhancements in accordance with embodiments of the present disclosure. Conversely, the image 5703 of Figure 57B may represent the visibility of a representative image 5720b of a shaft and/or an end effector 5714b associated therewith, wherein such shaft and/or end effector includes one or more echogenicity enhancements according to any one or more of the embodiments disclosed herein. As is apparent from a comparison of the images 5701 and 5703, the visibility of the shaft and/or end effector represented in the image 5703 may be substantially enhanced compared to the image 5701.

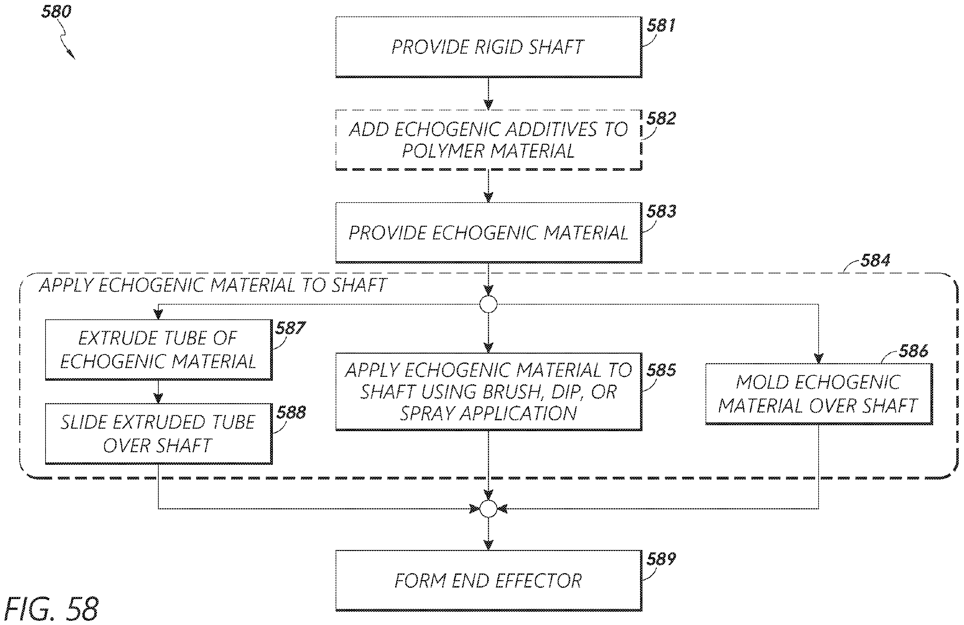

Figure 58 is a flow diagram illustrating process 580 for manufacturing echogenic medical device components in accordance with aspects of the present disclosure. In some medical device shaft manufacturing processes, a stainless steel or other at least partially rigid shaft may have a polymer (e.g., polyether block amide (PEBAX^{®} elastomer)) extrusion disposed/formed thereon, wherein an end effector is formed using a die from the polymer extrusion. Alternatively, the end effector may be formed by over-molding on to the tube structure of the shaft, such as onto a polymer extrusion or directly onto the rigid tube of the shaft. The process 580 may be similar in certain respects to such processes, as described below.

At block 582, the process 580 involves providing a rigid shaft, which may comprise stainless steel or other metal or plastic. At block 583, the process 580 involves providing/producing an echogenic material that is configured to be applied (e.g., molded) to the rigid shaft. In some embodiments, the process 580 involves, at block 583 adding certain echogenic additives to a polymer material to enhance the echogenicity characteristics of the material, to thereby produce the echogenic material referred to at block 583.

The process 580 can include a subprocess 584 for applying the echogenic material to the shaft to provide enhanced echogenicity therefor. For example, at block 586, the process 580 involves molding the echogenic material to/over the shaft, which may involve exposing at least a portion of the shaft in a mold and pouring the echogenic material over at least a portion thereof, such that the echogenic material assumes the form of the mold walls and adheres at least in part to the shaft.

In an alternative implementation, the process 580 may involve, at block 587, extruding a length of tube of the echogenic material provided at block 583, and further sliding the extruded sleeve over the rigid tube/shaft (block 588). The extrusion may have a length of tube and may or may not have an end effector formed therewith. For example, in some embodiments, an end effector may be welded at an end of the extrusion and/or to the rigid shaft. In some embodiments, the process 580 involves heat shrinking the extrusion to the shaft and/or to the end effector. Use of an extruded sleeve/tube may be a desirable alternative to over-molding the echogenic tube over the shaft due to wall thickness considerations. For example, in some embodiments, the desired wall thickness of the echogenic material around the shaft may be relatively thin, such that over molding thereof may be difficult.

In some embodiments, the echogenic material may be applied to the shaft through brush or dip application, as shown at block 585. The echogenic material may be applied to only a portion of the shaft in some cases.

At block 589, the process 580 involves forming an end effector and/or coupling the same to the shaft in some manner. Although shown as a final block in the flow diagram of Figure 58, it should be understood that formation of the end effector may be performed at any temporal point in the process 580 and/or in connection with any of the other operations of the process 580. For example, end effector formation may be achieved in connection with the molding of the echogenic material over the shaft at block 586. Furthermore, although the subprocess 584 is described as relating to the application of the echogenic material to the shaft, the application of echogenic material in accordance with the process 580 can be limited to the distal end of the shaft and/or to the end effector. That is, in some implementations, the process 580 produces a shaft having an echogenically-enhanced end effector, wherein the portion of the shaft that is proximal to the end effector is not substantially enhanced with respect to echogenicity. In some implementations, the end effector can be molded or otherwise manufactured separately from the shaft and glued, crimped, heat shrunk, or otherwise attached to the shaft.

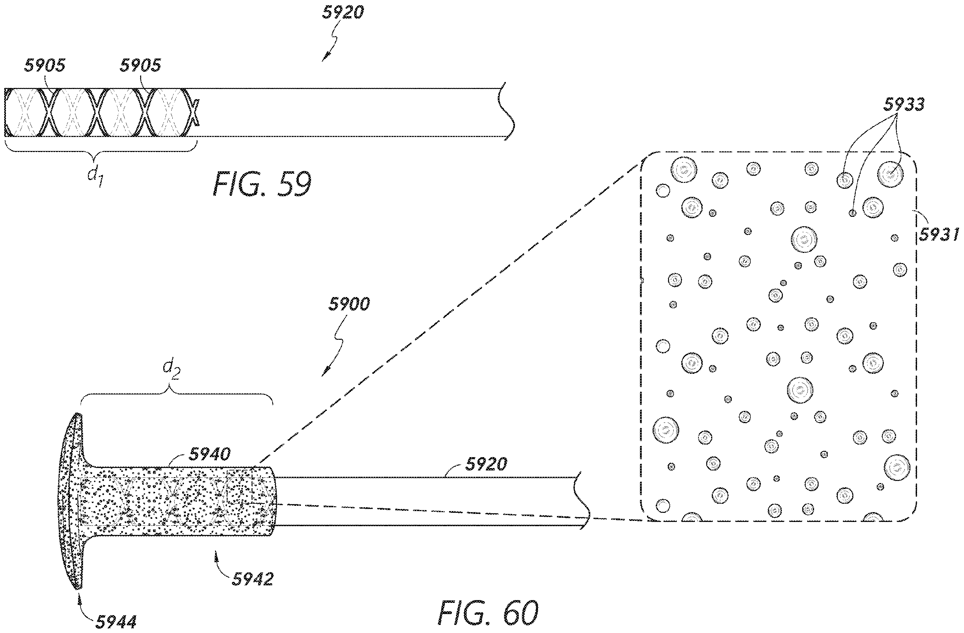

Figure 59 shows a side view of a medical device shaft 5920 having one or more channels 5905 formed therein in accordance with one or more examples of the present disclosure. The shaft 5920 may comprise a rigid elongate tube forming at least one internal lumen. The channel(s) 5920 can be configured as spiral/helix grooves that provide for improved adhesion of one or more end effector or other-type form(s) to the rigid (e.g., metal) tube 5920. The channel(s) 5920 may be formed in an exterior portion of the shaft 5920, as shown. The channel(s) 5920 may cover the entire length of the shaft 5920, or just a portion thereof. For example, the channel(s) 5920 may cover a distal portion di of the shaft 5920, as shown in Figure 59.

The channel(s) 5905 can provide image-enhancement due to sonic reflections (e.g., echo) off of the various surfaces thereof. Furthermore, the channel(s) 5905 can serve to provide improved adhesion, engagement, or purchase for a polymer or similar form applied to the shaft 5920 to secure such form in place on the shaft. Figure 60 shows a shaft device 5900 with the form 5940 applied to the shaft 5920. The form may have certain echogenicity-enhancing characteristics. In some examples, the form 5940 may be associated with a distal end portion d₂ of the shaft 5920. The form 5940 may cover some or all of the length di of the shaft 5920 that is covered by the channel(s) 5905. In some examples, only a portion of the length of the shaft 5920 that is covered by the channels(s) 5905 is covered by the form 5940. For example, in some instances, the channel(s) 5905 may span substantially the entire length of the shaft 5920, whereas only a distal end portion of the shaft is covered by the form 5940.

In examples in which the form 5940 is disposed on a distal end of the shaft 5920, the form 5940 may include a shaft portion 5942 and an end effector portion 5944 that projects radially outward and provides an atraumatic tissue-contact surface, as described in detail herein. The shaft portion 5942 may have any suitable or desirable axial length and/or radial thickness.

Although helically-shaped, and other geometrically-shaped, channels, grooves, and/or recesses are disclosed for enhancing echogenicity and/or improving physical coupling between the shaft 5920 and the echogenic form 5940 in accordance with certain examples disclosed herein, such channels, grooves, and/or recesses may be irregularly-shaped and/or non-geometrical in shape in some implementations. For example, the shaft 5920 may have channels, grooves, and/or recesses that are non-geometric in shape and/or irregular or without continuous pattern (e.g., non-contiguous channels or divots/recesses) in shape or form that are configured to provide uneven surfaces designed to increase echogenicity and/or surfaces/features to physically engage with a polymer form on the shaft for increased coupling. Furthermore, such channels, grooves, and/or recesses may be associated with any other type of device or assembly other than the shaft 5920, including other types of medical or non-medical devices. In some examples, the channels, grooves, and/or recesses are knurled in shape/form or circumferential, longitudinal grooves, or some other pattern of removed material from a shaft or other device.

The form 5940 may comprise polymer or other material and may be similar in certain respects to various other examples disclosed herein for shaft coverings or components, including end effectors and the like. In some implementations, hollow glass micro-spheres are compounded into a plastic, rubber, or other polymer and injected into a molding or extruded to form the echogenic form 5940.

The form 5940 may serve to enhance echogenicity for the shaft device 5900 and/or may provide an atraumatic distal contact for the device to reduce the risk of tissue damage during use. In some examples, the form 5940 comprises polymer 5931 having relatively echogenic microsphere additives 5933 associated with one or more portions thereof in accordance with one or more examples of the present disclosure. As described above, small air bubbles trapped within the spheres 5933 associated with the form 5940 can increase the echogenicity of the form 5940, and therefore the device 5900, due at least in part to the non-homogeneous surfaces provided by such spheres/bubbles that enhance the scattering of the echo image. Therefore, the shaft device 5900 may be rendered more easily visible from a relatively wider range of angles.

The microspheres 5933 may be substantially solid, or may be air- or other gas-filled spheres. Although illustrated in the detailed view in Figure 60 has comprising microspheres of non-uniform sizes, it should be understood that in some examples, the microspheres 5933 are substantially uniform in size, shape, and/or with respect to one or more other dimensions thereof. The microspheres 5933 advantageously have an appropriate pressure grade with respect to associated manufacturing processes to thereby prevent or reduce the instances of breakage or damage to the microspheres, which may result in certain health risks.

In some instances, the microspheres 5933 may be embedded and/or mixed-in with a base material 5931 of the form 5940, which may comprise any suitable or desirable material, such as a polymer. For example, the shaft 5920 may be composed of the material 5931, or the material 5931 may be applied thereto and/or disposed thereon, as shown if Figure 60.

The microspheres 5933 may be applied to the form 5940 and/or integrated with the thickness thereof. That is, the form 5940 may be formed of material having microspheres 5933 embedded at least partially therein or may be coated or covered with a coating/covering having microspheres associated therewith. As with other examples, it may be particularly desirable for the distal end of the shaft 5920 to have enhanced echogenicity characteristics. For example, the form 5940 may serve as an end effector.

The microspheres 5933 may comprise micro balloons (e.g., UREFIL^{™} 15 filler), which may be added to, for example, urethane and/or other material(s) to create a relatively lightweight casting for use with at least the distal portion of the shaft 5920. The micro-spheres 5933 may comprise glass spheres in some examples, or other small and/or hollow spheres. In some examples, the microspheres 5933 are mixed with urethane rubber and poured into a mold to form the form 5940.

With reference to the detailed view of the microspheres 5933 in Figure 60, the microspheres 5933 may have a diameter of approximately 0.127 mm (.005 inches) or less in some examples. In some examples, the microspheres 5933 have a circumference of about 0.254 mm (.01) inches or less. In some examples, the microspheres 5933 have a particle size (e.g., diameter) of between 2-25 microns. The mean particle size of the microspheres may be approximately 12 microns in some examples. The microspheres 5933 may have a maximum working pressure of approximately 70 MPa (about 10,000 pounds per square inch (psi)), or more. An example-type of microsphere that may be used in connection with example instances is a borosilicate glass hollow microsphere, for example, SPHERICEL^{®} microspheres (PQ Corp, Malvern, Pennsylvania). In some implementations, microspheres may be used according to a loading percentage between 10%-40%. For example, micro-spheres may be used according to a 20%-30% loading percentage, which may provide a suitable or desirable balance between visibility and moldability. In some implementations, the form 5940 comprises a resin with URE-FIL^{™} microspheres. In some implementations, the form 5940 comprises PEBAX^{®} elastomer (Arkema, Colombes, France) with 10%-40% by weight 110P8 SPHERICEL^{®} hollow microspheres.

In some examples, the form 5940 may comprise embedded gas bubbles embedded in, for example, a polyurethane form adhered to the shaft 5920, which may be achieved by adding gas during the extrusion or molding process(es) associated therewith. In some implementations, gas bubbles may be formed in, for example, rubber or other material used for the form 5940 using chlorine, hydrogen chloride gas, or other gas. Such gas may be sonicated to produce the desired bubbles. Adding the microspheres or bubbles to a plastic or rubber form can advantageously enhance its visibility under echo. In some implementations, the form 5940 is injection molded. The plastic/rubber with the compounded hollow microspheres can be injection molded or extruded into nearly any suitable or desirable shape. For example, it can be extruded into a tube for a catheter or can be injection molded into a satellite dish shape that points at the echo probe, or any other configuration for use with a shaft-like medical instrument.

### Additional Embodiments

Depending on the embodiment, certain acts, events, or functions of any of the processes or algorithms described herein can be performed in a different sequence, may be added, merged, or left out altogether. Thus, in certain embodiments, not all described acts or events are necessary for the practice of the processes.

Conditional language used herein, such as, among others, "can," "could," "might," "may," "e.g.," and the like, unless specifically stated otherwise, or otherwise understood within the context as used, is intended in its ordinary sense and is generally intended to convey that certain embodiments include, while other embodiments do not include, certain features, elements and/or steps. Thus, such conditional language is not generally intended to imply that features, elements and/or steps are in any way required for one or more embodiments or that one or more embodiments necessarily include logic for deciding, with or without author input or prompting, whether these features, elements and/or steps are included or are to be performed in any particular embodiment. The terms "comprising," "including," "having," and the like are synonymous, are used in their ordinary sense, and are used inclusively, in an open-ended fashion, and do not exclude additional elements, features, acts, operations, and so forth. Also, the term "or" is used in its inclusive sense (and not in its exclusive sense) so that when used, for example, to connect a list of elements, the term "or" means one, some, or all of the elements in the list. Conjunctive language such as the phrase "at least one of X, Y and Z," unless specifically stated otherwise, is understood with the context as used in general to convey that an item, term, element, etc. may be either X, Y or Z. Thus, such conjunctive language is not generally intended to imply that certain embodiments require at least one of X, at least one of Y and at least one of Z to each be present.

It should be appreciated that in the above description of embodiments, various features are sometimes grouped together in a single embodiment, Figure, or description thereof for the purpose of streamlining the disclosure and aiding in the understanding of one or more of the various aspects. This method of disclosure, however, is not to be interpreted as reflecting an intention that any claim require more features than are expressly recited in that claim. Moreover, any components, features, or steps illustrated and/or described in a particular embodiment herein can be applied to or used with any other embodiment(s). Further, no component, feature, step, or group of components, features, or steps are necessary or indispensable for each embodiment. Thus, it is intended that the scope of the disclosure herein disclosed and claimed below should not be limited by the particular embodiments described above but should be determined only by a fair reading of the claims that follow.

It should be understood that certain ordinal terms (e.g., "first" or "second") may be provided for ease of reference and do not necessarily imply physical characteristics or ordering. Therefore, as used herein, an ordinal term (e.g., "first," "second," "third," etc.) used to modify an element, such as a structure, a component, an operation, etc., does not necessarily indicate priority or order of the element with respect to any other element, but rather may generally distinguish the element from another element having a similar or identical name (but for use of the ordinal terms). In addition, as used herein, indefinite articles ("a" and "an") may indicate "one or more" rather than "one." Further, an operation performed "based on" a condition or event may also be performed based on one or more other conditions or events not explicitly recited.

Unless otherwise defined, all terms (including technical and scientific terms) used herein have the same meaning as commonly understood by one of ordinary skill in the art to which example embodiments belong. It be further understood that terms, such as those defined in commonly used dictionaries, should be interpreted as having a meaning that is consistent with their meaning in the context of the relevant art and not be interpreted in an idealized or overly formal sense unless expressly so defined herein.

The spatially relative terms "outer," "inner," "upper," "lower," "below," "above," "vertical," "horizontal," and similar terms, may be used herein for ease of description to describe the relations between one element or component and another element or component as illustrated in the drawings. It be understood that the spatially relative terms are intended to encompass different orientations of the device in use or operation, in addition to the orientation depicted in the drawings. For example, in the case where a device shown in the drawing is turned over, the device positioned "below" or "beneath" another device may be placed "above" another device. Accordingly, the illustrative term "below" may include both the lower and upper positions. The device may also be oriented in the other direction, and thus the spatially relative terms may be interpreted differently depending on the orientations.

Unless otherwise expressly stated, comparative and/or quantitative terms, such as "less," "more," "greater," and the like, are intended to encompass the concepts of equality. For example, "less" can mean not only "less" in the strictest mathematical sense, but also, "less than or equal to."

## Claims

1. A medical device shaft comprising:
a rigid, elongate shaft (110; 4920);
an end effector form (114; 4914) coupled to a distal end of the elongate shaft (110; 4920); and
echogenic additives associated with at least one of the elongate shaft (110) and the end effector form (114, 4914);
wherein the echogenic additives are mixed with a coating material (4931) disposed on at least a portion of the elongate shaft (110; 4920); wherein the coating material (4931) has an extruded sleeve form, wherein the end effector form (114, 4914) is a unitary form with the extruded sleeve.

2. The medical device shaft of claim 1, wherein the echogenic additives comprise microspheres (4933); in particular wherein the microspheres (4933) are gas-filled glass spheres.

3. The medical device shaft of any of claims 1 or 2, wherein the echogenic additives are embedded within the end effector form (114, 4914).

4. The medical device shaft of any of claims 1 through 3, wherein the end effector form (114; 4914) is die-cut from the extruded sleeve.

5. The medical device shaft of any of claims 1 through 4, wherein:
the echogenic additives comprise particulates mixed into a polymeric material; and
the particulates are more dense than the polymeric material.

6. The medical device shaft of claim 5, wherein the particulates comprise one or more of zinc oxide, iron oxide, titanium dioxide, platinum oxide, and silver oxide.

7. A medical device shaft comprising:
a rigid, elongate shaft (110; 5320; 5620);
a washer-type end effector form (114, 5314; 5614) coupled to a distal end of the elongate shaft (110, 5320; 5620); and
a metal echogenic-enhancement feature coupled to the end effector form (114, 5314; 5614); wherein the metal echogenic-enhancement feature comprises a washer form coupled to a distal end of the end effector form (114, 5314; 5614).

8. The medical device shaft of claim 7, wherein the metal echogenic-enhancement feature comprises one or more loops of wire (5632) secured to one or more of the elongate shaft (110; 5320; 5620) and the end effector form (114, 5314; 5614).

9. The medical device shaft of any of claims 7 or 8, wherein the washer form is at least partially embedded in the end effector form (114, 5314; 5614).

10. A method of manufacturing a medical instrument, the method comprising:
providing a rigid, elongate shaft (110; 4920); and
coupling an end effector form (114; 4914) to the elongate shaft (110; 4920);
wherein the end effector form (114; 4914) includes echogenicity-enhancing features,
the method further comprising:
adding echogenic additives to a polymeric material; wherein said coupling the end effector form (114; 4914) to the elongate shaft (110; 4920) involves molding the end effector form (114; 4914) from the polymeric material to the elongate shaft (110; 4920).

11. The method of claim 10, further comprising applying the polymeric material with the echogenic additives to at least a portion of the elongate shaft (110; 4920); or further comprising applying the polymeric material with the echogenic additives to at least a portion of the end effector form (114; 4914).

12. The method of any of claims 10 or 11, further comprising, prior to said molding the end effector form (114; 4914) to the elongate shaft (110; 4920), applying a liquid to one or more of the elongate shaft (110; 4920) and a mold used to mold the end effector form (114; 4914) to produce echogenic surface features in the end effector form (114; 4914).

13. The method of any of claims 10 through 12, further comprising using an abrasive surface to produce a rough surface on one or more of the elongate shaft (110; 4920) and the end effector form (114; 4914), wherein the rough surface is configured to produce echo scattering.

14. The method of any of claims 10 through 13, wherein the end effector form (114; 4914) comprises porous material having greater echogenicity characteristics than the elongate shaft (110; 4920); in particular wherein the porous material is aerogel.

15. The method of claim 10, further comprising forming one or more channels in an exterior surface of the elongate shaft (110; 4920); in particular wherein the end effector form (114; 4914) is disposed at least partially within the one or more channels.

## Patentansprüche

1. Schaft einer medizinischen Vorrichtung, umfassend:
einen starren länglichen Schaft (110; 4920);
eine Endeffektorform (114; 4914), die an ein distales Ende des länglichen Schafts (110; 4920) gekoppelt ist; und
echogene Additive, die mit mindestens einem von dem länglichen Schaft (110) und der Endeffektorform (114, 4914) assoziiert sind;
wobei die echogenen Additive mit einem Beschichtungsmaterial (4931) ge-mischt sind, das auf mindestens einem Abschnitt des länglichen Schafts (110; 4920) angeordnet ist; wobei das Beschichtungsmaterial (4931) eine extrudierte Hülsenform hat, wobei die Endeffektorform (114, 4914) eine einheitliche Form mit der extrudierten Hülse ist.

2. Schaft einer medizinischen Vorrichtung nach Anspruch 1, wobei die echo-genen Additive Mikrokugeln (4933) umfassen; wobei die Mikrokugeln (4933) ins-besondere gasgefüllte Glaskugeln sind.

3. Schaft einer medizinischen Vorrichtung nach einem der Ansprüche 1 oder 2, wobei die echogenen Additive innerhalb der Endeffektorform (114, 4914) eingebettet sind.

4. Schaft einer medizinischen Vorrichtung nach einem der Ansprüche 1 bis 3, wobei die Endeffektorform (114; 4914) aus der extrudierten Hülse ausgestanzt ist.

5. Schaft einer medizinischen Vorrichtung nach einem der Ansprüche 1 bis 4, wobei die echogenen Additive Partikelmaterial umfassen, das in ein polymeres Material gemischt ist; und wobei das Partikelmaterial dichter als das polymere Material ist.

6. Schaft einer medizinischen Vorrichtung nach Anspruch 5, wobei das Parti-kelmaterial ein oder mehrere von Zinkoxid, Eisenoxid, Titandioxid, Platinoxid und Silberoxid umfasst.

7. Schaft einer medizinischen Vorrichtung, umfassend:
einen starren länglichen Schaft (110; 5320; 5620);
eine Endeffektorform (114, 5314; 5614) vom Typ Unterlegscheibe, die an ein distales Ende des länglichen Schafts (110, 5320; 5620) gekoppelt ist; und
ein Echogenizitätsverbesserungsmerkmal aus Metall, das an die Endef-fektorform (114, 5314; 5614) gekoppelt ist; wobei das Echogenizitätsverbesse-rungsmerkmal aus Metall eine Unterlegscheibenform umfasst, die an ein distales Ende der Endeffektorform (114, 5314; 5614) gekoppelt ist.

8. Schaft einer medizinischen Vorrichtung nach Anspruch 7, wobei das Echo-genizitätsverbesserungsmerkmal aus Metall eine oder mehrere Drahtschlaufen (5632) umfasst, die an einem oder mehreren von dem länglichen Schaft (110; 5320; 5620) und der Endeffektorform (114, 5314; 5614) befestigt sind.

9. Schaft einer medizinischen Vorrichtung nach einem der Ansprüche 7 oder 8, wobei die Unterlegscheibenform mindestens teilweise in die Endeffektorform (114, 5314; 5614) eingebettet ist.

10. Verfahren zum Fertigen eines medizinischen Instruments, wobei das Verfahren umfasst:
Bereitstellen eines starren länglichen Schafts (110; 4920); und
Koppeln einer Endeffektorform (114; 4914) an den länglichen Schaft (110; 4920);
wobei die Endeffektorform (114; 4914) Echogenizitätsverbesserungsmerk-male einschließt, wobei das Verfahren des Weiteren umfasst:
Zugeben von echogenen Additiven zu einem polymeren Material; wobei das Koppeln der Endeffektorform (114; 4914) an den länglichen Schaft (110; 4920) Formen der Endeffektorform (114; 4914) aus dem polymeren Material zu dem länglichen Schaft (110; 4920) beinhaltet.

11. Verfahren nach Anspruch 10, des Weiteren umfassend Aufbringen des po-lymeren Materials mit den echogenen Additiven auf mindestens einen Abschnitt des länglichen Schafts (110; 4920); oder des Weiteren umfassend Aufbringen des polymeren Materials mit den echogenen Additiven auf mindestens einen Ab-schnitt der Endeffektorform (114; 4914).

12. Verfahren nach einem der Ansprüche 10 oder 11, des Weiteren umfassend, vor dem Formen der Endeffektorform (114; 4914) zu dem länglichen Schaft (110; 4920), Aufbringen einer Flüssigkeit auf ein oder mehrere von dem länglichen Schaft (110; 4920) und einem Formwerkzeug, das zum Formen der Endeffektor-form (114; 4914) verwendet wird, um echogene Oberflächenmerkmale in der Endeffektorform (114; 4914) zu produzieren.

13. Verfahren nach einem der Ansprüche 10 bis 12, des Weiteren umfassend Verwenden einer abrasiven Oberfläche, um eine raue Oberfläche auf einem oder mehreren von dem länglichen Schaft (110; 4920) und der Endeffektorform (114; 4914) zu produzieren, wobei die raue Oberfläche ausgestaltet ist, um Echostreuung zu produzieren.

14. Verfahren nach einem der Ansprüche 10 bis 13, wobei die Endeffektorform (114; 4914) poröses Material mit größeren Echogenizitätscharakteristika als der längliche Schaft (110; 4920) umfasst; wobei insbesondere das poröse Material Aerogel ist.

15. Verfahren nach Anspruch 10, des Weiteren umfassend Bilden von einem oder mehreren Kanälen in einer äußeren Oberfläche des länglichen Schafts (110; 4920); wobei insbesondere die Endeffektorform (114; 4914) mindestens teilweise innerhalb des einen oder der mehreren Kanäle angeordnet ist.

## Revendications

1. Tige de dispositif médical comprenant :
une tige allongée (110; 4920) rigide;
une forme d'effecteur terminal (114; 4914) accouplée à une extrémité distale de la tige allongée (110; 4920); et
des additifs échogènes associés à au moins un élément parmi la tige allongée (110) et la forme d'effecteur terminal (114, 4914);
les additifs échogènes étant mélangés avec un matériau de revêtement (4931) disposé sur au moins une partie de la tige allongée (110; 4920); le matériau de revêtement (4931) présentant une forme de manchon extrudé, la forme d'effecteur terminal (114, 4914) étant une forme unitaire avec le manchon extrudé.

2. Tige de dispositif médical selon la revendication 1, les additifs échogènes comprenant des microsphères (4933); en particulier, les microsphères (4933) étant des sphères de verre remplies de gaz.

3. Tige de dispositif médical selon l'une quelconque des revendications 1 ou 2, les additifs échogènes étant incorporés à l'intérieur de la forme d'effecteur terminal (114, 4914).

4. Tige de dispositif médical selon l'une quelconque des revendications 1 à 3, la forme d'effecteur terminal (114; 4914) étant découpée à l'emporte-pièce à partir du manchon extrudé.

5. Tige de dispositif médical selon l'une quelconque des revendications 1 à 4, les additifs échogènes comprenant des particules mélangées dans un matériau polymère; et les particules étant plus denses que le matériau polymère.

6. Tige de dispositif médical de la revendication 5, les particules comprenant une ou plusieurs substances parmi l'oxyde de zinc, l'oxyde de fer, le dioxyde de titane, l'oxyde de platine et l'oxyde d'argent.

7. Tige de dispositif médical comprenant :
une tige allongée (110; 5320; 5620) rigide;
une forme d'effecteur terminal (114, 5314; 5614) de type rondelle accouplée à une extrémité distale de la tige allongée (110, 5320; 5620); et
une caractéristique d'amélioration échogène métallique accouplée à la forme d'effecteur terminal (114, 5314; 5614); la caractéristique d'amélioration échogène métallique comprenant une forme de rondelle accouplée à une extrémité distale de la forme d'effecteur terminal (114, 5314; 5614).

8. Tige de dispositif médical selon la revendication 7, la caractéristique d'amélioration échogène métallique comprenant une ou plusieurs boucles de fil (5632) fixées à un ou plusieurs éléments parmi la tige allongée (110; 5320; 5620) et la forme d'effecteur terminal (114, 5314; 5614).

9. Tige de dispositif médical selon l'une quelconque des revendications 7 ou 8, la forme de rondelle étant au moins partiellement incorporée dans la forme d'effecteur terminal (114, 5314; 5614).

10. Procédé de fabrication d'un instrument médical, le procédé comprenant :
la fourniture d'une tige allongée (110; 4920) rigide; et
l'accouplement d'une forme d'effecteur terminal (114; 4914) à la tige allongée (110; 4920);
la forme d'effecteur terminal (114; 4914) comprenant des caractéristiques d'amélioration de l'échogénicité, le procédé comprenant en outre:
l'ajout d'additifs échogènes à un matériau polymère; ledit accouplement de la forme d'effecteur terminal (114; 4914) à la tige allongée (110; 4920) impliquant le moulage de la forme d'effecteur terminal (114; 4914) à partir du matériau polymère à la tige allongée (110; 4920).

11. Procédé selon la revendication 10, comprenant en outre l'application du matériau polymère présentant les additifs échogènes à au moins une partie de la tige allongée (110; 4920); ou comprenant en outre l'application du matériau polymère présentant les additifs échogènes à au moins une partie de la forme d'effecteur terminal (114; 4914).

12. Procédé selon l'une quelconque des revendications 10 ou 11, comprenant en outre, avant ledit moulage de la forme d'effecteur terminal (114; 4914) à la tige allongée (110; 4920), l'application d'un liquide à un ou plusieurs éléments parmi la tige allongée (110; 4920) et un moule utilisé pour mouler la forme d'effecteur terminal (114; 4914) afin de produire des caractéristiques de surface échogènes dans la forme d'effecteur terminal (114; 4914).

13. Procédé selon l'une quelconque des revendications 10 à 12, comprenant en outre l'utilisation d'une surface abrasive afin de produire une surface rugueuse sur un ou plusieurs éléments parmi la tige allongée (110; 4920) et la forme d'effecteur terminal (114; 4914), la surface rugueuse étant conçue pour produire une diffusion d'écho.

14. Procédé selon l'une quelconque des revendications 10 à 13, la forme d'effecteur terminal (114; 4914) comprenant un matériau poreux présentant des caractéristiques d'échogénicité supérieures à celles de la tige allongée (110; 4920); en particulier, le matériau poreux étant un aérogel.

15. Procédé selon la revendication 10, comprenant en outre la formation d'un ou de plusieurs canaux dans une surface extérieure de la tige allongée (110; 4920); en particulier, la forme d'effecteur terminal (114; 4914) étant disposée au moins partiellement à l'intérieur dudit un ou desdits plusieurs canaux.
